# EUROPEAN PATENT APPLICATION

(11) **EP 4 421 078 A1**
(43) Date of publication of application: **28.08.2024**
(21) Application number: 22882897.6
(22) Date of filing: 19.10.2022
(51) Int. Cl.: C07F 5/02, A61K 31/69, A61P 31/04

(54) **TRICYCLIC BORONIC ACID DERIVATIVE, AND PREPARATION METHOD THEREFOR AND APPLICATION THEREOF**

(30) Priority: 19.10.2021 CN 202111217905; 13.10.2022 CN 202211255968
(71) Applicant: Shanghai Jemincare Pharmaceutical Co., Ltd., Shanghai 201315 (CN)
(72) Inventor: ZHANG, Jianmin, Shanghai 201203 (CN); GUO, Shuchun, Shanghai 201203 (CN); ZHANG, Zilong, Shanghai 201203 (CN); ZHENG, Cheng, Shanghai 201203 (CN); LI, Lingjun, Shanghai 201203 (CN); FENG, Jianbo, Shanghai 201203 (CN); BAO, Fang, Shanghai 201203 (CN); HU, He, Shanghai 201203 (CN); WU, Nan, Shanghai 201203 (CN); ZHANG, Zhitao, Shanghai 201203 (CN); WANG, Yu, Shanghai 201203 (CN); YE, Yan, Shanghai 201203 (CN); PENG, Jianbiao, Shanghai 201203 (CN)
(74) Representative: De Clercq & Partners
(86) International application number: PCT/CN2022/126142
(87) International publication number: WO 2023/066292

(57) **Abstract**

Disclosed are a tricyclic boronic acid derivative, and a preparation method therefor and an application thereof. Specifically, discloses are a compound represented by formula (I), an optical isomer and pharmaceutically acceptable salt thereof, and an application of the compound as a β-lactamase inhibitor and an application of a composition as an antibacterial drug.

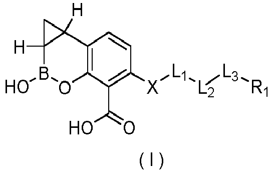

## Description

The present application claims the right of the following priorities:
CN202111217905.0, application date: October 19, 2021;
CN202211255968.X, application date: October 13, 2022.

### TECHNICAL FIELD

The present disclosure belongs to the field of medicinal chemistry. Specifically, the present disclosure relates to a compound of formula (I), an optical isomer thereof and a pharmaceutically acceptable salt thereof, and an application of the compound as a β-lactamase inhibitor and an application of a composition as an antibacterial drug.

### BACKGROUND

Due to the extensive and overuse of antibiotics, pathogenic bacteria have rapidly evolved the ability to resist antimicrobial drugs, resulting in a large number of drug-resistant bacteria, multi-drug-resistant bacteria and even superbugs. Drug resistance of bacteria has become a major challenge in the field of global public health. According to the Global Review of Antibiotic Resistance in 2014, 700,000 people have died of antibiotic resistance every year. In China, the drug resistance rate of bacteria is much higher than that in European and American countries, about 45%. More than 80,000 people in China died from the infection of drug-resistant bacteria every year. If the current situation is not improved and new antibiotics against drug-resistant bacteria are not available, one million people will die every year in China by 2050, and the GDP loss will exceed $20 trillion.

β-Lactam antibiotics are the most important antibacterial drugs in clinic at present, with a total market share of more than 50%. β-lactam antibiotics refer to a large class of antibiotics with β-lactam ring in their chemical structure, including penicillin and cephalosporin, which are the most commonly used in clinic, as well as other atypical β-lactam antibiotics such as carbapenems and monocyclic β-lactams. These antibiotics have the advantages of strong bactericidal activity, low toxicity, wide indications, and good clinical efficacy. The mechanisms of action of β-lactam antibiotics are similar in that they all can inhibit cell wall mucopeptide synthetase, i.e., a penicillin-binding protein, thus hindering the synthesis of cell wall mucopeptide, resulting in bacterial cell wall defects and bacterial expansion and lysis.

One of the main mechanisms of bacterial resistance to β-lactam antibiotics is the production of β-lactamase. Up to now, more than 2800 kinds of β-lactamases have been reported. There are many differences in terms of sources, substrates, inhibitors, structures, etc. In 1980, based on amino acid sequence analysis, Ambler classified P-lactamases into four categories according to their molecular structures, class A for penicillinases, class B for metallo β-lactamases, class C for cephalosporinases, and class D for oxacillinase. The reactive groups of classes A, C and D are serine β-lactamase (SBL) and class B is metal zinc ion β-lactamase (MBL).

β-lactamase inhibitors can bind to β-lactamase to inactivate the enzyme, and can be combined with β-lactam antibiotics in clinic to inhibit drug-resistant bacteria, reduce dosage, expand antibacterial spectrum and enhance antibacterial activity. Up to now, there are 6 kinds of β-lactamase inhibitors approved by FDA, but all of them have great limitations and cannot effectively treat MBL-mediated resistance of superbug. Clavulanic acid, sulbactam, and tazobactam only inhibit part of class A SBL; avibactam inhibits class A, C, and D SBLs, but has no effect on pathogenic bacteria resistant through MBL. MBL can hydrolyze all β-lactam antimicrobials except monocyclic β-lactams, including penicillins, cephalosporins, extended-spectrum cephalosporins, and carbapenems. According to China antimicrobial surveillance network (CHINET), among the strains clinically isolated in China in 2018, the resistance of Klebsiella pneumoniae, Pseudomonas aeruginosa, and Acinetobacter to carbapenem antibiotics such as meropenem reached 26.3%, 25.8%, and 73.9% respectively, and the resistance rate of Klebsiella pneumoniae to meropenem increased from 2.9% in 2005 to 26.3%, while the resistance rate of Acinetobacter baumannii increased from 41.3% in 2005 to 73.9%. Acinetobacter baumannii has a strong ability to acquire drug resistance and clone transmission, and has evolved into an important pathogen of nosocomial infection. Carbapenem-resistant Acinetobacter baumannii ranks very high in the WHO priority list of effective drug research and development for antibiotic-resistant bacteria. Carbapenems are the last line of defense against bacterial infection. Once drug resistance develops, the available drugs are extremely limited, and even no drugs are available in some cases. Therefore, there is an urgent clinical need for new antibacterial drugs to break through the treatment dilemma and solve the increasingly prominent drug resistance challenge.

There are many mechanisms of bacterial drug resistance, which can be summarized as follows: (1) The permeability changes of bacterial cell wall or outer membrane, such as the decrease of pore protein expression and the increase of efflux pump expression, making it impossible or rare to reach the target site in bacteria, and the outer membrane of gram-negative bacteria is the first barrier to limit the penetration of β-lactam antibiotics into bacteria; (2) Mutation of target protein leads to lower binding to antibiotics, increase in target proteins, or generation of new target proteins. For example, methicillin-resistant Staphylococcus aureus is highly resistant due to the production of a new PBP2' between the original PBP2 and PBP3, and the low and moderate drug resistance is caused by the increase in the production of PBPs or a decrease in affinity with methicillin, etc.; (3) Bacteria produce β-lactamases to hydrolyze and inactivate antibiotics, or to bind to antibiotics, causing them to stay in the extracellular space and unable to enter cells and bind to target proteins. The main drug resistance mechanism of Gram-positive bacteria is the production of β-lactamase and PBP mutation, while the main drug resistance mechanism of Gram-negative bacteria is the production of β-lactamase.

### CONTENT OF THE PRESENT INVENTION

In the first aspect of the present disclosure, the present disclosure provides a compound of formula (I), an optical isomer thereof, and a pharmaceutically acceptable salt thereof, 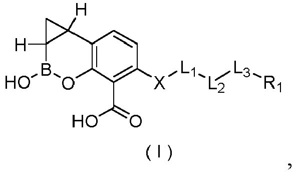 wherein
X is selected from S, S(=O), and S(=O)₂;
L₁ is selected from a single bond, C₁₋₆ alkyl, and C₁₋₆ heteroalkyl, and the C₁₋₆ alkyl or C₁₋₆ heteroalkyl is optionally substituted by 1, 2, or 3 R;
L₂ is selected from a single bond, C₃₋₆ cycloalkyl, and 4- to 9-membered heterocycloalkyl, and the C₃₋₆ cycloalkyl or 4- to 9-membered heterocycloalkyl is optionally substituted by 1, 2, or 3 R;
L₃ is selected from a single bond, C₁₋₆ alkyl, C₁₋₆ heteroalkyl, and 4- to 9-membered heterocycloalkyl, and the C₁₋₆ alkyl, C₁₋₆ heteroalkyl, or 4- to 9-membered heterocycloalkyl is optionally substituted by 1, 2, or 3 R;
R₁ is selected from NH₂, 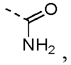 -NRaC(=NH)R_{b}, -NRₐC(=NH)NR_{b1}R_{b2}, -C(=NH)R_{b}, C₁₋₆ alkyl, C₃₋₆ cycloalkyl, 4- to 8-membered heterocycloalkyl, partially unsaturated 4- to 8-membered heterocycloalkyl, and 5- to 6-membered heteroaryl, and the C₁₋₆ alkyl, C₃₋₆ cycloalkyl, 4- to 8-membered heterocycloalkyl, partially unsaturated 4- to 8-membered heterocycloalkyl, or 5- to 6-membered heteroaryl is optionally substituted by 1, 2, or 3 R;
R, Rₐ, R_{b}, R_{b1}, and R_{b2} are each independently selected from H, F, Cl, Br, OH, NH₂, CN, C₁₋₆ alkyl, and C₃₋₆ cycloalkyl, and the C₁₋₆ alkyl or C₃₋₆ cycloalkyl is optionally substituted by 1, 2, or 3 R';
each R' is independently selected from F, Cl, Br, I, NH₂, OH, and Me;
the C₁₋₆ heteroalkyl, 4- to 9-membered heterocycloalkyl, partially unsaturated 4- to 8-membered heterocycloalkyl, or 5- to 6-membered heteroaryl comprises 1, 2, or 3 heteroatoms or heteroatom group independently selected from O, NH, S, C(=O), C(=NH), C(=O)O, S(=O), S(=O)₂, and N.

In some embodiments of the present disclosure, the compound of formula (I), the optical isomer thereof, and the pharmaceutically acceptable salt thereof are selected from compounds of formulas (I-A) and (I-B), optical isomers thereof, and pharmaceutically acceptable salts thereof, 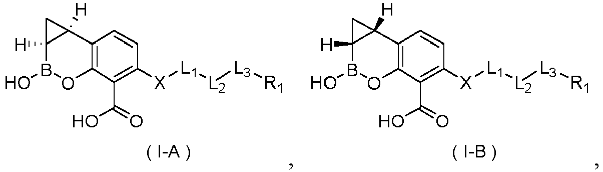 wherein
X, L₁, L₂, L₃, and R₁ are as defined above.

In some embodiments of the present disclosure, each R is independently selected from R selected from F, Cl Br, OH NH₂, CH₃, 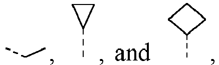 and the CH₃, 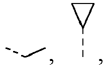 or 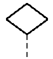 is optionally substituted by 1, 2, or 3 R', and the remaining variables are as defined in the present disclosure.

In some embodiments of the present disclosure, each R is independently selected from F, Cl, Br, OH, NH₂, CH₃, 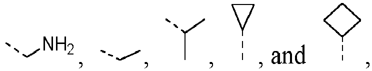 and the remaining variables are as defined in the present disclosure.

In some embodiments of the present disclosure, L₁ is selected from a single bond, C₁₋₃ alkyl, -C₁₋₃ alkyl-O-, -C₁₋₃ alkyl-S-, -C₁₋₃ alkyl-NH-, and -C₁₋₃ alkyl-C(=O)-, and the C₁₋₃ alkyl, -C₁₋₃ alkyl-O-, -C₁₋₃ alkyl-S-, -C₁₋₃ alkyl-NH-, or -C₁₋₃ alkyl-C(=O) - is optionally substituted by 1, 2, or 3 R, and the remaining variables are as defined in the present disclosure.

In some embodiments of the present disclosure, L₁ is selected from a single bond, CH₂, 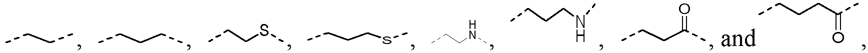 and the CH₂, 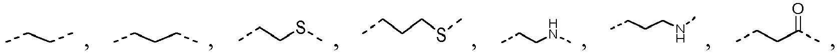 or 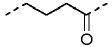 is optionally substituted by 1, 2, or 3 R, and the remaining variables are as defined in the present disclosure.

In some embodiments of the present disclosure, L₁ is selected from a single bond, CH₂, 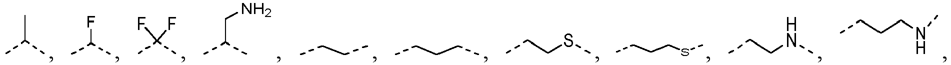 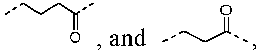 and the remaining variables are as defined in the present disclosure.

In some embodiments of the present disclosure, L₂ is selected from a single bond, cyclobutyl, cyclopentyl, azetidinyl, piperidinyl, piperazinyl, morpholinyl, and 2,6-diazaspiro[3.3]heptyl, and the cyclobutyl, cyclopentyl, azetidinyl, piperidinyl, piperazinyl, morpholinyl, or 2,6-diazaspiro[3.3]heptyl is optionally substituted by 1, 2, or 3 R, and the remaining variables are as defined in the present disclosure.

In some embodiments of the present disclosure, L₂ is selected from a single bond, 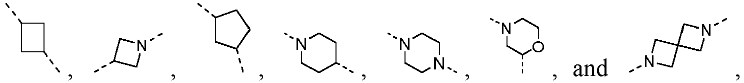 and the remaining variables are as defined in the present disclosure.

In some embodiments of the present disclosure, L₃ is selected from a single bond, C(=O), C₁₋₃ alkyl, -C₁₋₃ alkyl-C(=O)-, and -C₁₋₃ alkyl-C(=O)NH-, and the C₁₋₃ alkyl, -C₁₋₃ alkyl-C(=O)-, or -C₁₋₃ alkyl-C(=O)NH- is optionally substituted by 1, 2, or 3 R, and the remaining variables are as defined in the present disclosure.

In some embodiments of the present disclosure, L₃ is selected from a single bond, C(=O), 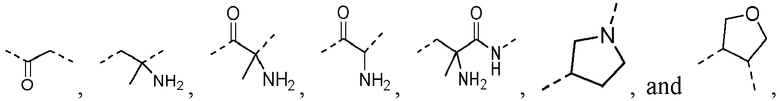 and the remaining variables are as defined in the present disclosure.

In some embodiments of the present disclosure, R₁ is selected from NH₂, 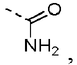 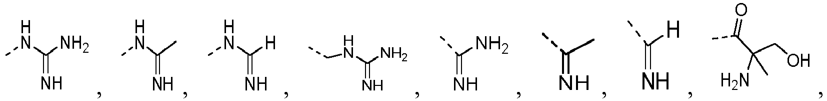 C₁₋₃ alkyl, cyclopropyl, cyclobutyl, oxetanyl, azetidinyl, pyrrolidinyl, tetrahydrofuranyl, tetrahydro-2H-pyranyl, morpholinyl, piperidinyl, piperazinyl, octahydrocyclopentadieno[c]pyrrolyl, 4,5-dihydro-1H-imidazolyl, imidazolyl, 4,5-dihydro-1H-imidazolyl, imidazolin-2-imino, tetrahydropyrimidin-2(1H)-imino, 2,6-diazaspiro[3.3]heptyl, 2-oxa-6-azaspiro[3.3]heptyl, 2,6-diazaspiro[3.4]octyl, 6-oxa-2-azaspiro[3.4]octyl, 2,6-diazaspiro[3.4]octyl, 3,6-diazabicyclo[3.1.1]heptyl, and 2,5-diazabicyclo[2.2.1]heptyl, and the C₁₋₃ alkyl, cyclopropyl, cyclobutyl, oxetanyl, azetidinyl, pyrrolidinyl, tetrahydrofuranyl, tetrahydro-2H-pyranyl, morpholinyl, piperidinyl, piperazinyl, octahydrocyclopentadieno[c]pyrrolyl, 4,5-dihydro-1H-imidazolyl, imidazolyl, 4,5-dihydro-1H-imidazolyl, imidazolin-2-imino, tetrahydropyrimidin-2(1H)-imino, 2,6-diazaspiro[3.3]heptyl, 2-oxa-6-azaspiro[3.3]heptyl, 2,6-diazaspiro[3.4]octyl, 6-oxa-2-azaspiro[3.4]octyl, 2,6-diazaspiro[3.4]octyl, 3,6-diazabicyclo[3.1.1]heptyl, or 2,5-diazabicyclo[2.2.1]heptyl is optionally substituted by 1, 2, or 3 R, the remaining variables are as defined in the present disclosure.

In some embodiments of the present disclosure, R₁ is selected from NH₂, 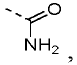 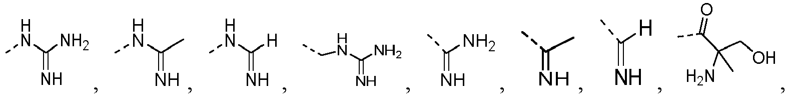 Me, 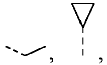 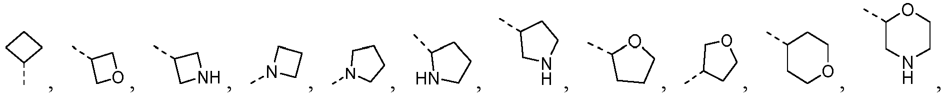 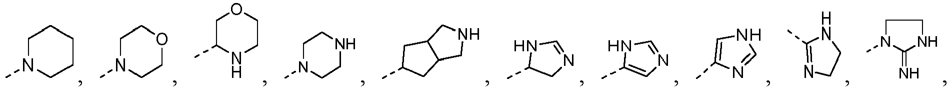 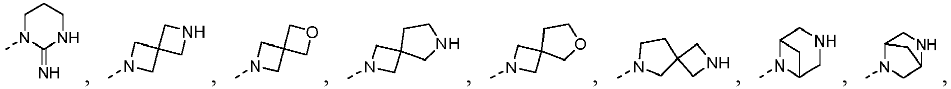 and 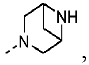 and the Me, 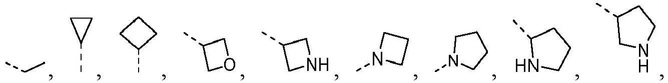 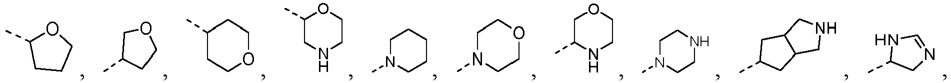 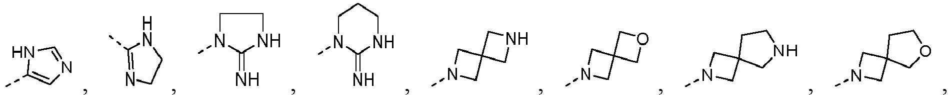 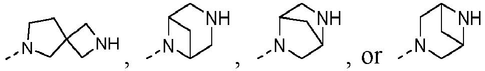 is optionally substituted by 1, 2, or 3 R, the remaining variables are as defined in the present disclosure.

In some embodiments of the present disclosure, R₁ is selected from NH₂, Me, CF₃, 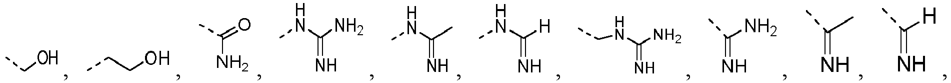 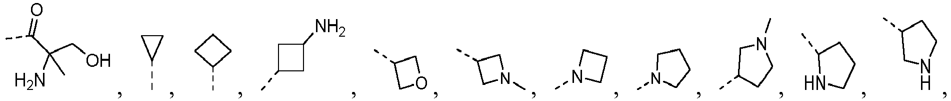 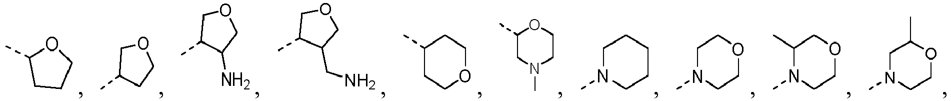 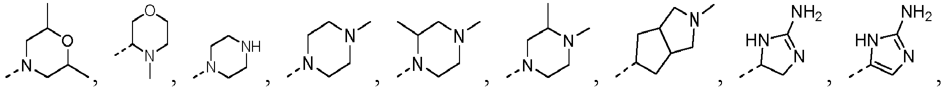 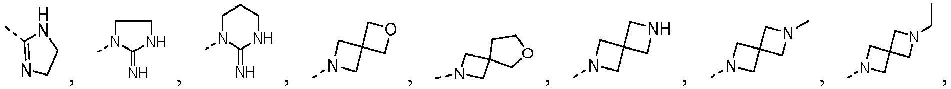 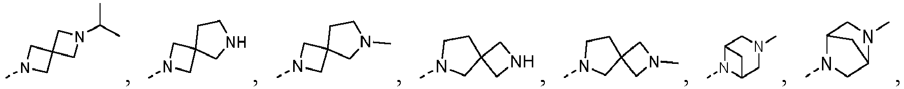 and 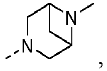 and the remaining variables are as defined in the present disclosure.

In some embodiments of the present disclosure, the structural moiety 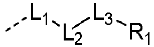 is selected from Me, CF₃, 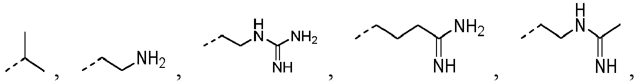 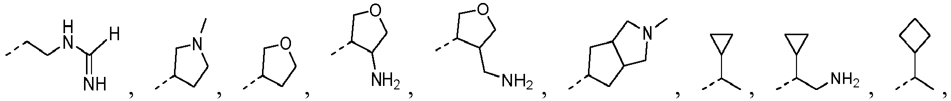 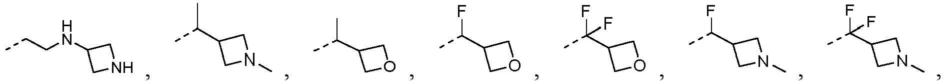 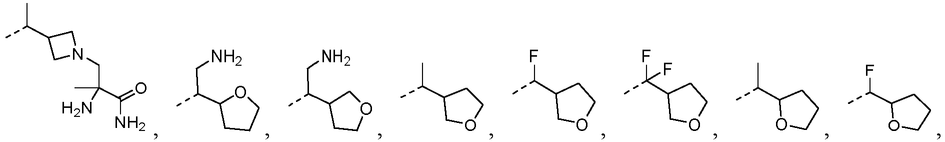 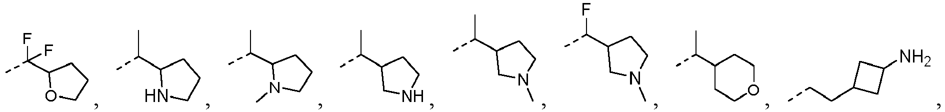 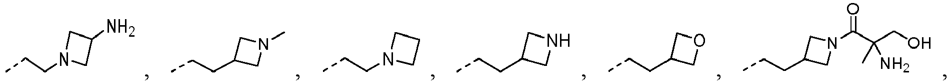 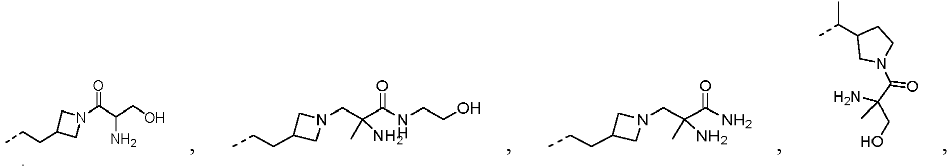 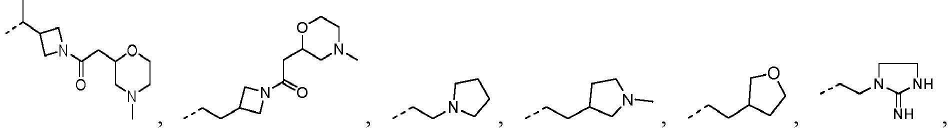 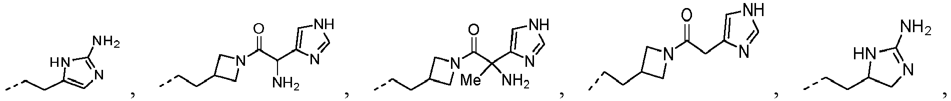 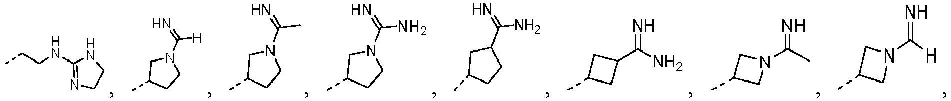 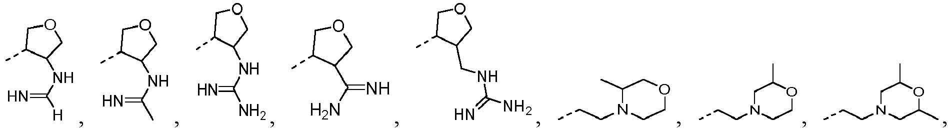 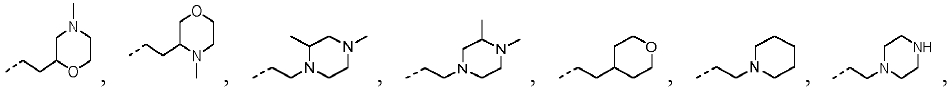 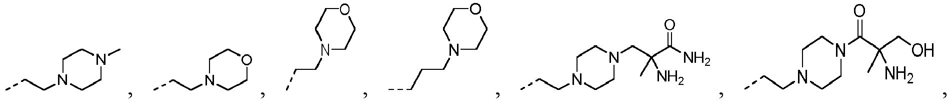 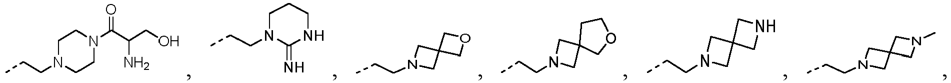 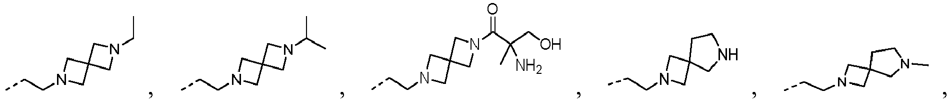 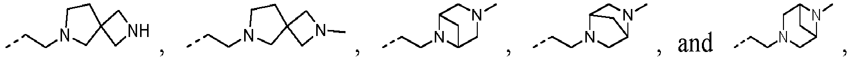 and the remaining variables are as defined in the present disclosure.

In the second aspect of the present disclosure, the present disclosure also provides a compound of the following formula, an optical isomer thereof, and a pharmaceutically acceptable salt thereof, selected from:

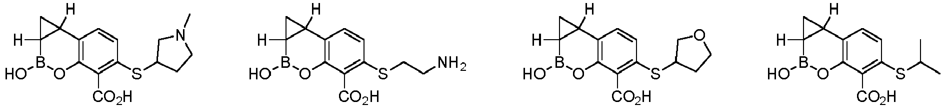

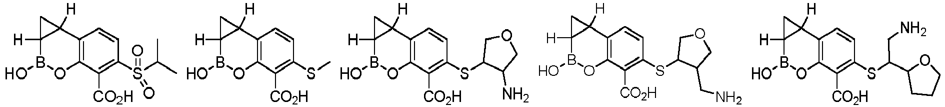

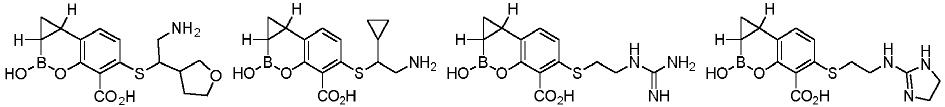

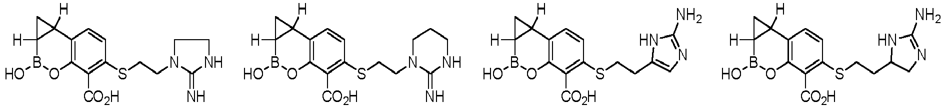

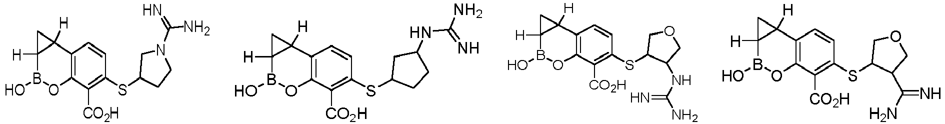

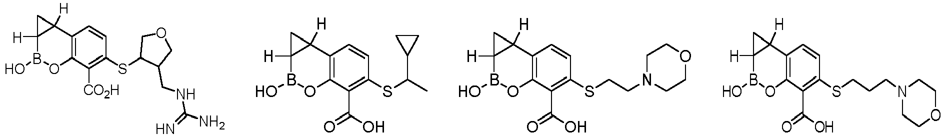

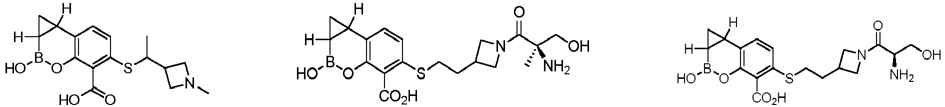

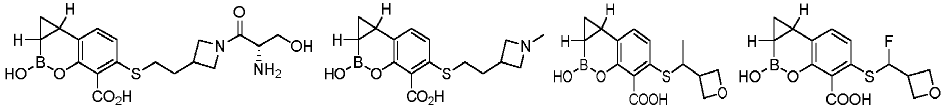

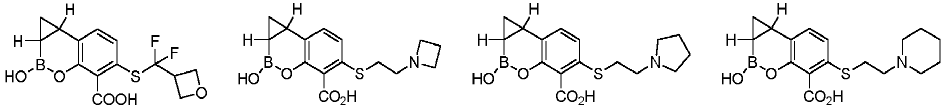

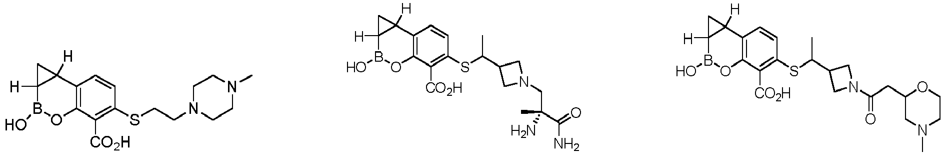

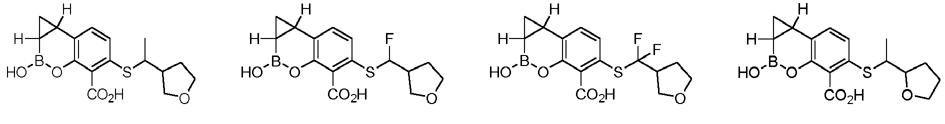

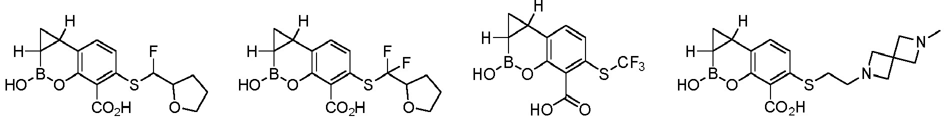

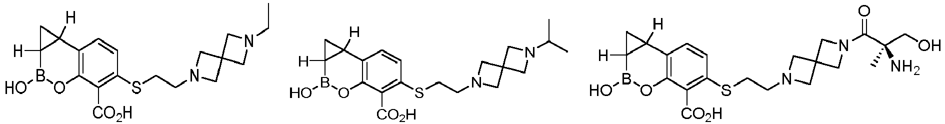

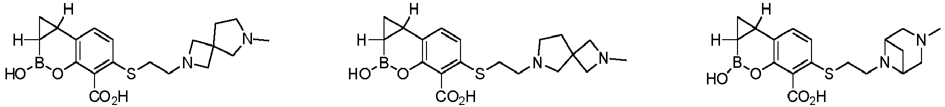

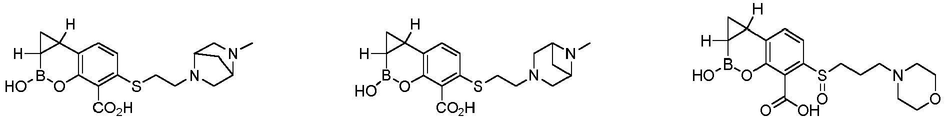

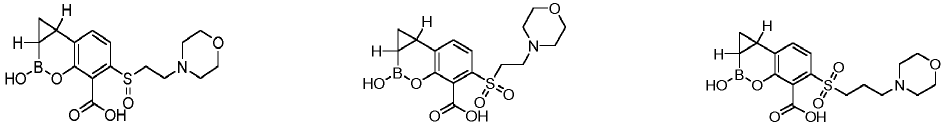

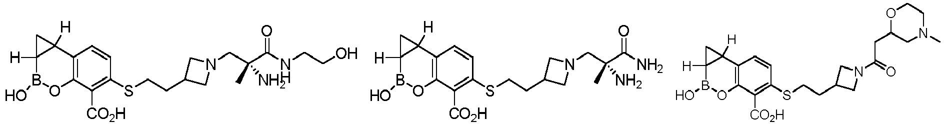

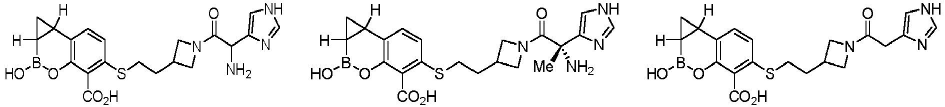

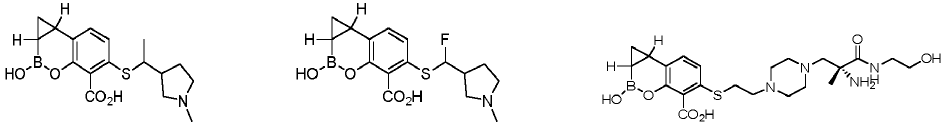

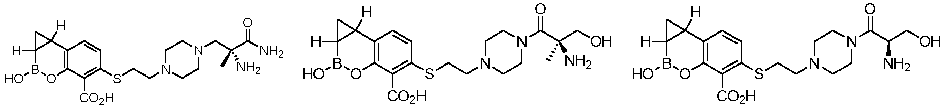

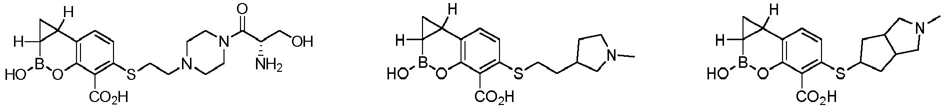

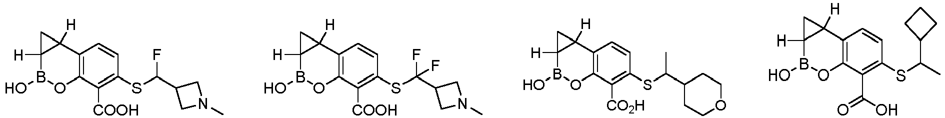

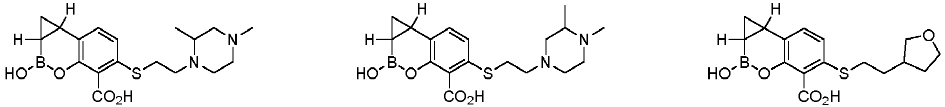

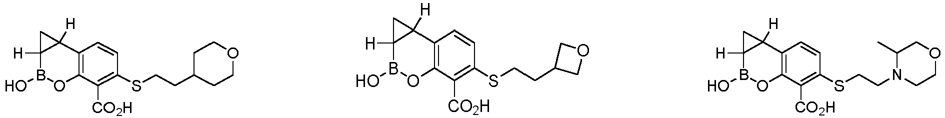

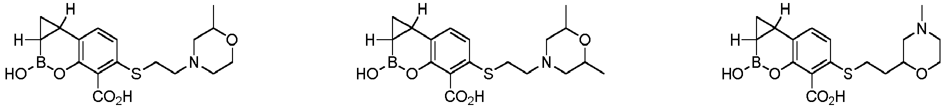

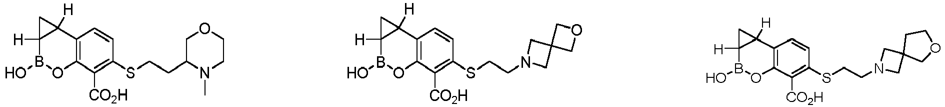

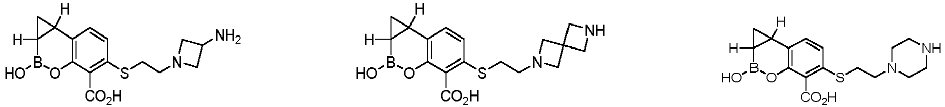

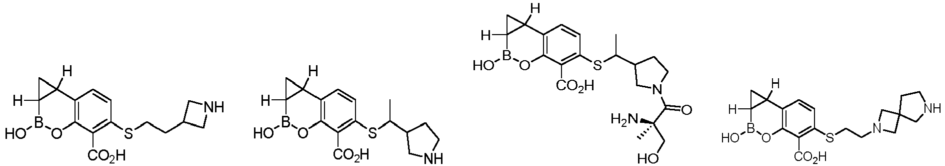

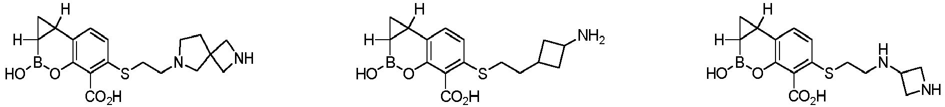

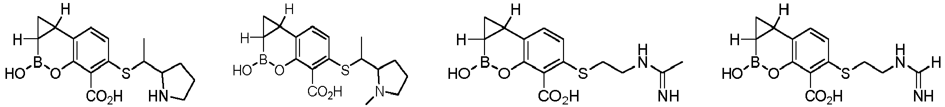

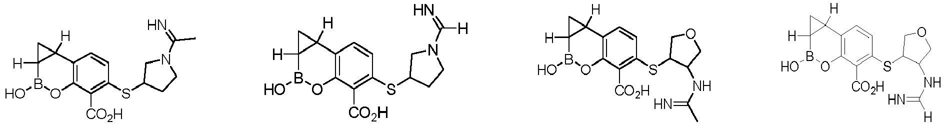

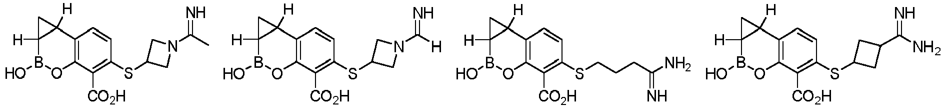

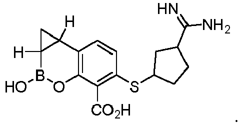

In another aspect of the present disclosure, the present disclosure also provides a compound of the following formula, an optical isomer thereof, and a pharmaceutically acceptable salt thereof, selected from:

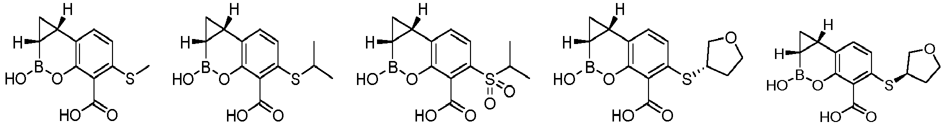

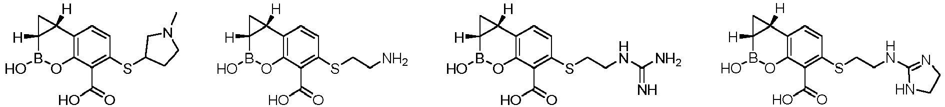

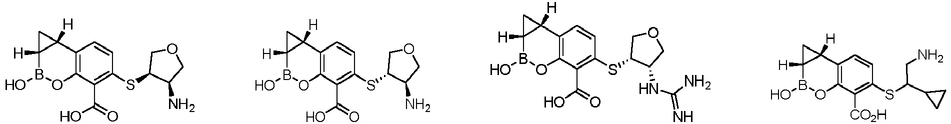

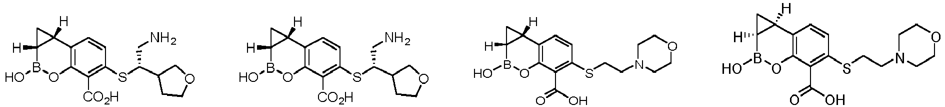

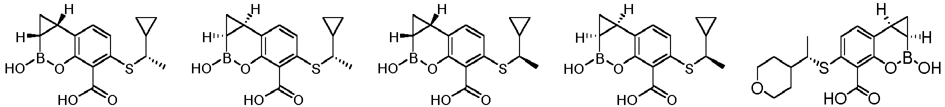

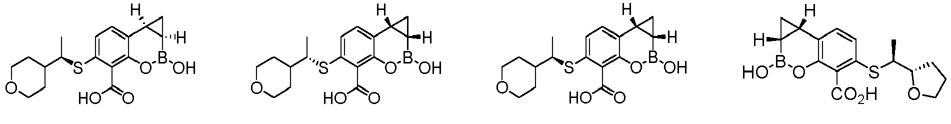

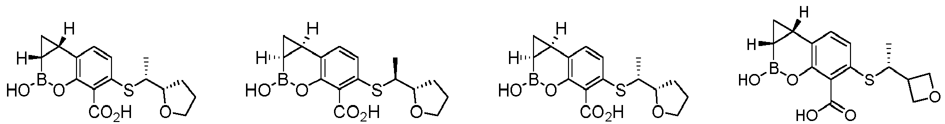

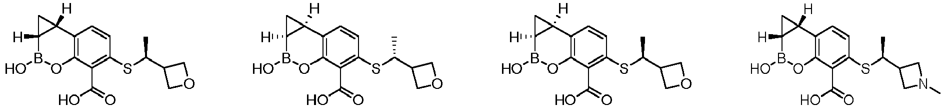

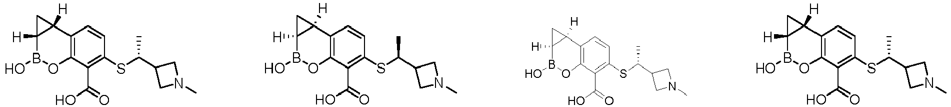

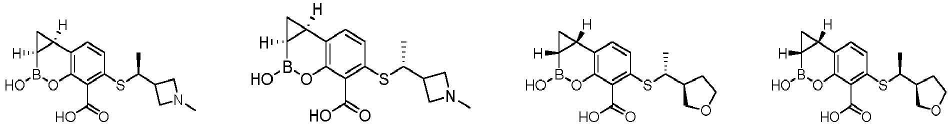

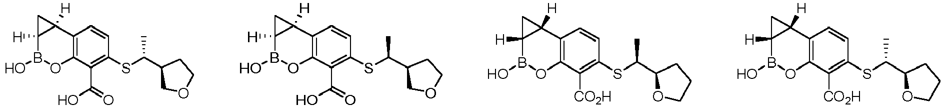

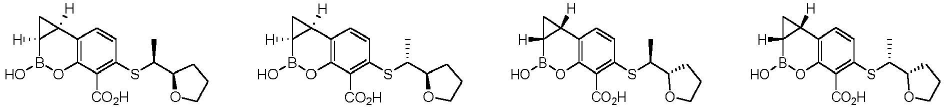

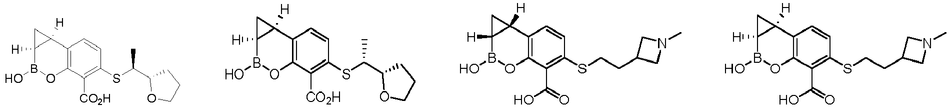

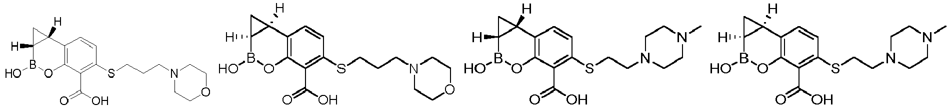

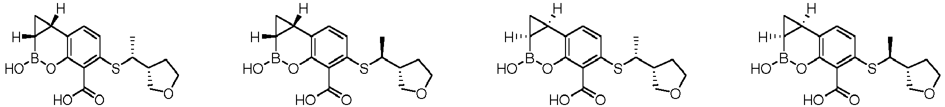

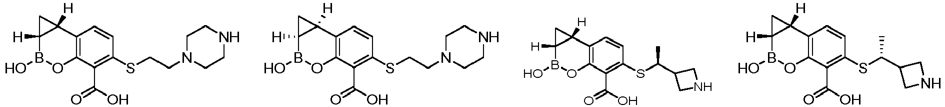

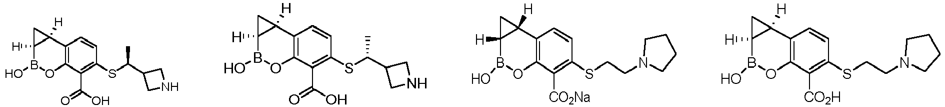

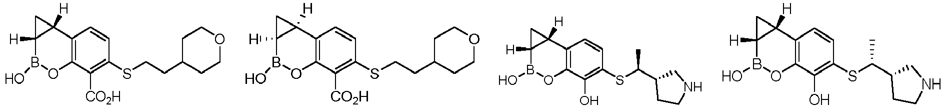

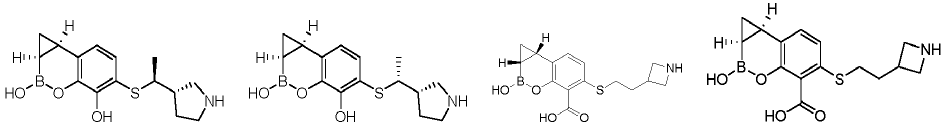

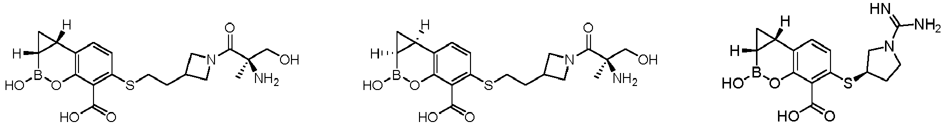

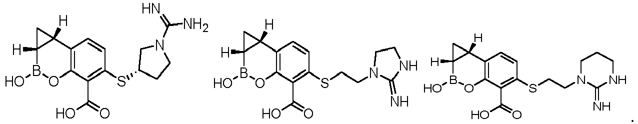

In yet another aspect of the present disclosure, the present disclosure also provides a use of the foregoing compound, the optical isomer thereof, and the pharmaceutically acceptable salt thereof in the manufacture of a medicament for the treatment of a bacterial infection disease.

In some embodiments of the present disclosure, the use further comprises a combination of the foregoing compound, the optical isomer thereof, and the pharmaceutically acceptable salt thereof with other β-lactam antibiotic.

In some embodiments of the present disclosure, the β-lactam antibiotic is selected from penicillin, cephalosporin, carbapenem, monocyclic β-lactam antibiotic, or a combination thereof.

In some embodiments of the present disclosure, the penicillin is selected from acid-resistant penicillin, enzyme-resistant penicillin, ampicillin, amoxicillin, pivampicillin, carbenicillin, sulbenicillin, ticarcillin, furbenicillin, azlocillin, and piperacillin.

In some embodiments of the present disclosure, the cephalosporin is selected from cefalexin, cefradine, cefazolin, cefuroxime, cefamandole, cefaclor, cefotaxime, ceftazidime, ceftriaxone, cefoperazone, ceftizoxime, cefepime, cefpirome, and ceftolozane.

In some embodiments of the present disclosure, the carbapenem is selected from imipenem, meropenem, panipenem, biapenem, ertapenem, and faropenem.

In some embodiments of the present disclosure, the monocyclic β-lactam is selected from aztreonam.

### Definition and description

Unless otherwise specified, the following terms and phrases when used herein have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

As used herein, the phrase "at least one" when referring to a list of one or more than one element should be understood to mean at least one element selected from any one or more elements in the list of elements, but not necessarily including at least one of each element specified in the list of elements, and not excluding any combination of elements in the list of elements. This definition also allows that elements may optionally be present other than the elements specifically identified within the list of elements to which the phrase "at least one" refers, whether related or unrelated to those elements specifically identified.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, other problems, or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine, magnesium, or similar salts. When the compound of the present disclosure contains a relatively basic functional group, an acid addition salt can be obtained by bringing the neutral form of the compound into contact with a sufficient amount of acid in a solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid; and an organic acid salt, wherein the organic acid includes, for example, acetic acid, propionic acid, isobutyric acid, trifluoroacetic acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; and salts of amino acid (such as arginine), and a salt of an organic acid such as glucuronic acid. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by a conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including cis and trans isomers, (-)- and (+)-enantiomers, (*R*)- and (*S*)-enantiomers, diastereomers, (*D*)-isomers, (*L*)-isomers, racemic mixtures, and other mixtures thereof, such as enantiomers or diastereomeric enriched mixtures, all of which are within the scope of the present disclosure. Additional asymmetric carbon atoms may be present in substituents such as alkyl. All these isomers and their mixtures are included within the scope of the present disclosure.

The compounds of the present disclosure may exist in specific. Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imine-enamine isomerization. Valence tautomer comprises some recombination of bonding electrons for mutual transformation. A specific example of keto-enol tautomerization is the tautomerism between two tautomers of pentane-2,4-dione and 4-hydroxypent-3-en-2-one.

The compound of the present disclosure may contain an unnatural proportion of atomic isotope at one or more than one atom that constitute the compound. For example, the compound can be radiolabeled with a radioactive isotope, such as tritium (³H), iodine-125 (¹²⁵I), or C-14 (¹⁴C). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs and the like. All isotopic variations of the compound of the present disclosure, whether radioactive or not, are encompassed within the scope of the present disclosure. "Optional" or "optionally" means that the subsequent event or condition may occur but not requisite, and the description includes the instance in which the event or condition occurs and the instance in which the event or condition does not occur.

When the valence bond of a group has a dashed line " 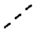 ", such as in " 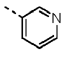 ", the dashed line indicates the linkage site of the group to the rest of the molecule. When there is " 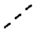 " on a single bond, for example, in " 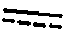 ", the dashed line represents a single bond or absent, and also means that " 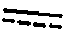 " represents a single bond " 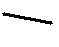 " or a double bond " 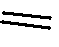 ".

The term "substituted" or "substituted by..." means one or more hydrogen atoms on a specific atom are substituted by the substituent, including deuterium and hydrogen variables, as long as the valence of the specific atom is normal and the substituted compound is stable. The term "optionally substituted" or "optionally substituted by..." means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 1, 2, or 3 R', the group can be optionally substituted by 1, 2, or 3 R', wherein the definition of R' at each occurrence is independent. Moreover, a combination of the substituent and/or the variant thereof is allowed only when the combination results in a stable compound.

When one of the variables is selected from a single bond, it means that the two groups linked by the single bond are linked directly. For example, when L₁ in 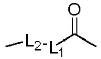 represents a single bond, the structure is actually

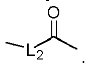

When the listed substituents do not indicate via which atom it is linked to the substituted group, this substituent can be bonded via any atom, for example, pyridyl, as a substituent, can link to the substituted group via any carbon atom on the pyridine ring.

When the listed linking group does not indicate the direction for linking, the direction for linking is arbitrary, for example, the linking group L contained in 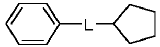 is - CH₂O-, then -CH₂O- can link phenyl and cyclopentyl to form 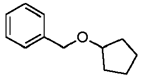 in the direction same as left-to-right reading order, and can link phenyl and cyclopentyl to form 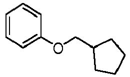 in the direction contrary to left-to-right reading order. A combination of the linking groups, substituents and/or variables thereof is allowed only when such combination can result in a stable compound.

Unless otherwise specified, the number of atoms on a ring is usually defined as the number of membered ring, such as a "3- to 6-membered ring" is a "ring" with 3 to 6 atoms arranged around it.

Unless otherwise specified, the term "C₁₋₆ alkyl" refers to a linear or branched saturated hydrocarbon group consisting of 1 to 6 carbon atoms. The C₁₋₆ alkyl includes C₁₋₅ alkyl, C₁₋₄ alkyl, C₁₋₃ alkyl, C₁₋₂ alkyl, C₂₋₆ alkyl, C₂₋₄ alkyl, C₆ alkyl, C₅ alkyl, etc.; it can be monovalent (such as CH₃), divalent (-CH₂-), or multivalent (such as 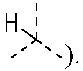 Examples of C₁₋₆ alkyl include, but are not limited to, CH₃, 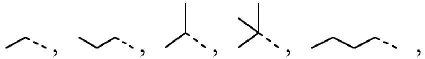 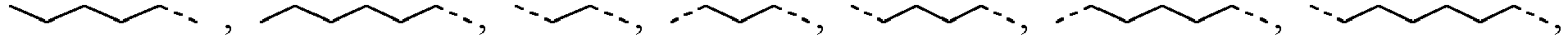 etc.

Unless otherwise specified, the term "C₁₋₄ alkyl" refers to a linear or branched saturated hydrocarbon group consisting of 1 to 4 carbon atoms. The C₁₋₄ alkyl includes C₁₋₂ alkyl, C₁₋₃ alkyl, C₃₋₄ alkyl, C₂₋₃ alkyl, etc.; it can be monovalent (such as CH₃), divalent (-CH₂-), or multivalent (such as 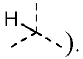 Examples of C₁₋₄ alkenyl include, but are not limited to, CH₃, 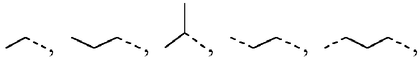 etc.

Unless otherwise specified, the term "heteroalkyl" by itself or in combination with another term means a stable linear or branched alkyl group or a combination thereof consisting of a certain number of carbon atoms, and at least one heteroatom or heteroatom group. In some embodiments, the heteroatom is selected from B, O, N, and S, wherein the nitrogen and sulfur atoms are optionally oxidized, and the nitrogen heteroatom is optionally quaternized. In other embodiments, the heteroatom group is selected from -C(=O)O-, -C(=O)-, -C(=S)-, - S(=O), -S(=O)₂-, -C(=O)N(H)-, -N(H)-, -C(=NH)-, -S(=O)₂N(H)-, and -S(=O)N(H)-. In some embodiments, the heteroalkyl is C₁₋₆ heteroalkyl; in other embodiments, the heteroalkyl is C₁₋₃ heteroalkyl. A heteroatom or heteroatom group may be located at any internal position within a heteroalkyl, including the site where the alkyl group is linked to the rest of the molecule, but the term "alkoxy" is a conventional expression referring to those alkyl groups linked to the rest of the molecule through an oxygen atom. Examples of heteroalkyl include, but are not limited to, -OCH₃, -OCH₂CH₃, -OCH₂CH₂CH₃, -OCH₂(CH₃)₂, -CH₂-CH₂-O-CH₃, -NHCH₃, -N(CH₃)₂, -NHCH₂CH₃, -N(CH₃)(CH₂CH₃), -CH₂-CH₂-NH-CH₃, -CH₂-CH₂-N(CH₃)-CH₃, -SCH₃, -SCH₂CH₃, -SCH₂CH₂CH₃, -SCH₂(CH₃)₂, -CH₂-S-CH₂-CH₃, -CH₂-CH₂, -S(=O)-CH₃, -CH₂-CH₂-S(=O)₂-CH₃, and up to two heteroatoms may be consecutive, e.g., - CH₂-NH-OCH₃.

Unless otherwise specified, the term "C₁₋₆ alkoxy" refers to an alkyl group containing 1 to 6 carbon atoms that are connected to the rest of the molecule through an oxygen atom. The C₁₋₆ alkoxy includes C₁₋₄ alkoxy, C₁₋₃ alkoxy, C₁₋₂ alkoxy, C₂₋₆ alkoxy, C₂₋₄ alkoxy, C₆ alkoxy, C₅ alkoxy, C₄ alkoxy, C₃ alkoxy, etc. Examples of C₁₋₆ alkoxy include, but are not limited to, methoxy, ethoxy, propoxy (including n-propoxy and isopropoxy), butoxy (including n-butoxy, isobutoxy, s-butoxy, and t-butoxy), pentyloxy (including n-pentyloxy, isopentyloxy, and neopentyloxy), hexyloxy, etc.

Unless otherwise specified, the term "C₁₋₃ alkoxy" refers to an alkyl group containing 1 to 3 carbon atoms that are connected to the rest of the molecule through an oxygen atom. The C₁₋₃ alkoxy includes C₁₋₃ alkoxy, C₁₋₂ alkoxy, C₂₋₃ alkoxy, C₁ alkoxy, C₂ alkoxy, C₃ alkoxy, etc. Examples of C₁₋₃ alkoxy include, but are not limited to, methoxy, ethoxy, propoxy (including n-propoxy and isopropoxy), etc.

Unless otherwise specified, the term "C₁₋₆ alkylamino" refers to an alkyl group containing 1 to 6 carbon atoms that are connected to the rest of the molecule through an amino group. The C₁₋₆ alkylamino includes C₁₋₄ alkylamino, C₁₋₃ alkylamino, C₁₋₂ alkylamino, C₂₋₆ alkylamino, C₂₋₄ alkylamino, C₆ alkylamino, C₅ alkylamino, C₄ alkylamino, C₃ alkylamino, C₂ alkylamino, etc. Examples of C₁₋₆ alkylamino include, but are not limited to, -NHCH₃, - N(CH₃)₂, -NHCH₂CH₃, -N(CH₃)CH₂CH₃, -N(CH₂CH₃)(CH₂CH₃), -NHCH₂CH₂CH₃, - NHCH₂(CH₃)₂, -NHCH₂CH₂CH₂CH₃, etc.

Unless otherwise specified, the term "C₁₋₃ alkylamino" refers to an alkyl group containing 1 to 3 carbon atoms that are connected to the rest of the molecule through an amino group. The C₁₋₃ alkylamino includes C₁₋₃ alkylamino, C₁₋₂ alkylamino, C₂₋₃ alkylamino, C₁ alkylamino, C₂ alkylamino, C₃ alkylamino, etc. Examples of C₁₋₃ alkylamino include, but are not limited to, -NHCH₃, -N(CH₃)₂, -NHCH₂CH₃, -N(CH₃)CH₂CH₃, -NHCH₂CH₂CH₃, - NHCH₂(CH₃)₂, etc.

Unless otherwise specified, the term "C₁₋₆ alkylthio" refers to an alkyl group containing 1 to 6 carbon atoms that are connected to the rest of the molecule through a sulfur atom. The C₁₋₆ alkylthio includes C₁₋₄ alkylthio, C₁₋₃ alkylthio, C₁₋₂ alkylthio, C₂₋₆ alkylthio, C₂₋₄ alkylthio, C₆ alkylthio, C₅ alkylthio, C₄ alkylthio, C₃ alkylthio, C₂ alkylthio, etc. Examples of C₁₋₆ alkylthio include, but are not limited to, -SCH₃, -SCH₂CH₃, -SCH₂CH₂CH₃, -SCH₂(CH₃)₂, etc.

Unless otherwise specified, the term "C₁₋₃ alkylthio" refers to an alkyl group containing 1 to 3 carbon atoms that are connected to the rest of the molecule through a sulfur atom. The C₁₋₃ alkylthio includes C₁₋₃ alkylthio, C₁₋₂ alkylthio, C₂₋₃ alkylthio, C₁ alkylthio, C₂ alkylthio, C₃ alkylthio, etc. Examples of C₁₋₃ alkylthio include, but are not limited to, - SCH₃, -SCH₂CH₃, -SCH₂CH₂CH₃, -SCH₂(CH₃)₂, etc.

Unless otherwise specified, the term "cycloalkyl" refers to a saturated or partially unsaturated monocyclic or polycyclic cyclic hydrocarbon substituent, the cycloalkyl ring comprising 3 to 20 carbon atoms, preferably 3 to 12 carbon atoms (which may be a specific point or an interval of any two points, e.g., 3, 4, 5, 6 ring atoms, 4 to 11 ring atoms, 6 to 12 ring atoms), more preferably comprising 3 to 8 carbon atoms, and most preferably comprising 3 to 6 (e.g., 3, 4, 5, or 6) carbon atoms. Non-limiting examples of the monocyclic cycloalkyl includes cyclopropyl, cyclobutyl, cyclopentyl, cyclopentenyl, cyclohexyl, cyclohexenyl, cyclohexadienyl, cycloheptyl, cycloheptatrienyl, cyclooctyl, etc., preferably cycloalkyl; and the polycyclic cycloalkyl includes cycloalkyl of spiro ring, fused ring, and bridged ring.

Unless otherwise specified, "C₃₋₆ cycloalkyl" refers to a saturated cyclic hydrocarbon group consisting of 3 to 6 carbon atoms, which is a monocyclic or bicyclic system, and the C₃₋₆ cycloalkyl includes C₃₋₅, C₄₋₅, and C₅₋₆ cycloalkyl, etc.; it can be monovalent, divalent, or multivalent. Examples of C₃₋₆ cycloalkyl include, but are not limited to, cyclopropyl, cyclobutyl, cyclopentyl, cyclohexyl, etc.

The term "heterocyclyl" refers to a saturated or partially unsaturated monocyclic or polycyclic cyclic hydrocarbon substituent comprising 3 to 20 ring atoms, wherein one or more than one ring atoms are heteroatoms selected from nitrogen, oxygen, or S(O)ₘ (wherein m is an integer from 0 to 2), but excluding a ring portion of -O-O-, -O-S-, or -S-S-, and the remaining ring atoms are carbon. The "heterocyclyl" preferably comprises 3 to 12 ring atoms (which may be a specific point or an interval of any two points, e.g., 3, 4, 5, 6 ring atoms, 4 to 11 ring atoms, 6 to 12 ring atoms), wherein 1 to 4 ring atoms are heteroatoms; preferably 3 to 8 ring atoms, wherein 1 to 3 ring atoms are heteroatoms; more preferably 3 to 6 ring atoms, wherein 1 to 3 ring atoms are heteroatoms. Non-limiting examples of monocyclic heterocyclyl include azetidinyl, pyrrolidinyl, imidazolidinyl, tetrahydrofuranyl, tetrahydropyranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, etc., preferably tetrahydropyranyl, piperidinyl, and pyrrolidinyl. Polycyclic heterocyclyl include heterocyclyl of spiro ring, fused ring, and bridged ring.

Unless otherwise specified, the term "3- to 6-membered heterocyclyl" by itself or in combination with other terms refers to a saturated cyclic group consisting of 3 to 6 ring atoms, wherein 1, 2, 3, or 4 ring atoms are heteroatoms independently selected from O, S, and N, and the rest are carbon atoms, wherein nitrogen atoms are optionally quaternized, and nitrogen and sulfur heteroatoms can be optionally oxidized (i.e., NO and S(O)ₚ, p is 1 or 2). It includes monocyclic and bicyclic systems, wherein the bicyclic system includes spiro rings, fused rings, and bridged rings. In addition, with regard to the "3- to 6-membered heterocyclyl", a heteroatom may occupy the connection position of the heterocyclyl with the rest of the molecule. The 3- to 6-membered heterocyclyl includes 4- to 6-membered, 5- to 6-membered, 4-membered, 5-membered, 6-membered heterocyclyl, etc. Examples of 3- to 6-membered heterocyclyl include, but are not limited to, azetidinyl, oxetanyl, thietidinyl, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxalkyl, dithianyl, isoxazolidinyl, isothiazolidinyl, 1,2-oxazinyl, 1,2-thiazinyl, hexahydropyridazinyl, homopiperazinyl, or homopiperidinyl, etc.

The term "aryl" refers to a 6- to 20-membered all-carbon monocyclic or fused polycyclic (i.e., rings sharing adjacent pairs of carbon atoms) group with a conjugated π-electron system, preferably 6- to 10-membered, more preferably 6-membered, such as phenyl and naphthyl. The aryl include the aryl group fused on heteroaryl, heterocyclyl, or a cycloalkyl ring, wherein the ring connected with the parent structure is an aryl ring, and non-limiting examples thereof include:

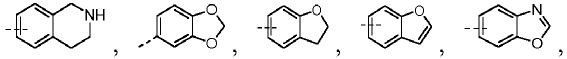

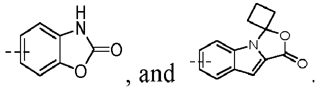

The aryl can be substituted or unsubstituted. When substituted, the substituent is preferably substituted with one or more than one of the following groups independently selected from one or more substituents of alkyl, alkenyl, alkynyl, alkoxy, alkylthio, alkylamino, halogen, mercapto, hydroxyl, nitro, cyano, cycloalkyl, heterocyclyl, aryl, heteroaryl, cycloalkoxy, heterocycloalkoxy, cycloalkylthio, and heterocycloalkylthio.

The term "heteroaryl" refers to a heteroaromatic system comprising 1 to 4 heteroatoms and 5 to 20 ring atoms, wherein the heteroatom is selected from oxygen, sulfur, and nitrogen. The heteroaryl is preferably 5- to 10-membered, containing 1 to 3 heteroatoms; more preferably 5-membered or 6-membered, containing 1 to 3 heteroatoms; non-limiting examples are pyrazolyl, imidazolyl, furyl, thienyl, thiazolyl, oxazolyl, pyrrolyl, triazolyl, tetrazolyl, pyridyl, pyrimidinyl, thiadiazolyl, pyrazinyl, etc. The heteroaryl ring can be fused to aryl, heterocyclyl, or a cycloalkyl ring, wherein the ring connected with the parent structure is a heteroaryl ring, and non-limiting examples thereof include:

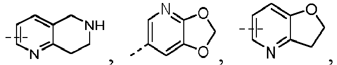

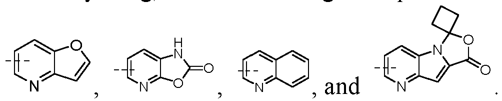

The heteroaryl can be optionally substituted or unsubstituted. When substituted, the substituent is preferably substituted with one or more than one of the following groups independently selected from one or more substituents of alkyl, alkenyl, alkynyl, alkoxy, alkylthio, alkylamino, halogen, mercapto, hydroxyl, nitro, cyano, cycloalkyl, heterocyclyl, aryl, heteroaryl, cycloalkoxy, heterocycloalkoxy, cycloalkylthio, and heterocycloalkylthio.

Unless otherwise specified, the terms "5- to 6-membered heteroaromatic ring" and "5-to 6-membered heteroaryl" in the present disclosure may be used interchangeably, and the term "5- to 6-membered heteroaryl" refers to a monocyclic group consisting of 5 to 6 ring atoms with conjugated π electronic system, wherein 1, 2, 3, or 4 ring atoms are heteroatoms independently selected from O, S, and N, and the rest are carbon atoms. The nitrogen atom is optionally quaternized, and the nitrogen and sulfur heteroatoms are optionally oxidized (i.e., NO and S(O)ₚ, p is 1 or 2). The 5- to 6-membered heteroaryl may be attached to the rest of the molecule through a heteroatom or a carbon atom. The 5- to 6-membered heteroaryl includes 5-membered and 6-membered heteroaryl. Examples of the 5- to 6-membered heteroaryl include, but are not limited to, pyrrolyl (including N-pyrrolyl, 2-pyrrolyl, 3-pyrrolyl, etc.), pyrazolyl (including 2-pyrazolyl, 3-pyrazolyl, etc.), imidazolyl (including *N*-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (1H 1,2,3-triazolyl, 2H-1,2,3-triazolyl, 1H-1,2,4-triazolyl, 4H-1,2,4-triazolyl, etc.), tetrazolyl, isoxazolyl (3-isoxazolyl, 4-isoxazolyl, 5-isoxazolyl, etc.), thiazolyl (including 2-thiazolyl, 4-thiazolyl, 5-thiazolyl, etc.), furanyl (including 2-furanyl, 3-furanyl, etc.), thienyl (including 2-thienyl, 3-thienyl, etc.), pyridinyl (including 2-pyridyl, 3-pyridyl, 4-pyridyl, etc.), pyrazinyl, or pyrimidinyl (including 2-pyrimidinyl, 4-pyrimidinyl, etc.).

Unless otherwise specified, Cₙ₋ₙ₊ₘ or Cₙ-Cₙ₊ₘ includes any specific instance of n to n+m carbons, for example, C₁₋₁₂ includes C₁, C₂, C₃, C₄, C₅, C₆, C₇, C₈, C₉, C₁₀, C₁₁, and C₁₂, also includes any range from n to n+m, for example, C₁₋₁₂ includes C₁₋₃, C₁₋₆, C₁₋₉, C₃₋₆, C₃₋₉, C₃₋₁₂, C₆₋₉, C₆₋₁₂, C₉₋₁₂, etc.; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, also includes any range from n to n+m, such as 3- to 12-membered ring includes 3- to 6-membered ring, 3- to 9-membered ring, 5- to 6-membered ring, 5- to 7-membered ring, 5- to 10-membered ring, 6- to 7-membered ring, 6- to 8-membered ring, 6- to 9-membered ring, and 6- to 10-membered ring.

The term "leaving group" refers to a functional group or atom which can be replaced by another functional group or atom through a substitution reaction (such as nucleophilic substitution reaction). For example, representative leaving groups include triflate; chlorine, bromine, and iodine; sulfonate group, such as mesylate, tosylate, p-bromobenzenesulfonate, p-toluenesulfonate; acyloxy, such as acetoxy, trifluoroacetoxy.

The term "protecting group" includes, but is not limited to, "amino protecting group", "hydroxyl protecting group", or "mercapto protecting group". The term "amino protecting group" refers to a protecting group suitable for preventing the side reactions occurring at the nitrogen of an amino. Representative amino protecting groups include, but are not limited to: formyl; acyl, such as alkanoyl (e.g., acetyl, trichloroacetyl, or trifluoroacetyl); alkoxycarbonyl, such as tert-butoxycarbonyl (Boc); arylmethoxycarbonyl such as benzyloxycarbonyl (Cbz) and 9-fluorenylmethoxycarbonyl (Fmoc); arylmethyl, such as benzyl (Bn), trityl (Tr), 1,1-bis-(4'-methoxyphenyl)methyl; silyl, such as trimethylsilyl (TMS) and tert-butyldimethylsilyl (TBS). The term "hydroxyl protecting group" refers to a protecting group suitable for preventing the side reactions of hydroxyl. Representative hydroxyl protecting groups include, but are not limited to: alkyl, such as methyl, ethyl, and tert-butyl; acyl, such as alkanoyl (e.g., acetyl); arylmethyl, such as benzyl (Bn), p-methoxybenzyl (PMB), 9-fluorenylmethyl (Fm), and diphenylmethyl (benzhydryl, DPM); silyl, such as trimethylsilyl (TMS) and tert-butyldimethylsilyl (TBS).

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The compounds of the present disclosure are named according to the conventional naming principles in the art or by ChemDraw^{®} software, and the commercially available compounds use the supplier catalog names.

### DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific embodiments have also been disclosed; for one skilled in the art, it is obvious to make various modifications and improvements to the embodiments of the present disclosure without departing from the spirit and scope of the present disclosure.

The ingredients used in the present disclosure are all commercially available unless otherwise specified.

The experimental materials and reagents used in the following examples can be obtained from commercially available sources unless otherwise specified.

The following abbreviations are used in the present disclosure: 9-BBN stands for 9-borabicyclo[3.3.1]nonane; Boc₂O stands for di-tert-butyl dicarbonate; B₂Pin₂ stands for bis(pinacolato)diboron; DCM stands for dichloromethane; DMAP stands for 4-dimethylaminopyridine; DMF stands for N,N-dimethylformamide; DIEA stands for N,N-diisopropylethylamine; DMP stands for Dess-Martin periodinane; HATU stands for 2-(7-azabenzotriazol-1-yl)-N,N,N',N'-tetramethyluronium hexafluorophosphate; HBF₄PCy₃ stands for tricyclohexyl tetrafluoroborate; LDA stands for lithium diisopropylamide; NaClO₂ stands for sodium chlorite; NFSI stands for N-fluorobenzenesulfonimide; NMM stands for N-methylmorpholine; PhI(OAc)₂ stands for (diacetoxyiodo)benzene; Pd(OAc)₂ stands for palladium acetate; Pd(dppf)Cl₂ [1,1'-bis(diphenylphosphino)ferrocene]dichloropalladium(II); THF stands for tetrahydrofuran; and Ti(OEt)₄ stands for ethyl titanate.

### Example 1: Synthesis of compound 1

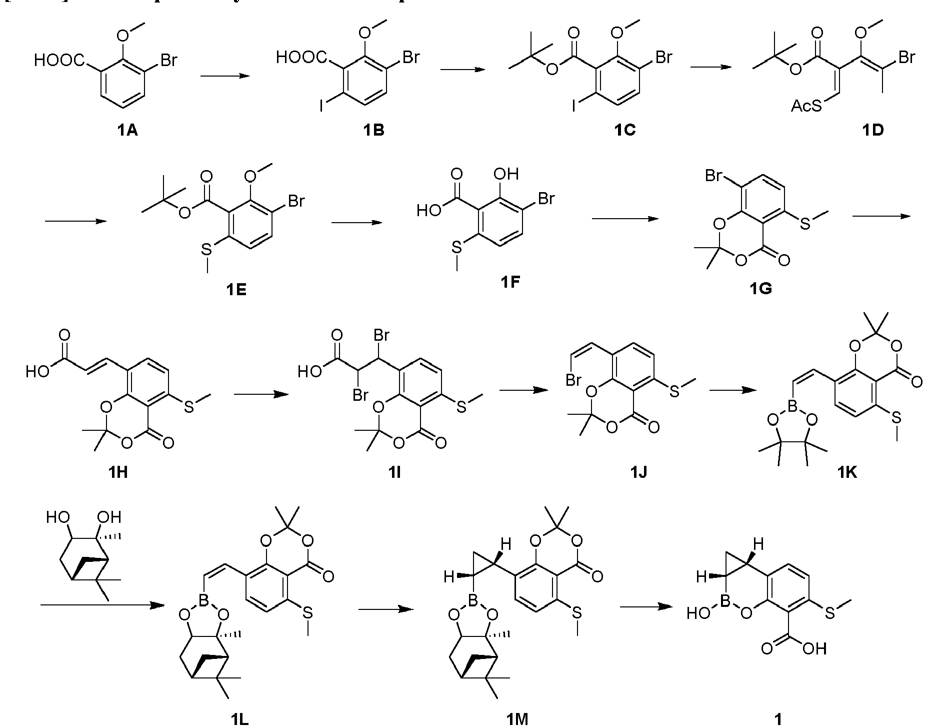

### Step 1: Preparation of compound 1B

Compound 1A (20 g, 86.96 mmol) was dissolved in DMF (200 mL), then (diacetoxyiodo)benzene (28 g, 86.96 mmol), I₂ (22 g, 86.96 mmol), and Pd(OAc)₂ (970 mg, 4.35 mmol) were added thereto, and the reaction mixture was reacted at 100°C for 16 hours. The reaction mixture was subjected to rotary evaporation until dryness to remove the solvent. Ethyl acetate was added thereto, and NaHSO₃ solution was add thereto and stirred, and the color changed from black to yellow. The reaction mixture was extracted twice with ethyl acetate, dried, filtered, subjected to rotary evaporation until dryness under reduced pressure, and separated by column chromatography (9/1 pentane/ethyl acetate) to obtain compound **1B** (22.4 g, 72%). ¹H NMR (400 MHz, DMSO-*d*₆) *δ* 13.79 (s, 1H), 7.56 (d, *J* = 8.4 Hz, 1H), 7.46 (d, *J* = 8.4 Hz, 1H), 3.80 (s, 3H).

### Step 2: Preparation of compound 1C

Under argon atmosphere, compound **1B** (13.8 g, 38.76 mmol) was dissolved in tert-butanol (20 mL), then DMAP (470 mg, 3.87 mmol) and Boc₂O (50 mL, 193.8 mmol) were added thereto and stirred at 80°C for 2 hours. The reaction mixture was subjected to rotary evaporation until dryness under reduced pressure and separated by column chromatography (9/1 pentane/ethyl acetate) to obtain compound **1C** (31.4 g, 87%). ¹H NMR (400 MHz, DMSO-*d*₆) δ 7.56 (d, *J* = 8.4 Hz, 1H), 7.47 (d, *J* = 8.4 Hz, 1H), 3.81 (s, 3H), 1.57 (s, 9H).

### Step 3: Preparation of compound 1D

Compound **1C** (6 g, 14.5 mmol, 1 eq) was dissolved in toluene (50 mL), then KSAc (6 g, 52 mmol, 3 eq), 1-10 phenanthroline (0.3 eq), and CuI (0.3 eq) were added thereto under N₂ atmosphere, and the reaction was carried out at 100°C for 2 days. The reaction mixture was subjected to rotary evaporation until dryness under reduced pressure and separated by column chromatography (9/1 pentane/ethyl acetate) to obtain compound **1D** (3.7 g, 54%). ¹H NMR (400 MHz, DMSO-*d*₆) δ 7.82 (d, *J* = 8.4 Hz, 1H), 7.23 (d, *J* = 8.4 Hz, 1H), 3.83 (s, 3H), 2.51 (q, *J* = 1.9 Hz, 3H), 1.52 (s, 9H).

### Step 4: Preparation of compound 1E

Compound **1D** (2.2 g, 6.11 mmol) was dissolved in methanol (30 mL), and cesium carbonate (3.0 g, 9.17 mmol) was added thereto.

The reaction was carried out at room temperature for 10 minutes under argon atmosphere. The substrate iodomethane (4.3 g, 30.56 mmol) was added thereto and stirred at 40°C for 2 hours. The inorganic salt was filtered through diatomite, subjected to rotary evaporation until dryness under reduced pressure and separated by column chromatography (9/1 pentane/ethyl acetate) to obtain compound **1E** (5.5 g, 85%). MS (ESI) m/z = 333.1 [M+H]⁺.

### Step 5: Preparation of compound 1F

Compound **1E** (5.5 g, 16.5 mmol) was dissolved in DCM (30 mL), and boron tribromide (50 mL, 50 mmol, 1 M dichloromethane solution) was added dropwise thereto at - 78°C under nitrogen atmosphere, and the reaction mixture was stirred at room temperature for 60 minutes. The reaction was quenched with deionized water (50 mL), the reaction mixture was subjected to rotary evaporation under reduced pressure to remove DCM, and the aqueous phase was extracted three times with ethyl acetate. The reaction mixture was dried, filtered and subjected to rotary evaporation until dryness under reduced pressure to obtain compound **1F** (2.6 g, 60%). MS (ESI) m/z = 263.0 [M+H]⁺.

### Step 6: Preparation of compound 1G

At room temperature, compound **1F** (2.6 g, 10.0 mmol) was added to trifluoroacetic acid (150 mL). At 80°C, acetone (3.5 g, 60 mmol, 44.1 mL) and trifluoroacetic anhydride (6.5 g, 31.2 mmol, 44.1 mL) were injected simultaneously with a syringe pump at a flow rate of 0.0306 mL/min. After the injection, the reaction mixture was concentrated, diluted with ethyl acetate, washed with saturated sodium bicarbonate aqueous solution, washed with water, and the organic phase was dried and concentrated, loaded with dry method, and subjected to silica gel column chromatography (9/1 petroleum ether/ethyl acetate) to obtain compound **1G** (1.3 g, 43%). MS (ESI) m/z = 305.1 [M+H]⁺.

### Step 7: Preparation of compound 1H

At room temperature, compound **1G** (1.0 g, 3.30 mmol), acrylic acid (713 mg, 9.90 mmol), tetrakis(triphenylphosphine)palladium (381 mg, 0.33 mmol) and triethylamine (1.66 g, 16.5 mmol) were added into dry DMF (30 mL), and the reaction mixture was stirred at 120°C for about 6 hours under argon atmosphere. The reaction mixture was filtered through diatomite, and the filtrate was concentrated, loaded with dry method, and subjected to silica gel column chromatography (9/1 petroleum ether/ethyl acetate) to obtain compound **1H** (0.5 g, 52%). MS (ESI) m/z = 295.2 [M+H]⁺.

### Step 8: Preparation of compound 11

At 0°C, Br₂ (0.92 g, 5.75 mmol) was added dropwise to a solution of compound **1H** (1.7 g, 5.25 mmol) in chloroform (40 mL) and reacted for 0.5 hours. LCMS showed that the raw materials disappeared. The reaction mixture was concentrated to obtain compound **11** (2.6 g, 100%), which was directly used in the next step.

### Step 9: Preparation of compound 1J

Et₃N (0.64 g, 6.3 mmol) was added dropwise to a solution of compound **1I** (2.6 g, 5.25 mmol) in DMF (30 mL) at 0°C, and the mixture was warmed to room temperature and reacted for 2 hours. The reaction mixture was added with ethyl acetate, washed with 1 M HCl for three times, and the organic phase was concentrated and purified, and separated by column chromatography (0-5% ethyl acetate/pentane) to obtain compound **1J** (580 mg, 31%). ¹H NMR (400 MHz, CDCl₃) δ 8.20 (d, *J* = 8.6 Hz, 1H), 7.13 (d, *J* = 8.1 Hz, 1H), 6.95 (d, *J* = 8.6 Hz, 1H), 6.50 (d, *J* = 8.1 Hz, 1H), 2.49 (s, 3H), 1.73 (s, 6H). MS (ESI) m/z = 329.0 [M+H]⁺.

### Step 10: Preparation of compound 1K

At room temperature, B₂pin₂ (637 mg, 2.5 mmol), KOAc (491 mg, 5.0 mmol) and Pd(PPh₃)₄ (193 mg, 0.17 mmol) were added to a solution of compound **1J** (550 mg, 1.7 mmol) in dioxane (8 mL) in sequence, and the system was replaced with argon three times. The reaction mixture was placed in a 70°C oil bath and reacted for 2 hours. The reaction mixture was filtered, concentrated and purified, and separated by column chromatography (0-10% ethyl acetate/pentane) to obtain compound **1K(510** mg, 81%). ¹H NMR (400 MHz, CDCl₃) δ 7.91 (d, *J* = 8.5 Hz, 1H), 7.29 (d, *J* = 14.8 Hz, 1H), 6.84 (d, *J* = 8.6 Hz, 1H), 5.65 (d, *J* = 14.9 Hz, 1H), 2.48 (s, 3H), 1.72 (s, 6H), 1.28 (s, 12H). MS (ESI) m/z = 377.2 [M+H]⁺.

### Step 11: Preparation of compound 1L

(1S,2S,3R,5S)-(+)-2,3-Pinanediol (461 mg, 2.7 mmol) was added to a solution of compound **1K** (510 mg, 1.4 mmol) in THF (8 mL), and the reaction mixture was placed in a 60°C oil bath and reacted for 2 hours. The reaction mixture was concentrated and purified, and separated by column chromatography (0-10% ethyl acetate/pentane) to obtain compound **1L** (320 mg, 55%). ¹H NMR (400 MHz, CDCl₃) δ 7.92 (dd, *J* = 8.5 Hz, 1H), 7.31 (d, *J* = 14.9 Hz, 1H), 6.85 (d, *J* = 8.5 Hz, 1H), 5.69 (d, *J* = 14.9 Hz, 1H), 4.33 (dd, *J* = 8.8, 2.0 Hz, 1H), 2.48 (s, 3H), 2.39 - 2.31 (m, 1H), 2.27 - 2.20 (m, 1H), 2.07 (t, *J* = 5.5 Hz, 1H), 1.95 - 1.92 (m, 1H), 1.88 - 1.83 (m, 1H), 1.73 - 1.71 (m, 6H), 1.41 (s, 3H), 1.30 (s, 3H), 1.17 (d, *J* = 10.9 Hz, 1H), 0.85 (s, 3H). MS (ESI) m/z = 429.2 [M+H]⁺.

### Step 12: Preparation of compound 1M

An ether solution of diazomethane (50 mL, about 0.8 M) was added to compound **1L** (320 mg, 0.75 mmol) and Pd(OAc)₂ in THF(4 mL) and reacted at room temperature for 4 hours. The reaction mixture was filtered, concentrated and purified, and separated by column chromatography (0-10% ethyl acetate/pentane) to obtain crude product (250 mg), which was prepared by HPLC to obtain compound **1M** (130 mg, 39%). MS (ESI) m/z = 443.2 [M+H]⁺.

### Step 13: Preparation of compound 1

NaOH (1 mL, 3 N aqueous solution) was added to compound **1M** (110 mg, 0.25 mmol) in dioxane (2 mL), and the reaction mixture was placed in a 40°C oil bath and reacted for 2 hours. LCMS showed that the raw materials disappeared. The reaction mixture was cooled to 0°C, then TES (1 mL), TFA (5 mL), and isobutylboronic acid (200 mg) were added thereto in sequence, and the reaction mixture was slowly warmed to room temperature and reacted for 1 hour. The reaction mixture was concentrated, alkalized with a 3 N NaOH aqueous solution until the pH reached 10, concentrated, and then prepared by alkaline HPLC to obtain compound **1** (32 mg, 52%). ¹H NMR (400 MHz, D₂O) δ 7.07 (d, *J* = 7.8 Hz, 1H), 6.68 (d, *J* = 7.8 Hz, 1H), 2.33 (s, 3H), 1.77 - 1.72 (m, 1H), 0.80 - 0.75 (m, 1H), 0.28 - 0.25 (m, 1H), 0.22 - 0.16 (m, 1H). MS (ESI) m/z = 232.8 [M+ H-18]⁺.

### Example 2: Synthesis of compound 2

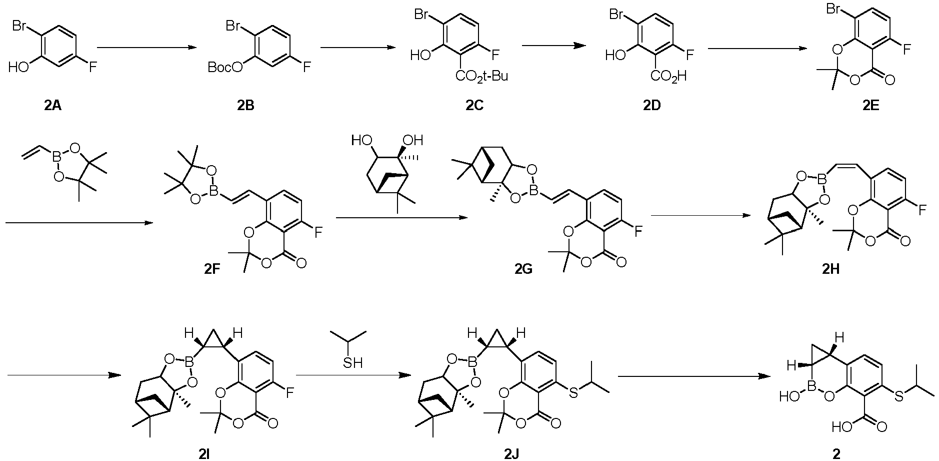

### Step 1: Preparation of compound 2B

At room temperature, compound **2A** (50.0 g, 262 mmol) was dissolved in dichloromethane (300 mL), then DMAP (3.20 g, 26.2 mmol) was added thereto, and then Boc₂O (66.2 mL, 288 mmol) was slowly added dropwise thereto. After the dropwise addition was completed, the reaction mixture was stirred at room temperature for 2 hours, then saturated NaHCO₃ solution and dichloromethane were added to the reaction mixture for phase separation. The organic phase was washed twice with saturated NaHCO₃ solution, then washed once with water, twice with 2 M citric acid, and twice with saturated brine in sequence. The organic phase was dried with anhydrous sodium sulfate, filtered, and concentrated to obtain compound **2B** (73.5 g, 97%), which was directly used in the next reaction step. ¹H NMR (400 MHz, CDCl₃) δ 7.55 (dt, J = 12.1, 6.1 Hz, 1H), 7.00 (dd, J = 8.7, 2.9 Hz, 1H), 6.89 (ddd, J = 8.8, 7.8, 2.9 Hz, 1H), 1.57 (s, 9H).

### Step 2: Preparation of compound 2C

At room temperature, compound **2B** (73.5 g, 252 Mmol) was dissolved in ultra-dry tetrahydrofuran (400 mL), and the system was replaced with nitrogen for three times, then cooled to -65°C with dry ice acetone, and LDA solution (132 mL, 264 mmol, 2 M tetrahydrofuran solution) was slowly added dropwise thereto. During the dropping process, the temperature was ensured not to exceed -60°C. After the dropwise addition was completed, the reaction mixture was stirred at this temperature for 4 hours, slowly warmed to 0°C, and saturated ammonium chloride solution (200 mL) was added thereto. The phases were separated, the aqueous phase was extracted with ethyl acetate for three times, and the combined organic phases were washed twice with saturated brine, dried with anhydrous sodium sulfate, filtered and concentrated to obtain compound **2C** (65.0 g, 88%), which was directly used in the next reaction step. ¹H NMR (400 MHz, DMSO-d₆) δ 7.56 (dd, J = 8.8, 6.4 Hz, 1H), 6.45 (t, J = 9.2 Hz, 1H), 1.51 (d, J = 6.2 Hz, 9H).

### Step 3: Preparation of compound 2D

At room temperature, compound **2C** (65.0 g, 223 mmol) was dissolved in dichloromethane (200 mL), then trifluoroacetic acid (100 mL) was added thereto, and the mixture was stirred at room temperature for 2 hours and concentrated to remove the solvent. Then the reaction mixture was slurried with dichloromethane (50 mL), filtered, and the filter residue was washed twice with a small amount of dichloromethane to obtain compound **2D** (40.0 g, 76%). ¹H NMR (400 MHz, DMSO-d₆) δ 7.79 (dd, J = 8.9, 5.7 Hz, 1H), 6.77 (dd, J = 10.4, 8.9 Hz, 1H).

### Step 4: Preparation of compound 2E

At room temperature, trifluoroacetic acid (200 mL) was added to compound **2D** (40.0 g, 170 mmol), and the raw material was insoluble. The system was replaced with nitrogen for three times. Then trifluoroacetic anhydride (71.0 mL, 511 mmol) was added thereto with a syringe, and the mixture was stirred at room temperature for 10 minutes. Then acetone (62.6 mL, 851 mmol) was slowly added thereto with a syringe, and the temperature was slowly raised to 100 °C (external temperature, internal temperature about 85°C). The raw materials were gradually dissolved and the solution turned yellow. At this temperature, the reaction mixture was stirred for 48 hours. The reaction mixture was cooled to room temperature, concentrated to remove the solvent, then ethyl acetate (300 mL) and saturated NaHCO₃ solution (100 mL) were added thereto. The phases were separated, and the aqueous phase was extracted with ethyl acetate for three times. The combined organic phases were washed with saturated NaHCO₃ solution and saturated brine in sequence, dried with anhydrous sodium sulfate, filtered and concentrated to obtain product **2E** (37.5 g, 80%). ¹H NMR (400 MHz, CDCl₃) δ 7.74 (dd, J = 9.0, 5.3 Hz, 1H), 6.80 (t, J = 9.2 Hz, 1H), 1.79 (s, 6H). MS (ESI) m/z = 275.0 [M+H]⁺.

### Step 5: Preparation of compound 2F

Compound **2E** (500 mg, 1.818 mmol), pinacol vinylboronate (560 mg, 3.636 mmol), palladium acetate (40.8 mg, 0.182 mmol), SPhos (149 mg, 0.364 mmol) and triethylamine (550.8 mg, 5.45 mmol) were added to dioxane (6.0 mL), and the reaction was completed for 16 hours under argon atmosphere at 80°C (LC-MS detection). The reaction mixture was directly concentrated, loaded with dry method, and subjected to silica gel column chromatography to obtain compound **2F** (400 mg, 63%). MS (ESI) m/z = 349.2 [M+H]⁺.

### Step 6: Preparation of compound 2G

Compound **2F** (400 mg, 1.149 mmol) and pinol (782 mg, 4.60 mmol) were added into tetrahydrofuran (20.0 mL), and the reaction was completed at 65°C for 4 hours (LC-MS detection). The reaction mixture was directly concentrated and subjected to silica gel column chromatography to obtain compound **2G** (400 mg, 88%). ¹H NMR (400 MHz, CDCl₃) δ 7.77 (dd, J = 8.8, 5.8 Hz, 1H), 7.47 (d, J = 18.6 Hz, 1H), 6.85 (t, J = 9.2 Hz, 1H), 6.19 (d, J = 18.6 Hz, 1H), 4.39 (dd, J = 8.7, 1.7 Hz, 1H), 2.46 - 2.35 (m, 1H), 2.31 - 2.21 (m, 1H), 2.16 - 2.08 (m, 1H), 2.00 - 1.91 (m, 2H), 1.78 (d, J = 2.9 Hz, 6H), 1.59 (d, J = 8.0 Hz, 1H), 1.45 (d, J = 6.5 Hz, 3H), 1.32 (s, 3H), 0.88 (s, 3H). MS (ESI) m/z = 401.2 [M+H]⁺.

### Step 7: Preparation of compound 2H

At room temperature, compound **2G** (200 mg, 0.500 mmol) was dissolved in dichloromethane (5.00 mL), then IrPPy3 (33 mg, 0.050 mmol) was added thereto. The system was replaced with nitrogen for three times, and irradiated with blue light (460 to 465 nm, 10 w) for 4 hours at room temperature, and the reaction mixture was directly mixed with silica gel and subjected to column chromatography (0%-10% EA/PE) to obtain the compound **2H** (120 mg, 60%). ¹H NMR (400 MHz, DMSO-d₆) δ 7.92-7.83 (m, 1H), 7.23 (d, J = 12.0 Hz, 1H), 7.11-7.01 (m, 1H), 5.73 (d, J = 12.0 Hz, 1H), 4.39-4.33 (m, 1H), 2.35-2.25 (m, 1H), 2.23-2.15 (m, 1H), 1.98-1.92 (m, 1H), 1.90-1.84 (m, 1H), 1.78-1.66 (m, 8H), 1.32 (s, 3H), 1.26 (s, 3H), 0.81 (s, 3H). MS (ESI) m/z = 401.2 [M+H]⁺.

### Step 8: Preparation of compound 21

At room temperature, compound **2H** (13.1 g, 32.8 mmol) was dissolved in ultra-dry THF (79 mL), then Pd(OAc)₂ (368 mg, 1.64 mmol) was added thereto, and the system was replaced with argon three times, cooled to about 0°C. An ether solution of diazomethane (200 mL, about 0.6 M) was slowly added thereto with a syringe, and the reaction mixture turned from yellow to black, and bubbles were generated. The reaction mixture was slowly warmed to room temperature and stirred for 2 hours. The reaction mixture was concentrated at room temperature and purified by column chromatography (0-10% EA/PE) to obtain the crude compound (10.9 g), which was prepared by acid HPLC to obtain compound **21** (4.3 g, 31%). MS (ESI) m/z = 415.0 [M+H]⁺.

### Step 11: Preparation of compound 2J

At room temperature, compound **21** (150 mg, 0.377 mmol) was dissolved in dimethyl sulfoxide (2 mL), then cesium carbonate (246 mg, 0.754 mmol) was added thereto, and the system was replaced with argon three times. Then 2-propanethiol (86.1 mg, 1.13 mmol) was added to the system, and stirred at 60°C overnight. The reaction mixture was cooled to room temperature, diluted with ethyl acetate (10 mL), extracted with water (3 mL) for three times, and the combined organic phases were washed with brine (3 mL), concentrated to obtain a crude product, which was purified by column chromatography (0-8% EA/PE) to obtain compound 2J (64 mg, 36%). MS (ESI) m/z = 471.1 [M+H]⁺.

### Step 12: Preparation of compound 2

At room temperature, compound **2J** (150 mg, 0.377 mmol) was dissolved in 1,4-dioxane (1 mL), then 3 M sodium hydroxide aqueous solution (1 mL, 0.816 mmol) was added thereto, and the mixture was stirred for 3 hours at room temperature. TLC plate showed that the raw materials was reacted completely. Triethoxysilane (0.126 mL, 112 mg, 0.681 mmol), trifluoroacetic acid (0.628 mL) and isobutylboronic acid (27.7 mg, 0.272 mmol) were added to the system in sequence, and the reaction mixture was stirred at room temperature for half an hour, filtered, concentrated, added with sodium bicarbonate aqueous solution to adjust the pH to 5, and concentrated to obtain a crude product, which was prepared and purified by HPLC to obtain compound 2 (12.8 mg, 34%). ¹H NMR (400 MHz, D₂O) δ 6.83 (d, *J* = 7.7 Hz, 1H), 6.54 (d, *J* = 7.7 Hz, 1H), 3.05 (dt, *J* = 13.3, 6.5 Hz, 1H), 1.54 (t, *J* = 6.7 Hz, 1H), 0.92 (d, *J* = 6.7 Hz, 6H), 0.58 (t, *J* = 7.3 Hz, 1H), 0.06 (s, 1H), -0.01 (dd, *J* = 15.9, 9.0 Hz, 1H). MS (ESI) m/z = 279.1 [M+H]⁺.

### Example 3: Synthesis of compound 3

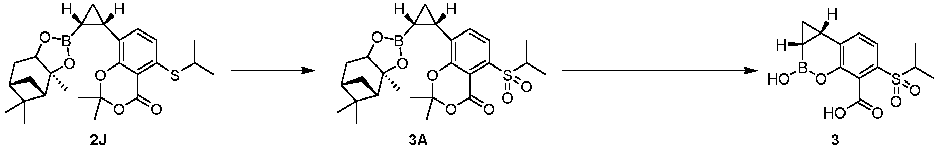

### Step 1: Preparation of compound 3A

At room temperature, compound **2J** (95.0 mg, 0.229 mmol) was dissolved in dichloromethane (2.00 mL), then m-chloroperoxybenzoic acid (119 mg, 0.688 mmol) was added thereto, and the mixture was stirred overnight at room temperature. The reaction mixture was diluted with ethyl acetate (10.0 mL), quenched with an appropriate amount of saturated sodium thiosulfate aqueous solution, and washed three times with saturated sodium bicarbonate solution (3.0 mL) and once with water (3.0 mL). The combined organic phases were washed with brine (3.0 mL) and concentrated to obtain a crude product, which was purified by column chromatography (8-10% EA/PE) to obtain compound **3A** (35 mg, 30%). MS (ESI) m/z = 503.1 [M+H]⁺.

### Step 2: Preparation of compound 3

Prepared according to the synthesis scheme of compound **2** in Example 2. Product **3** (6.8 mg, 37%) was obtained from compound **3A** (30 mg, 0.0597 mmol). ¹H NMR (400 MHz, D₂O) δ 7.37 (dd, *J* = 8.1, 3.1 Hz, 1H), 7.21 (dd, *J* = 8.1, 3.5 Hz, 1H), 3.79 - 3.58 (m, 1H), 2.26 - 2.02 (m, 1H), 1.18 (dd, *J* = 6.6, 3.0 Hz, 6H), 1.17 - 1.08 (m, 1H), 0.50 (d, *J* = 8.4 Hz, 1H), 0.44 (dd, *J* = 10.5, 7.3 Hz, 1H). MS (ESI) m/z = 311.1 [M+H]⁺.

### Example 4: Synthesis of compound 4

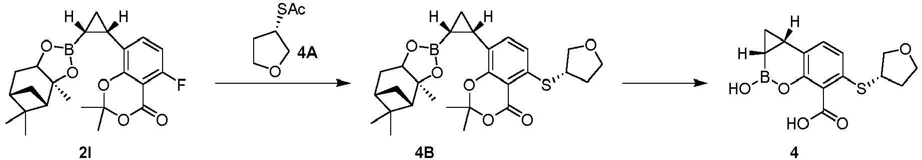

### Step 1: Preparation of compound 4B

Prepared according to the synthesis scheme of compound **2J** in Example 2. Product **4B** (120 mg, 99%) was obtained from compound **21** (100 mg, 0.241 mmol) and compound **4A** (236 mg, 1.61 mmol). MS (ESI) m/z = 499.1 [M+H]⁺.

### Step 2: Preparation of compound 4

Prepared according to the synthesis scheme of compound 2 in Example 2. Product **4** (36.8 mg, 40%) was obtained from compound **4B** (150 mg, 0.301 mmol). ¹H NMR (400 MHz, D₂O+NaOH) δ 6.84 (d, J = 7.8 Hz, 1H), 6.55 (d, J = 7.8 Hz, 1H), 3.71 - 3.64 (m, 2H), 3.56 (ddd, J = 16.4, 9.3, 4.8 Hz, 2H), 3.44 (dd, J = 9.3, 3.8 Hz, 1H), 1.98 (dd, J = 13.2, 7.6 Hz, 1H), 1.69 - 1.49 (m, 2H), 0.58 (t, J = 7.3 Hz, 1H), 0.12 - 0.04 (m, 2H). MS (ESI) m/z = 307.1 [M+H]⁺.

### Example 5: Synthesis of compound 5

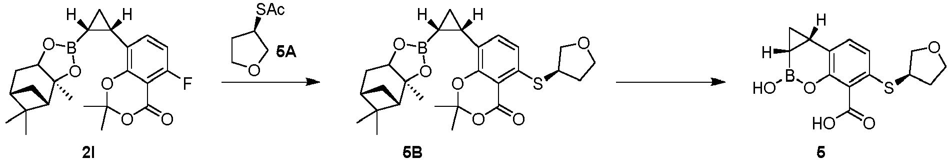

### Step 1: Preparation of compound 5B

Prepared according to the synthesis scheme of compound **2J** in Example 2. Product 5B (210 mg, 87%) was obtained from compound **21** (200 mg, 0.483 mmol) and compound **5A** (212 mg, 1.45 mmol). MS (ESI) m/z = 499.1 [M+H]⁺.

### Step 2: Preparation of compound 5

Prepared according to the synthesis scheme of compound **2** in Example 2. Product **5** (91.5 mg, 78%) was obtained from compound **5B** (190 mg, 0.381 mmol). ¹H NMR (400 MHz, D₂O+NaOH) δ 7.08 (d, J = 7.8 Hz, 1H), 6.79 (d, J = 7.8 Hz, 1H), 3.97 - 3.86 (m, 2H), 3.80 (qd, J = 8.3, 4.9 Hz, 2H), 3.69 (dd, J = 9.3, 3.7 Hz, 1H), 2.22 (dd, J = 13.4, 7.4 Hz, 1H), 1.82 (dd, J = 26.8, 4.3 Hz, 2H), 0.82 (dd, J = 12.1, 4.8 Hz, 1H), 0.37 - 0.28 (m, 1H), 0.28 - 0.18 (m, 1H). MS (ESI) m/z = 307.1 [M+H]⁺.

### Example 6: Synthesis of compound 6

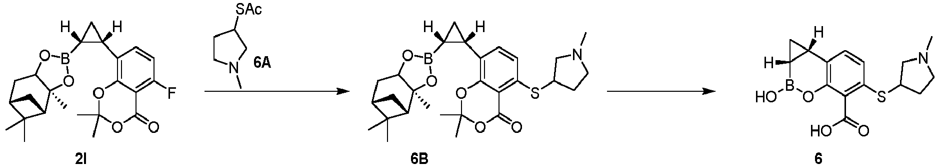

### Step 1: Preparation of compound 6B

Prepared according to the synthesis scheme of compound 2J in Example 2. Product **6B** (144 mg, 78%) was obtained from compound **21** (150 mg, 0.36 mmol) and compound **6A** (288 mg, 1.81 mmol). MS (ESI) m/z = 512.1 [M+H]⁺.

### Step 2: Preparation of compound 6

Prepared according to the synthesis scheme of compound **2** in Example 2. Product **6** (8.4 mg, 9%) was obtained from compound **6B** (144 mg, 0.282 mmol). ¹H NMR (400 MHz, D2O) δ 7.15 (d, J = 7.9 Hz, 1H), 6.89 (d, J = 7.7 Hz, 1H), 4.01 (s, 1H), 3.77 (s, 1H), 3.44 (s, 1H), 3.21 (s, 2H), 2.87 (d, J = 14.4 Hz, 3H), 2.48 (s, 1H), 1.95 (s, 2H), 1.01 (s, 1H), 0.41 - 0.27 (m, 2H). MS (ESI) m/z = 320.1 [M+H]⁺.

### Example 7: Synthesis of compound 7

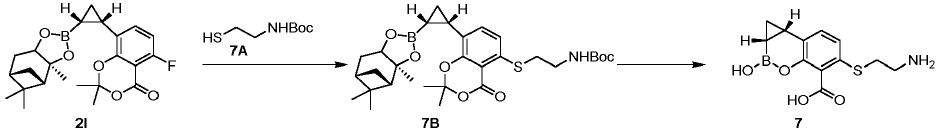

### Step 1: Preparation of compound 7B

Prepared according to the synthesis scheme of compound **2J** in Example 2. Product **7B** (180 mg, 65%) was obtained from compound **21** (200 mg, 0.483 mmol) and compound **7A** (514 mg, 2.90 mmol). MS (ESI) m/z = 471.1 [M+H]⁺.

### Step 2: Preparation of compound 7

Prepared according to the synthesis scheme of compound 2 in Example 2. Product 7 (40 mg, 46%) was obtained from compound **7B** (180 mg, 0.315 mmol). ¹H NMR (400 MHz, D₂O) δ 6.84 (d, *J* = 7.8 Hz, 1H), 6.55 (d, *J* = 7.8 Hz, 1H), 2.64 (t, *J* = 5.9 Hz, 2H), 2.53 (t, *J* = 6.3 Hz, 2H), 2.44 - 2.41 (m, 2H), 1.54 (td, *J* = 8.5, 3.5 Hz, 1H), 0.58 (t, *J* = 7.2 Hz, 1H). MS (ESI) m/z = 280.1 [M+H]⁺.

### Example 8: Synthesis of compound 8

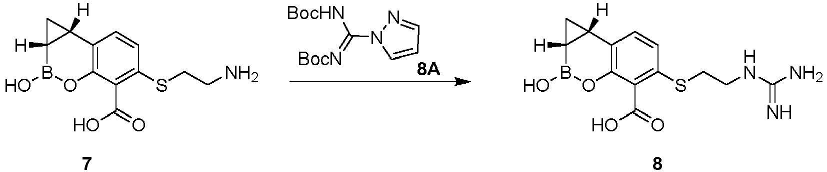

### Step 1: Preparation of compound 8

At room temperature, compound 7 (40.0 mg, 0.143 mmol) was dissolved in dimethyl sulfoxide (4.0 mL), and N,N-diisopropylethylamine (27.8 mg, 0.215 mmol) was added thereto. The system was replaced with argon three times, then compound **8A** (48.8 mg, 1.57 mmol) was added to the system and stirred at 60°C overnight. The reaction mixture was cooled to room temperature, diluted with ethyl acetate (10.0 mL), extracted with water (3.0 mL) for three times, and the combined organic phases were washed with brine (3.0 mL) and concentrated to obtain a crude product. Then, a solution of hydrochloric acid in methanol (4 M, 4 mL) was added thereto and stirred overnight at room temperature. The reaction mixture was prepared and purified by alkaline HPLC (0.1% NH₄OH) to obtain compound **8** (4.2 mg, 9%). ¹H NMR (400 MHz, CD₃OD) δ 7.31 (d, J= 7.9 Hz, 1H), 7.08 (d, *J* = 7.8 Hz, 1H), 3.47 (dd, *J=* 14.6, 8.4 Hz, 2H), 3.05 (t, *J* = 6.1 Hz, 2H), 2.19 (t, *J* = 7.5 Hz, 1H), 1.33 (s, 1H), 1.28 (s, 1H), 0.59 - 0.50 (m, 1H). MS (ESI) m/z = 322.0 [M+H]⁺.

### Example 9: Synthesis of compound 9

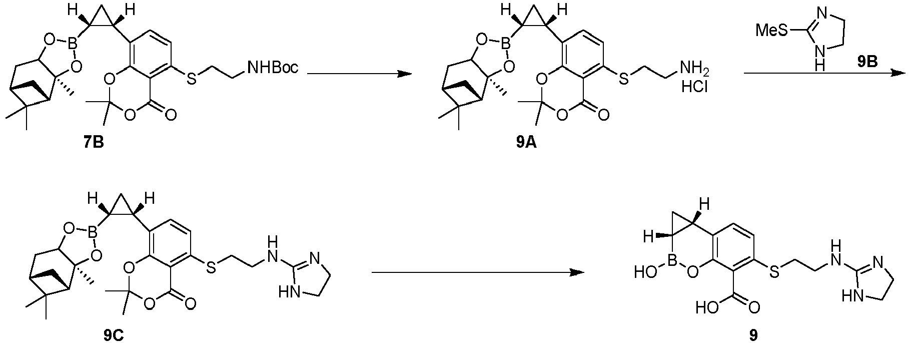

### Step 1: Preparation of compound 9A

At room temperature, compound **7B** (24.0 mg, 0.051 mmol) was dissolved in ethyl acetate solution of hydrogen chloride (4 M, 5.0 mL) and the reaction mixture was stirred at room temperature for 18 hours. TLC plate showed that the reaction of raw materials was complete. The reaction mixture was concentrated to obtain crude compound **9A** (26.0 mg), which was directly used in the next step. MS (ESI) m/z = 472.4 [M+H]⁺.

### Step 2: Preparation of compound 9C

At room temperature, compound **9A** (20 mg, 0.042 mmol) was dissolved in tetrahydrofuran (4.0 mL), and sodium bicarbonate (7.0 mg, 0.085 mmol) was added thereto. The system was replaced with argon, and compound **9B** (21 mg, 0.085 mmol) was added to the system, and the mixture was stirred at 70°C overnight. The reaction mixture was cooled to room temperature, diluted with ethyl acetate (10.0 mL), extracted with water (3.0 mL) for three times, and the combined organic phases were washed with brine (3.0 mL) and concentrated to obtain a crude product. The crude product was purified by column chromatography (MeOH/DCM = 1/9) to obtain compound **9C** (18 mg, 79%). MS (ESI) m/z = 540.0 [M+H]⁺.

### Step 3: Preparation of compound 9

Prepared according to the synthesis scheme of compound 2 in Example 2. Compound 9 (1.8 mg, 15%) was obtained from compound 7**B** (18 mg, 0.033 mmol). ¹H NMR (400 MHz, D₂O) δ 7.18 (d, J = 7.8 Hz, 1H), 6.87 (d, J = 7.8 Hz, 1H), 3.59 (s, 4H), 3.40 (t, J = 5.7 Hz, 2H), 3.05 (t, J = 5.6 Hz, 2H), 1.86 (t, J = 6.6 Hz, 1H), 0.90 (t, J = 7.9 Hz, 1H), 0.47 - 0.25 (m, 2H). MS (ESI) m/z = 348.0 [M+H]⁺.

### Example 10: Synthesis of compound 10

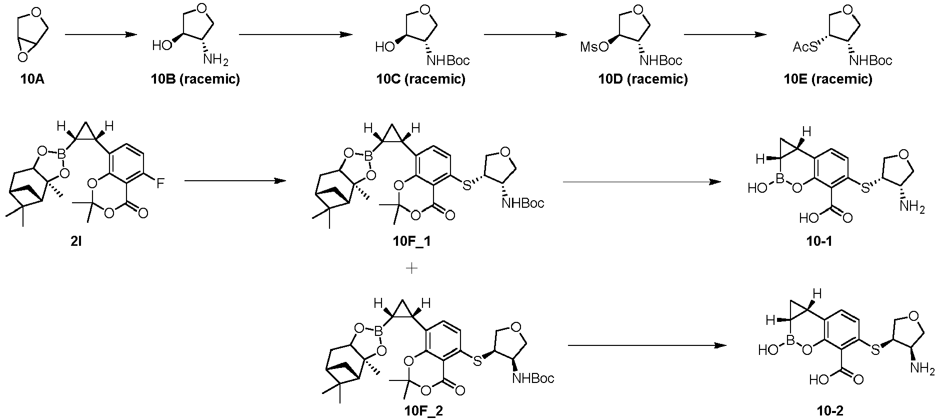

### Step 1: Preparation of compound 10B

At room temperature, ammonia water (20 mL) was added to a sealed can containing 10A (4.0 g, 46.5 mmol), and the sealed can was bubbled and replaced with argon for 1 minute, tightened, and then stirred at 110°C for 14 hours. TLC (PE: EA = 5:1) showed that the raw materials disappeared, and the reaction mixture was concentrated to obtain racemic compound 10B (4.8 g, 100%). The crude product was directly used in the next reaction step without purification.

### Step 2: Preparation of compound 10C

At room temperature, racemic **10B** (4.8 g, 46.5 mmol) was dissolved in tetrahydrofuran (50 mL), then Boc₂O (12.8 mL, 55.8 mmol) and NaOH solution (31 mL, 93 Mmol, 3 m) were added thereto and stirred at room temperature for 2 hours. TLC (PE/EA = 5: 1) showed that the raw materials disappeared. Ethyl acetate and saturated brine were added to the reaction mixture, and the phases were separated. The aqueous phase was extracted with ethyl acetate twice, and the combined organic phases were washed with saturated brine for three times, dried with anhydrous sodium sulfate, filtered and concentrated to obtain racemic compound **10C** (6.0 g, 64%). The crude product was directly used in the next reaction step without purification. ¹H NMR (400 MHz, CDCl₃) δ 4.76 (s, 1H), 4.29 (s, 1H), 4.15 - 4.01 (m, 2H), 3.96 (s, 1H), 3.70 (d, J = 8.2 Hz, 1H), 3.62 (dd, J = 9.3, 2.4 Hz, 1H), 2.66 (s, 1H), 1.45 (s, 9H).

### Step 3: Preparation of compound 10D

At room temperature, compound **10C** (1.0 g, 4.92 mmol) was dissolved in dichloromethane (10 mL), then triethylamine (0.89 mL, 6.40 mmol) was added thereto, cooled to 0°C in an ice bath, and then MsCl (0.46 mL, 5.90 mmol) was slowly added dropwise thereto. After the dropwise addition was completed, the reaction mixture was warmed to room temperature and stirred for 2 hours. Saturated brine was added to the reaction mixture, and the phases were separated, and the organic phase was washed twice with saturated brine, dried with anhydrous sodium sulfate, filtered and concentrated to obtain compound **10D** (1.3 g, 94%). The crude product was directly used in the next reaction step without purification.

### Step 4: Preparation of compound 10E

At room temperature, compound **10D** (1.3 g, 4.62 mmol) was dissolved in ultra-dry DMF (30 mL) and the system was replaced with argon three times. Then potassium thioacetate (792 mg, 6.93 mmol) was added thereto in batches, and the reaction mixture was yellow. After slowly heating to 65°C and stirring for 18 hours, the raw materials were basically consumed, and the reaction mixture turned black and a large number of solids were produced. The reaction mixture was cooled to room temperature, added with water (200 mL) and ethyl acetate (50 mL), and the phases were separated. The aqueous phase was extracted with ethyl acetate for three times, and the combined organic phases were washed with saturated brine for four times, dried with anhydrous sodium sulfate, filtered, concentrated to obtain a crude product, which was purified by column chromatography (0-50% EA/PE) to obtain compound **10E** (570 mg, 47%). ¹H NMR (400 MHz, CDCl₃) δ 4.75 (s, 1H), 4.50 (s, 1H), 4.20 (dt, J = 13.3, 7.4 Hz, 2H), 4.02 (dd, J = 9.3, 5.8 Hz, 1H), 3.67 (dd, J = 8.8, 6.4 Hz, 1H), 3.64 - 3.55 (m, 1H), 2.36 (s, 3H), 1.51 - 1.39 (m, 9H). MS (ESI) m/z = 206.1 [M-56]⁺.

### Step 5: Preparation of compound 10F

Prepared according to the synthesis scheme of compound **2J** in Example 2. Crude racemic compound **10F** (mixture of **10F_1** and **10F_2,** 130 mg) was obtained from compound **21** (150 mg, 0.362 mmol) and compound **10E** (284 mg, 1.09 mmol). Compound **10F_P1** (isomer **10F_1** or **10F_2,** 30 mg, 13.5%) and compound **10F_P2** (isomer **10F_2** or **10F_1,** 32 mg, 14.4%) were prepared and separated by alkaline HPLC. Compound **10F_P1:** MS (ESI) m/z = 636.2 [M+Na]⁺; compound **10F_P2:** MS (ESI) m/z = 636.2 [M+Na]⁺.

### Step 6: Preparation of compound 10

Prepared according to the synthesis scheme of compound **2** in Example 2.

Compound **10-P1** (isomer **10_1** or **10_2,** 6.9 mg, 44%) was obtained from compound **10F_P1** (30 mg, 0.0489 mmol). ¹H NMR (400 MHz, D₂O) δ 7.13 (d, J = 7.9 Hz, 1H), 6.95 (d, J = 7.9 Hz, 1H), 4.17 (t, J = 9.1 Hz, 1H), 4.04 - 3.96 (m, 1H), 3.87 (d, J = 3.9 Hz, 2H), 3.82 - 3.72 (m, 1H), 3.60 (t, J = 9.0 Hz, 1H), 1.93 (dd, J = 8.1, 3.7 Hz, 1H), 1.01 (t, J = 7.6 Hz, 1H), 0.40 - 0.29 (m, 1H), 0.29 - 0.16 (m, 1H). MS (ESI) m/z = 322.0 [M+H]⁺.

Compound **10-P2** (isomer **10_1** or **10_2,** 13.6 mg, 87%) was obtained from compound **10F_P2** (30 mg, 0.0489 mmol). ¹H NMR (400 MHz, D₂O) δ 7.16 (d, J = 7.8 Hz, 1H), 6.94 (d, J = 7.9 Hz, 1H), 4.23 (d, J = 9.2 Hz, 1H), 4.05 (dd, J = 14.6, 8.7 Hz, 1H), 4.00 - 3.91 (m, 2H), 3.81 (s, 1H), 3.67 (t, J = 9.1 Hz, 1H), 1.87 (dd, J = 15.5, 8.0 Hz, 1H), 0.95 (d, J = 6.8 Hz, 1H), 0.30 (d, J = 4.3 Hz, 2H). MS (ESI) m/z = 322.0 [M+H]⁺.

### Example 11: Synthesis of compound 11

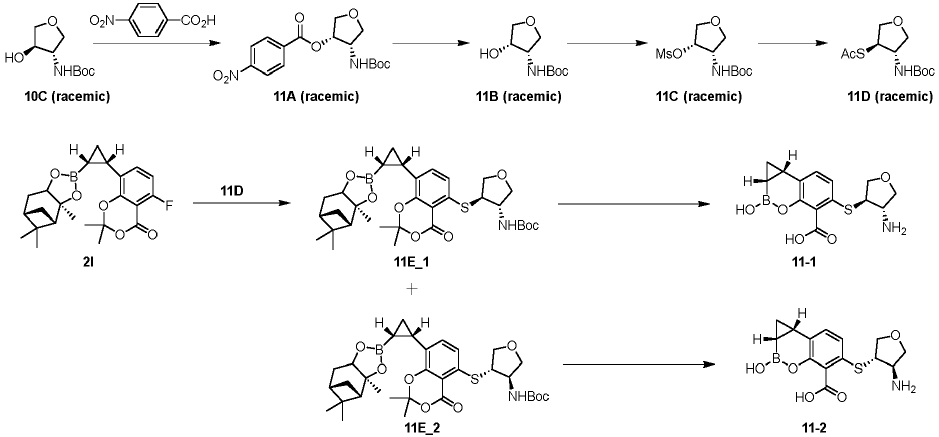

### Step 1: Preparation of compound 11A

At room temperature, compound **10C** (2.0 g, 9.85 mmol) was dissolved in tetrahydrofuran (20 mL), then p-nitrobenzoic acid (1.8 g, 10.8 mmol) and triphenylphosphine (3.1 g, 11.8 mmol) were added thereto, cooled to 0°C in an ice bath, and then diethyl azodicarboxylate (2.06 g, 11.8 mmol) was slowly added dropwise thereto. After the dropwise addition was completed, the reaction mixture was warmed to room temperature and stirred for 16 hours. Ethyl acetate and water were added to the reaction mixture, and the phases were separated. The organic phase was washed twice with saturated brine, dried with anhydrous sodium sulfate, filtered and concentrated to obtain a crude product, which was purified by column chromatography (0-20% EA/PE) to obtain compound **11A** (1.00 g, 29%). MS (ESI) m/z = 338.0 [M-14]⁺.

### Step 2: Preparation of compound 11B

At room temperature, compound **11A** (1.00 g, 2.84 mmol) was dissolved in methanol (10 mL), then lithium hydroxide monohydrate (357 mg, 8.51 mmol) dissolved in water (2 mL) was added thereto, and the reaction mixture was stirred for 2 hours at room temperature. TLC (EA/PE = 3:7) showed that the reaction of raw materials was complete. Ethyl acetate and water were added to the reaction mixture, and the phases were separated. The organic phase was washed twice with saturated sodium bicarbonate aqueous solution, then washed with saturated brine, dried with anhydrous sodium sulfate, filtered and concentrated to obtain compound **11B** (512 mg, 89%). The crude product was directly used in the next reaction step without purification.

### Step 3: Preparation of compound 11C

Prepared according to the synthesis scheme of compound **10D** in Example 10. Compound **11C** (590 mg, 83.2%) was obtained from compound **11B** (512 mg, 2.52 mmol). MS (ESI) m/z = 181.0 [M-100]⁺.

### Step 4: Preparation of compound 11D

Prepared according to the synthesis scheme of compound **10E** in Example 10. Compound **11D** (250 mg, 46%) was obtained from compound 1**1C** (590 mg, 2.10 mmol). MS (ESI) m/z = 284.0 [M+Na]⁺.

### Step 5: Preparation of compound 11E

Prepared according to the synthesis scheme of compound **2J** in Example 2. The racemic compound **11E** (mixture of isomers **10E_1** and **10E_2,** 120 mg) was obtained from compound **21** (132 mg, 0.319 mmol) and compound **11D** (250 mg, 0.958 mmol). Compound **11E_P1** (isomer **11E_1** or **11E_2,** 50 mg, 26%) and compound **11E_P2** (isomer **11E_2** or **11E_1,** 40 mg, 21%) were prepared and separated by alkaline HPLC. Compound **11E_P1:** MS (ESI) m/z = 636.3 [M+Na]⁺; compound **11E_P2:** MS (ESI) m/z = 636.3 [M+Na]⁺.

### Step 6: Preparation of compound 11

Prepared according to the synthesis scheme of compound **2** in Example 2.

Product **11-P1** (isomer **11_1** or **11_2,** 23.2 mg, 89%) was obtained from compound **11E_P1** (50 mg, 0.0815 mmol). ¹H NMR (400 MHz, D₂O) δ 7.23 (d, *J* = 7.8 Hz, 1H), 7.04 (d, *J* = 7.8 Hz, 1H), 4.28 (dd, *J* = 9.8, 6.9 Hz, 1H), 4.08 (dd, *J* = 10.6, 5.5 Hz, 1H), 3.87 (dd, *J* = 10.6, 2.7 Hz, 1H), 3.80 (dt, *J* = 5.6, 2.9 Hz, 1H), 3.75 (dd, *J* = 11.1, 4.6 Hz, 1H), 3.68 (dd, J = 9.8, 5.7 Hz, 1H), 2.12 (d, *J* = 3.7 Hz, 1H), 1.18 (t, *J* = 7.5 Hz, 1H), 0.48 (dd, *J* = 16.9, 7.6 Hz, 1H), 0.41 - 0.32 (m, 1H). MS (ESI) m/z = 322.0 [M+H]⁺.

Product **11-P2** (isomer **11_1** or **11_2,** 18.4 mg, 88%) was obtained from compound **11E_P2** (40 mg, 0.0652 mmol). ¹H NMR (400 MHz, D₂O) δ 7.23 (d, *J* = 7.7 Hz, 1H), 7.04 (d, *J* = 7.9 Hz, 1H), 4.27 (dd, *J* = 9.8, 6.8 Hz, 1H), 4.08 (dd, *J* = 10.6, 5.6 Hz, 1H), 3.87 (dd, *J* = 10.6, 2.8 Hz, 1H), 3.82 - 3.78 (m, 1H), 3.75 (dd, *J* = 11.0, 4.5 Hz, 1H), 3.68 (dd, *J* = 9.8, 5.6 Hz, 1H), 2.10 (t, *J* = 9.9 Hz, 1H), 1.16 (t, *J* = 8.2 Hz, 1H), 0.46 (dd, *J* = 16.2, 8.1 Hz, 1H), 0.40 - 0.31 (m, 1H). MS (ESI) m/z = 322.0 [M+H]⁺.

### Example 12: Synthesis of compound 12

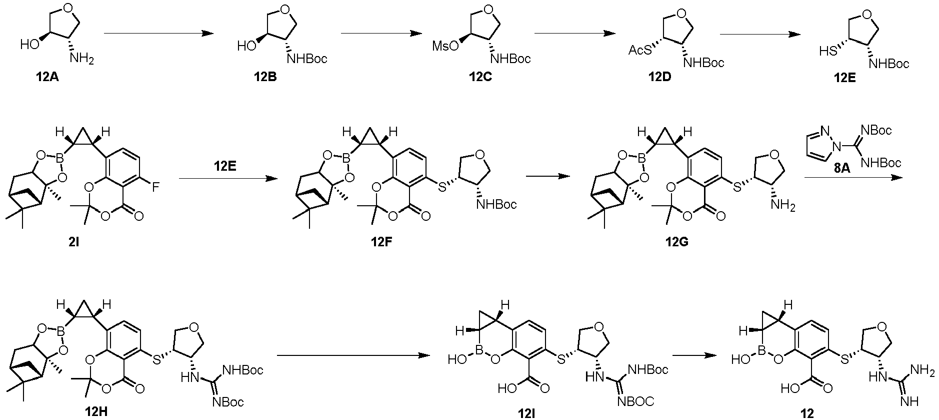

### Step 1: Preparation of compound 12B

Prepared according to the synthesis scheme of compound 10C in Example 10. Compound **12B** (1.9 g, 96%) was obtained from compound **12A** (1.0 g, 9.7 mmol). ¹H NMR (400 MHz, DMSO-*d*₆) δ 7.06 (d, *J* = 6.7 Hz, 1H), 5.15 (d, *J* = 4.0 Hz, 1H), 4.02 (tt, *J* = 4.2, 2.1 Hz, 1H), 3.86 (dd, *J* = 8.8, 5.6 Hz, 1H), 3.81 - 3.76 (m, 1H), 3.72 (s, 1H), 3.45 (dt, *J* = 8.8, 2.3 Hz, 2H), 1.39 (s, 9H).

### Step 2: Preparation of compound 12C

Prepared according to the synthesis scheme of compound **10D** in Example 10. Compound **12C** (688 mg, 100%) was obtained from compound **12B** (500 mg, 2.46 mmol). MS (ESI) m/z = 181.2 [M-100]⁺.

### Step 3: Preparation of compound 12D

Prepared according to the synthesis scheme of compound **10E** in Example 10. Compound **12D** (430 mg, 67%) was obtained from compound **12C** (688 mg, 2.46 mmol). MS (ESI) m/z = 206.2 [M+ H-56]⁺.

### Step 4: Preparation of compound 12E

Compound **12D** (430 mg, 1.65 mmol) and sodium hydroxide (400 mg, 10.0 mmol) were dissolved in methanol (10 mL), and the system was replaced with argon and reacted at 0°C for 0.5 hours. The reaction mixture was added with 0.5 M dilute hydrochloric acid to adjust the pH to 6, extracted twice with ethyl acetate, dried with anhydrous sodium sulfate, concentrated, and separated by column chromatography (PE/EA = 80:20) to obtain compound 12E (320 mg, 59%). MS (ESI) m/z = 164.2 [M+ H-56]⁺.

### Step 5: Preparation of compound 12F

Prepared according to the synthesis scheme of compound **2J** in Example 2. Compound **12F** (100 mg, 58%) was obtained from compound **21** (100 mg, 0.24 mmol) and compound **12E** (110 mg, 0.5 mmol). MS (ESI) m/z = 514.4 [M+ H-100]⁺.

### Step 6: Preparation of compound 12G

A solution of compound **12F** (100 mg, 0.163 mmol) in tetrahydrofuran (1 mL) was added with dioxane hydrogen chloride (2 mL), and the reaction mixture was stirred at 0°C for 2 hours. After the reaction was completed, the reaction mixture was concentrated to obtain compound **12G** (90 mg, 100%). The crude product was directly used in the next reaction step without purification. MS (ESI) m/z = 514.4 [M+H]⁺.

### Step 7: Preparation of compound 12H

Compound **12G** (90 mg, 0.163 mmol), compound **8A** (186 mg, 0.6 mmol) and DIPEA (78 mg, 0.6 mmol) were dissolved in acetonitrile (4.0 mL), and the reaction mixture was stirred overnight at room temperature, and separated by reversed-phase column to obtain compound **12H** (110 mg, 89%). MS (ESI) m/z = 756.6 [M+H]⁺.

### Step 8: Preparation of compound 121

Compound **12H** (30 mg, 0.047 mmol) and sodium hydroxide (80 mg, 2.0 mmol) were dissolved in tetrahydrofuran/water (0.5 mL/0.5 mL), and the reaction mixture was stirred at 30°C for 3 hours. After the reaction was completed, the reaction mixture was cooled to 0°C, added with trifluoroacetic acid to adjust the pH to 3-4, and then trifluoroacetic acid (1.0 mL), triethylsilane (0.3 mL) and isobutylboronic acid (51 mg, 0.5 mmol) were added thereto in sequence at 0°C, and then the reaction mixture was stirred for another 2 hours at 0°C After the reaction was completed, sodium hydroxide was added to the reaction mixture at 0°C to adjust the pH to 9. Compound **121** (50 mg, 38%) was prepared by alkaline HPLC. MS (ESI) m/z = 402.1 [M+ H-100]⁺.

### Step 9: Preparation of compound 12

Compound **121** (8 mg, 0.018 mmol) was dissolved in tetrahydrofuran (0.1 mL), then dioxane hydrogen chloride (0.3 mL) was added thereto and stirred at 0°C for 2 hours. After the reaction was completed, compound **12** (1.1 mg, 17%) was prepared by alkaline HPLC. ¹H NMR (400 MHz, D₂O) δ 6.93 (d, *J* = 7.8 Hz, 1H), 6.64 (d, *J* = 7.8 Hz, 1H), 4.10-4.07 (m, 1H), 3.98-3.94 (m, 1H), 3.85-3.81 (m, 2H), 3.67-3.64 (m, 1H), 3.54-3.50 (m, 1H), 1.65-1.62 (m, 1H), 0.69-0.66 (m, 1H), 0.20-0.03 (m, 2H). MS (ESI) m/z = 364.0 [M+H]⁺.

### Example 13: Synthesis of compound 13

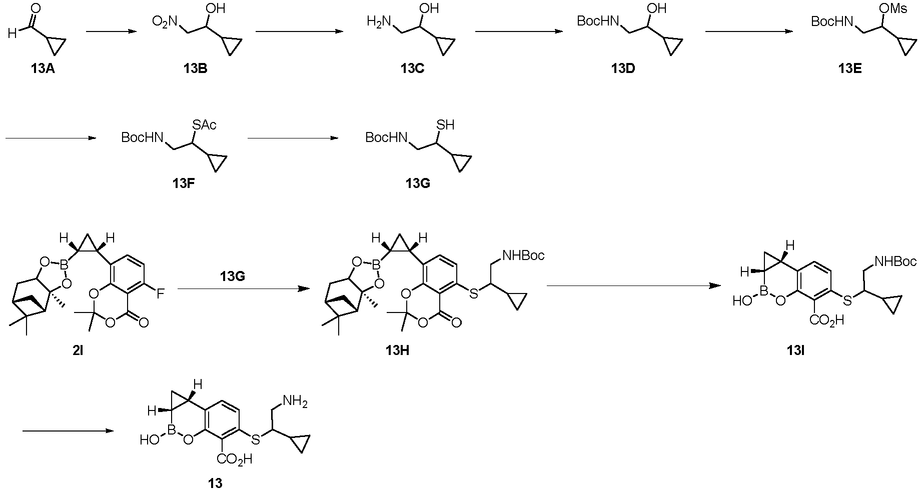

### Step 1: Preparation of compound 13B

Compound **13A** (10.0 g, 142.8 mmol) was dissolved in tert-butanol (50 mL), then sodium tert-butoxide (14.4 g, 150 mmol) and nitromethane (9.2 g, 150 mmol) were added thereto in batches at 0°C. The reaction mixture was stirred at 0°C for 2 hours, diluted with ethyl acetate (500 mL), and the turbid solution was filtered. The mother liquor was washed with 1 M HCl aqueous solution, and the organic phase was washed once with saturated NaCl solution, dried with anhydrous sodium sulfate, and concentrated to obtain compound **13B** (18.7 g, 100%). The crude product was directly used in the next reaction step without purification. MS (ESI) m/z = 114.1[M+H-18]⁺.

### Step 2: Preparation of compound 13C

Compound **13B** (18.1 g, 142.8 mmol) and Pd/C(1.0 g) were dissolved in methanol (300 mL), and the system was replaced with hydrogen and stirred overnight at room temperature. The reaction mixture was filtered through diatomite and directly concentrated to obtain compound **13C** (14.4 g, 100%). MS (ESI) m/z = 102.4 [M+H]⁺.

### Step 3: Preparation of compound 13D

Prepared according to the synthesis scheme of compound **10C** in Example 10. Compound **13D** (13.2 g, 46%) was obtained from compound **13C** (14.4 g, 142.8 mmol). MS (ESI) m/z = 202.6 [M+H]⁺.

### Step 4: Preparation of compound 13E

Prepared according to the synthesis scheme of compound **10D** in Example 10. Compound **13E** (11.0 g, 70%) was obtained from compound **13D** (10.0 g, 49.5 mmol). MS (ESI) m/z = 180.2 [M+ H-100]⁺.

### Step 5: Preparation of compound 13F

Prepared according to the synthesis scheme of compound **10E** in Example 10. Compound **13F** (6.8 g, 66%) was obtained from compound **13E** (11.0 g, 39.6 mmol). MS (ESI) m/z = 160.2 [M+ H-100]⁺.

### Step 6: Preparation of compound 13G

Prepared according to the synthesis scheme of compound **12E** in Example 12. Compound **13G** (200 mg, 24%) was obtained from compound **13F** (1.0 g, 3.86 mmol). MS (ESI) m/z = 218.2 [M+H]⁺.

### Step 7: Preparation of compound 13H

Prepared according to the synthesis scheme of compound **2J** in Example 2. Compound **13H** (30 mg, 32%) was obtained from compound **21** (60 mg, 0.145 mmol) and compound **13G** (110 mg, 0.5 mmol). MS (ESI) m/z = 512.0 [M+ H-100]⁺.

### Step 8: Preparation of compound 131

Prepared according to the synthesis scheme of compound **121** in Example 12. Compound **131** (8 mg, 38%) was obtained from compound **13H** (30 mg, 0.047 mmol). MS (ESI) m/z = 320.2 [M+ H-100]⁺.

### Step 10: Preparation of compound 13

Prepared according to the synthesis scheme of compound **12** in Example 12. Compound 13 (0.7 mg, 9%) was obtained from compound **131** (10 mg, 0.0238 mmol). ¹H NMR (400 MHz, D₂O) δ 7.57 (d, J = 8.0 Hz, 1H), 6.70 (d, J = 8.0 Hz, 1H), 2.75 - 2.50 (m, 2H), 2.35 - 2.25 (m, 1H), 1.25 - 1.75 (m, 1H), 0.70 - 0.60 (m, 2H), 0.60 - 0.40 (m, 2H), 0.30 - 0.20 (m, 4H). MS (ESI) m/z = 320.0 [M+H]⁺.

### Example 14: Synthesis of compound 14

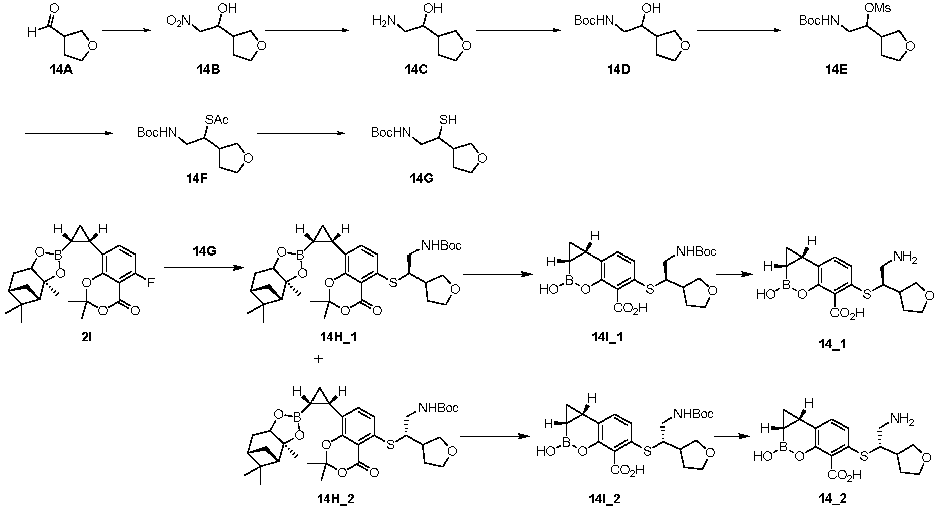

### Step 1: Preparation of compound 14B

Prepared according to the synthesis scheme of compound **13B** in Example 13. Compound 14B (16.1 g, 100%) was obtained from compound **14A** (10.0 g, 100 mmol). MS (ESI) m/z = 161.60 [M+H]⁺.

### Step 2: Preparation of compound 14C

Prepared according to the synthesis scheme of compound **13C** in Example 13. Compound **14C** (13.1 g, 100%) was obtained from compound **14B** (16.1 g, 100 mmol). MS (ESI) m/z = 131.60 [M+H]⁺.

### Step 3: Preparation of compound 14D

Prepared according to the synthesis scheme of compound **10C** in Example 10. Compound **14D** (15.2 g, 66%) was obtained from compound **14C** (13.1 g, 100 mmol). MS (ESI) m/z = 231.6 [M+H]⁺.

### Step 4: Preparation of compound 14E

Prepared according to the synthesis scheme of compound **10D** in Example 10. Compound **14E** (13.5 g, 100%) was obtained from compound **14D** (10.0 g, 43.3 mmol). MS (ESI) m/z = 210.0 [M+ H-100]⁺.

### Step 5: Preparation of compound 14F

Prepared according to the synthesis scheme of compound **10E** in Example 10. Compound **14F** (8.7 g, 69%) was obtained from compound **14E** (13.5 g, 43.7 mmol). MS (ESI) m/z = 190.2 [M+ H-100]⁺.

### Step 6: Preparation of compound 14G

Prepared according to the synthesis scheme of compound **12E** in Example 12. Compound **14G** (500 mg, 59%) was obtained from compound **14F** (1.0 g, 3.45 mmol). MS (ESI) m/z = 248.0 [M+H]⁺.

### Step 7: Preparation of compound 14H

Prepared according to the synthesis scheme of compound **2J** in Example 2. Compound **14H_P1** (isomer **14H_1** or **14H_2,** 32 mg, 34%) and compound **14H_P2** (isomer **14H_2** or **14H_1,** 30 mg, 32%) were obtained from compound **21** (60 mg, 0.145 mmol) and compound **14G** (124 mg, 0.5 mmol). **14H_P1:** MS (ESI) m/z = 542.0 [M+H-100]⁺; **14H_P2:** MS (ESI) m/z = 542.0 [M+H-100]⁺.

### Step 8: Preparation of compound 141

Prepared according to the synthesis scheme of compound **121** in Example 12. Compound **141_1** (isomer **14I_1** or **14I_2,** 9 mg, 40%) was obtained from compound **14H_P1** (32 mg, 0.05 mmol); compound **14I_P2** (isomer **141_2** or **14I_1,** 8 mg, 38%) was obtained from compound **14H_P2** (30 mg, 0.05 mmol). **14I_P1:** MS (ESI) m/z = 350.2 [M+H-100]⁺; **14I_P2:** MS (ESI) m/z = 350.2 [M+H-100]⁺.

### Step 10: Preparation of compound 14

Prepared according to the synthesis scheme of compound **12** in Example 12.

Compound **14_P1** (isomer **14_1** or **14_2,** 5.5 mg, 78%) was obtained from compound **14I_P1** (9 mg, 0.02 mmol). ¹H NMR (400 MHz, D₂O)δ 6.78 (d, *J* = 7.7 Hz, 1H), 6.50 (d, *J* = 7.7 Hz,1H), 3.75 - 3.51 (m, 3H), 3.47 - 3.39 (m, 2H), 3.31 - 3.21 (m, 2H), 2.81 - 2.75 ( m, 1H), 2.37 - 2.32 (m, 1H), 2.26 - 2.16 (m, 1H), 1.88 - 1.78 (m, 1H), 1.52 - 1.46 (m, 1H), 0.58 - 0.50 (m, 1H), 0.03 - 0.01 (m, 1H). MS (ESI) m/z = 350.2 [M+H]⁺.

Compound **14_P2** (isomer **14_2** or **14_1,** 1.1 mg, 17%) was obtained from compound **14I_P2** (8 mg, 0.018 mmol). ¹H NMR (400 MHz, D₂O) δ 6.82 (d, *J* = 7.8 Hz, 1H), 6.54 (d, *J=* 7.5 Hz, 1H), 3.78 - 3.60 (m, 2H), 3.48 - 3.44 (m, 1H), 3.32 - 3.29 (m, 1H), 2.84 - 2.80 (m, 1H), 2.42 - 2.21 (m, 3H), 1.88 - 1.80 (m, 1H), 1.55 - 1.50 (m, 2H), 0.58 - 0.56 (m, 1H), 0.10 - 0.07 (m, 2H). MS (ESI) m/z = 350.2 [M+H]⁺.

### Example 15: Synthesis of compound 15

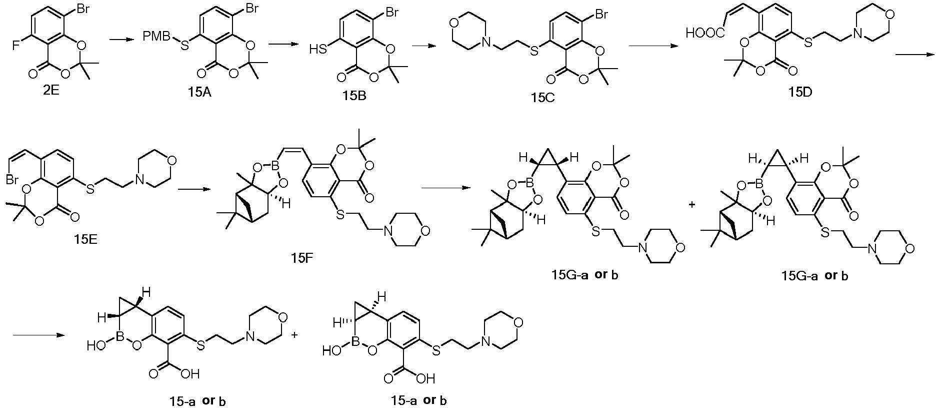

### Step 1: Preparation of compound 15A

Compound **2E** (5 g, 18.2 mmol) was dissolved in N,N-dimethylformamide (100 mL). Under nitrogen atmosphere, p-methoxybenzyl mercaptan (4.2 g, 27.3 mmol) and cesium carbonate (17.7 g, 54.6 mmol) were added thereto and stirred at room temperature for 16 hours. After the reaction was completed, the reaction mixture was filtered, the filter cake was washed with ethyl acetate, and the filtrate was collected, concentrated under reduced pressure, added with 50 mL of methanol and stirred for 10 minutes, filtered. The filter cake was washed with methanol, and dried under reduced pressure in vacuum to obtain a white solid **15A** (5.7 g, yield: 76.1%).

**¹H NMR (400 MHz, DMSO-d6)** δ 7.85 (d, J = 8.7 Hz, 1H), 7.39 - 7.34 (m, 2H), 7.15 (d, J = 8.8 Hz, 1H), 6.95 - 6.88 (m, 2H), 4.20 (s, 2H), 3.74 (s, 3H), 1.69 (s, 6H).

### Step 2: Preparation of compound 15B

Compound **15A** (3 g, 7.3 mmol) was dissolved in dichloromethane (30 mL), and trifluoromethanesulfonic acid (2 mL) was added thereto at 0°C and stirred for 1 hour. After the reaction was completed, the reaction mixture was added with sodium bicarbonate solution to adjust the pH to neutral and extracted with dichloromethane (50 mL × 3). The organic phases were combined, washed with saturated brine, dried with anhydrous sodium sulfate, filtered, and concentrated under reduced pressure to obtain 3 g of crude **15B,** which was used directly in the next step without purification. MS(ESI)m/z:(M+H)⁺= 288.80.

### Step 3: Preparation of compound 15C

At room temperature, compound **15B** (3 g, 7.3 mmol) was dissolved in N,N-dimethylformamide (30 mL), then potassium carbonate (1.5 g, 10.95 mmol) and 2-(4-morpholine) ethyl bromide (2.0 g, 7.3 mmol) were added thereto and stirred for 2 hours. After the reaction was completed, the reaction mixture was washed with water (100 mL), extracted with ethyl acetate (50 mL × 3), and the organic phases were combined, washed with saturated brine, dried with anhydrous sodium sulfate, filtered and concentrated under reduced pressure to obtain a crude product, which was purified by column chromatography (petroleum ether/ethyl acetate = 5:3) to obtain the compound **15C** (2.6 g, two-step yield: 88.4%). MS(ESI)m/z:(M+H)⁺ = 401.8.

### Step 4: Preparation of compound 15D

Compound **15C** (1.6 g, 3.9 mmol), acrylic acid (861.8 mg, 11.9 mmol), palladium acetate (175.1 mg, 0.78 mmol), tri(o-tolyl)phosphine (948.4 mg, 3.1 mmol), and diisopropylethylenediamine (2.5 g, 19.5 mmol) were dissolved in N,N-dimethylformamide (20 mL), and the system was replaced with nitrogen for three times, heated to 100°C, and stirred for 16 hours. After the reaction was completed, the reaction mixture was cooled to room temperature, added with water (50 mL), extracted with ethyl acetate (50 mL × 3), and the organic phases were combined, washed with saturated brine, dried with anhydrous sodium sulfate, filtered and concentrated under reduced pressure to obtain a crude product, which was purified by column chromatography (dichloromethane/methanol = 10: 1) to obtain compound **15D** (1.1 g, yield: 70.5%). MS(ESI)m/z(M+H)⁺ = 394.0.

### Step 5: Preparation of compound 15E

Compound **15D** (676 mg, 1.72 mmol) was dissolved in chloroform (6 mL), then acetic acid (2 mL) was added thereto under an ice bath, and then liquid bromine (330 mg, 2.06 mmol) was slowly added dropwise thereto, and the reaction mixture was stirred for 2 hours at 0°C. After the reaction was completed, the reaction mixture was concentrated under reduced pressure, and the resulting residual solid was added with N,N-dimethylformamide (6 mL), and added with triethylamine (347.4 mg, 3.44 mmol) at room temperature, and the mixture was stirred for another 1 hour. After the reaction was completed, the reaction mixture was diluted with ethyl acetate (20 mL), washed with water (50 mL × 3), washed with saturated brine, dried with anhydrous sodium sulfate, filtered and concentrated under reduced pressure to obtain a crude product, which was purified by column chromatography (petroleum ether/ethyl acetate = 5:3) to obtain compound **15E** (488 mg, yield: 66.4%). MS(ESI)m/z:(M+H)⁺= 428.0.

### Step 6: Preparation of compound 15F

Compound **15E** (1.3 g, 3.04 mmol) was dissolved in isopropyl acetate (10 mL), then bis[(1S,2S,3R,5S)(+)-pinanediolato]diboron (1.63 g, 4.56 mmol), allylpalladium (II) chloride dimer (111.0 mg, 0.304 mmol), 2-dicyclohexylphosphino-2',4',6'-triisopropylbiphenyl (289.8 mg, 0.608 mmol) and potassium isooctanoate (1.2 g, 6.69 mmol) were added thereto, and the system was replaced with argon for 4 times, heated to 35°C and stirred for 16 hours. After the reaction was completed, the reaction mixture was washed with water (100 mL), extracted with ethyl acetate (50 mL × 3), and the organic phases were combined, washed with saturated brine, dried with anhydrous sodium sulfate, filtered and concentrated under reduced pressure to obtain a crude product, which was purified by column chromatography (petroleum ether/ethyl acetate = 5:3) to obtain compound **15F** (372 mg, yield: 23.5%). MS(ESI)m/z:(M+H)⁺= 528.2.

### Step 7: Preparation of compounds 15G-a and 15G-b

At -20 °C, , an ether solution of CH₂N₂ (0.2 M, 9.19 mL) was added to a solution of compound **15F** (97 mg, 183.89 µmol) and Pd(OAc)₂ (4.13 mg, 18.39 µmol) in THF (3 mL) under nitrogen atmosphere, and then the mixture was returned to room temperature and stirred for 16 hours. After the reaction was completed, the reaction mixture was concentrated under reduced pressure to obtain a crude product, which was purified by column chromatography (0 to 40% petroleum ether/ethyl acetate) to obtain a mixture of yellow oily compound **15G** (60 mg, yield: 60.25%), , which was purified by SFC to obtain compounds **15G-a** and **15G-b.**

Separation conditions of SFC: chiral column: Chiralpak IC-3 150 × 4.6 mm I.D., 3 µm; mobile phase: 50% of IPA (0.05% DEA) in CO₂; flow rate: 2.5 mL/min; column temperature: 35°C; ABPR: 1500 psi.

**15G-a:** retention time: t =4.021 min, HPLC: 91.78%; retention time: t =4.52 min, 25 mg white solid.

**15G-b:** retention time: t =4.860 min, HPLC: 94.11%; retention time: t =4.58 min, 25 mg white solid.

MS (ESI) m/z (M+1)⁺=542.4.

### Step 13: Preparation of compounds 15-a and 15-b

NaOH solution (3 M, 46.17 uL) was added to a solution of compound **15G-a** (25 mg, 46.17 µmol) in dioxane (1 mL), and the reaction mixture was stirred at room temperature for 24 hours. Subsequently, the reaction mixture was cooled to 0°C, added with triethylsilane (0.1 mL), trifluoroacetic acid (0.2 mL) and isobutylboronic acid (9.41 mg, 92.34µmol)in sequence, and stirred for 1 hour. After the reaction was completed, the reaction mixture was concentrated under reduced pressure to obtain a crude product, which was purified by HPLC to obtain **15-a** (5 mg, yield: 31.01%).

**¹H NMR (400MHz, METHANOL-d4)** δ = 7.39 (d, J = 8.1 Hz, 1H), 7.24 (d, J = 8.0 Hz, 1H), 3.89 (br s, 4H), 3.28 - 3.19 (m, 8H), 2.27 (dt, J = 4.2, 7.9 Hz, 1H), 1.42 - 1.32 (m, 1H), 0.66 - 0.58 (m, 1H), 0.41 - 0.33 (m, 1H). MS (ESI) m/z (M+1)+=350.1.

HPLC separation and purification conditions: chromatographic column: Welch Xtimate C18 100 * 40 mm * 3 µm; mobile phase: [water (TFA)-acetonitrile]; B%: 8%-38%,8 min);

HPLC 99.92%; retention time: t = 1.82 min.

5 mg of **15-b** was prepared using **15G-b** as raw material, and the method was the same as that of **15-a.**

**¹H NMR (400MHz, METHANOL-d4)** δ = 7.39 (d, J = 7.9 Hz, 1H), 7.25 (d, J = 7.9 Hz, 1H), 3.89 (br s, 4H), 3.27 - 3.17 (m, 8H), 2.28 (dt, J = 4.1, 8.1 Hz, 1H), 1.42 - 1.33 (m, 1H), 0.66 - 0.58 (m, 1H), 0.41 - 0.34 (m, 1H). MS (ESI) m/z (M+1)+=350.1.

HPLC 99.89%; retention time: t = 1.69 min.

### Example 16: Synthesis of compound 16

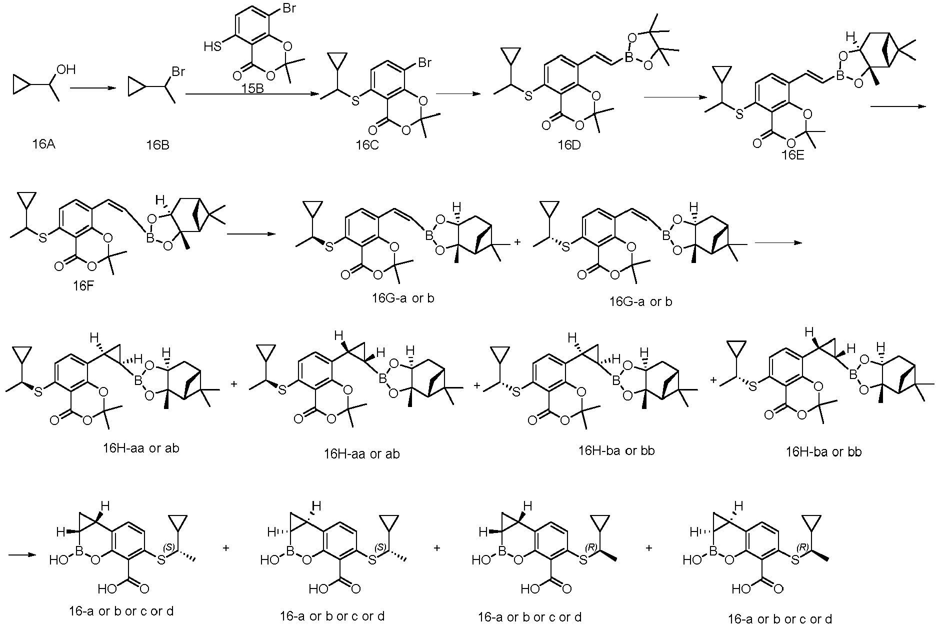

### Step 1: Preparation of compound 16B

At -20°C, liquid bromine (2.05 g, 12.79 mmol) was slowly added to a toluene (10 mL) solution of compound **16A** (1.00 g, 11.62 mmol) and triphenylphosphine (3.35g, 12.79 mmol), and the reaction mixture was continuously stirred for 2 hours. After the reaction was completed, the reaction mixture was returned to room temperature, filtered and washed with toluene (2 mL) to obtain a toluene solution (11 mL,1.06 N) of compound **16B,** which was directly used in the next step without purification.

### Step 2: Preparation of compound 16C

At room temperature, a toluene solution (0.4 mL) of **16B** was slowly added dropwise into N,N-dimethylformamide (3 mL) in which compound **15B** (100 mg, 0.346 mmol) and potassium carbonate (96.0 mg, 0.69 mmol) were dissolved under nitrogen atmosphere, and the reaction mixture was stirred at room temperature for 1 hour. After the reaction was completed, the reaction mixture was quenched with water (10 mL) and extracted with ethyl acetate (5 mL × 3), and the organic phase was collected, washed with saturated brine, dried with anhydrous sodium sulfate, filtered and concentrated under reduced pressure to obtain a crude product, which was purified by column chromatography (petroleum ether/ethyl acetate = 5:1) to obtain compound **16C** (50 mg, yield: 41%). MS(ESI)m/z:(M+H)⁺= 357.0.

### Step 3: Preparation of compound 16D

A toluene (3.0 mL) solution of **16C** (200 mg, 0.56 mmol), pinacol vinylboronate (260 mg,1.68 mmol), tris[dibenzylideneacetone]dipalladium (0) (50.0 mg, 0.056 mmol), tri-tert-butylphosphine tetrafluoroborate (32.0 mg, 0.11 mmol), and N,N-diisopropylethylamine (215 mg,1.68 mmol) was stirred at 115°C for 1 hour under nitrogen atmosphere. After the reaction was completed, the reaction mixture was quenched with water (15 mL) and extracted with ethyl acetate (5 mL × 3), and the organic phase was combined, washed with saturated brine, dried with anhydrous sodium sulfate, filtered and concentrated under reduced pressure to obtain a crude product, which was purified by column chromatography (petroleum ether: ethyl acetate = 20:1) to obtain compound **16D** (140 mg, yield: 58%). MS(ESI)m/z:(M+H)⁺= 431.2.

### Step 4: Preparation of compound 16E

At room temperature, (1S,2S,3R,5S)-(+)-2,3-pinanediol (2.00 g, 12.2 mmol) was added to a tetrahydrofuran (12 mL) solution of compound **16D** (1.05 g, 2.43 mmol) and stirred for 2 hours. After the reaction was completed, the reaction mixture was concentrated under reduced pressure to obtain a crude product, which was purified by column chromatography (petroleum ether: ethyl acetate = 10:1) to obtain compound **16E** (1.00g, yield: 85.4%). MS (ESI) m/z (M- H) ⁺ = 483.2.

### Step 5: Preparation of compound 16F

Compound **16E** (80.0 mg, 0.166 mmol) was dissolved in acetonitrile (8mL), and tris(2-phenylpyridine)iridium (8.00 mg) was added thereto at room temperature. After being irradiated by LED blue light (wavelength =450 nM, 10 W) for 1.5 hours under nitrogen atmosphere, the reaction mixture was concentrated under reduced pressure to obtain a crude product, which was purified by column chromatography (petroleum ether: ethyl acetate =20:1) to obtain compound **16F** (36 mg, yield: 45%). MS (ESI) m/z (M+ H) ⁺ = 483.2.

### Step 7: Preparation of compound 16G-a or 16G-b

Compound **16F** (400 mg, 829.12 µmol) was subjected to preparative chiral resolution to obtain white solid compound **16G-a** (120 mg, yield: 30.00%), retention time: t = 3.700 min.

Separation conditions of SFC: chiral column: Chiralcel OD-3 150 mm * 4.6 mm I.D., 3 µm; mobile phase: A: CO₂, B: isopropanol (0.05% DEA); gradient: Mobile phase B increased from 5% to 40% in 5 minutes, then decreased from 40% to 5% in 30 seconds and remained for 1.5 minutes; flow rate: 2.5 mL/min; column temperature: 35°C; ABPR: 1500 psi; retention time: t = 3.700 min.

**16G-b** (130 mg, yield: 32.50%), white solid, retention time: t = 4.046 min.

Separation conditions of SFC: chiral column: Chiralcel OD-3 150 mm * 4.6 mm I.D., 3 µm; mobile phase: A: CO₂ B: isopropanol (0.05% DEA); gradient: Mobile phase B increased from 5% to 40% in 5 minutes, then decreased from 40% to 5% in 30 seconds and remained for 1.5 minutes; flow rate: 2.5 mL/min; column temperature: 35°C; ABPR: 1500 psi; retention time: t = 4.046 min.

### Step 8: Preparation of compounds 16H-aa, ab, ba and bb.

At -20°C, Pd(OAc)₂ (55.84 mg, 248.74 µmol) was added to a tetrahydrofuran (10 mL) solution of compound **16G-a** (120 mg, 248.74 µmol), and then a solution of CH₂N₂ (0.2 M, 6.22 mL) was slowly added dropwise thereto, and the reaction mixture was reacted at room temperature for 16 hours. After the reaction was completed, the reaction mixture was concentrated under reduced pressure to obtain a crude product, which was first purified by column chromatography (ethyl acetate/petroleum ether =15%), and then subjected to chiral resolution to obtain compound **16H-aa** (45 mg, yield: 36.44%) as a white solid and compound **16H-ab** (40 mg, yield: 32.39%) as a white solid.

**¹H NMR (400MHz, CHLOROFORM-d)** δ = 7.21 (d, J = 8.5 Hz, 1H), 6.84 (d, J = 8.3 Hz, 1H), 4.00 (br d, J = 7.3 Hz, 1H), 2.94 - 2.85 (m, 1H), 2.32 - 2.24 (m, 1H), 2.17 (br dd, J = 8.6, 14.4 Hz, 1H), 1.85 - 1.78 (m, 2H), 1.76 (br s, 2H), 1.75 (s, 3H), 1.71 (s, 3H), 1.44 (d, J = 6.6 Hz, 3H), 1.18 (d, J = 3.9 Hz, 7H), 1.15 - 1.10 (m, 1H), 1.08 - 1.00 (m, 1H), 1.05 (br dd, J = 4.8, 13.0 Hz, 1H), 0.73 (s, 3H), 0.65 - 0.59 (m, 2H), 0.55 - 0.47 (m, 2H), 0.43 - 0.32 (m, 2H). MS (ESI) m/z (M+ H)+=497.4.

**16H-ab** (40 mg, yield: 32.39%), white solid.

**¹H NMR (400MHz, CHLOROFORM-d)** δ = 7.23 (d, J = 8.5 Hz, 1H), 6.82 (d, J = 8.5 Hz, 1H), 4.02 (d, J = 6.8 Hz, 1H), 2.85 - 2.78 (m, 1H), 2.34 - 2.26 (m, 1H), 2.15 - 2.07 (m, 1H), 2.02 - 1.95 (m, 1H), 1.85 (t, J = 5.6 Hz, 1H), 1.73 (d, J = 3.5 Hz, 6H), 1.41 (d, J = 6.5 Hz, 3H), 1.33 (br s, 1H), 1.29 - 1.23 (m, 2H), 1.19 (s, 3H), 1.15 - 1.10 (m, 1H), 1.08 (s, 3H), 1.04 - 0.99 (m, 1H), 0.72 (s, 3H), 0.63 (br d, J = 8.8 Hz, 3H), 0.58 - 0.52 (m, 1H), 0.43 - 0.38 (m, 1H), 0.36 - 0.31 (m, 1H). MS (ESI) m/z (M+ H)+=497.4.

Separation conditions of SFC: chiral column: Chiralpak IC-3 100 mm * 4.6 mm I.D., 3 µm; mobile phase: A: CO₂ B: methanol (0.05% DEA); gradient: Mobile phase B increased from 5% to 40% in four minutes and remained for 2.5 minutes, and then the mobile phase remained at 5% for 1.5 minutes; flow rate: 2.8 mL/min; column temperature: 35°C; ABPR: 1500 psi; retention time: t = 2.873 min.

HPLC conditions: mobile phase: 2.75 mL/4 L TFA/water (mobile phase A), 2.5 mL/4 L TFA/acetonitrile (mobile phase B), using mobile phase B to increase from 30% to 90% within 6 minutes and with a retention time of 2 min at a flow rate of 1.2 mL/min; chromatographic column: Ultimate C18 3.0 * 50 mm, 3 µm; wavelength: UV220 nm, 215 nm, 254 nm; column temperature: 40 °C; retention time: t = 6.36 min.

At -20°C, CH₂N₂ (10.45 mg, 248.74 µmol) was added to a solution of compound **16G-b** (120.00 mg, 248.74 µmol) and Pd(OAc)₂ (55.84 mg, 248.74 µmol) in tetrahydrofuran (20 mL), and the reaction was carried out at room temperature for 16 hours. After the reaction was completed, the reaction mixture was concentrated under reduced pressure to obtain a crude product, which was purified by column chromatography (ethyl acetate/petroleum ether =15%), and then subjected to chiral resolution to obtain compound **16H-ba** (45 mg, yield: 36.44%, retention time: t = 3.711 min.) as a white solid and compound **16H-bb** (40 mg, yield: 32.39%, retention time: t =4.242 min.) as a white solid.

SFC conditions: chiral column: (S,S)Whelk-01 100 × 4.6 mm I.D., 5.0 µm; mobile phase: A: CO₂ B: ethanol (0.05% DEA); gradient: mobile phase B increased from 5% to 40% in 4.5 minutes and remained for 1.5 minutes; flow rate: 2.5 mL/min; column temperature: 40°C; ABPR: 100 bar;
HPLC analysis conditions: mobile phase: 2.75 mL/4 L TFA/water (mobile phase A), 2.5 mL/4 L TFA/acetonitrile (mobile phase B), using mobile phase B to increase from 30% to 90% within 6 minutes with a retention time of 2 min at a flow rate of 1.2 mL/min; chromatographic column: Ultimate C18 3.0 * 50 mm, 3 µm; wavelength: UV220 nm, 215 nm, 254 nm; column temperature: 40°C.

### Step 9: Preparation of compounds 16-a, b, c, d

At room temperature, sodium hydroxide aqueous solution (3 M, 80 µL) was added to a mixed solvent of acetonitrile (1 mL) and water (0.5 mL) in which compound **16H-aa** (40 mg, 80 µmol) was dissolved, and then the reaction mixture was heated to 60°C and stirred for 2 hours. The reaction mixture was cooled to room temperature, and isobutylboronic acid (41 mg, 0.4 mmol) and hydrochloric acid (100 uL) were added to the above reaction mixture, and the mixture was stirred at room temperature for another half an hour. After the reaction was completed, the reaction mixture was concentrated under reduced pressure to obtain a crude product, which was purified by HPLC (chromatographic column: Boston Green ODS 150 * 30 mm * 5 µm; mobile phase: [water (HCl)-ACN]; B%: 40%-60%, 10 min) to obtain compound **16-a** (10 mg, yield: 41%).

**¹H NMR (400 MHz, Methanol-d4)** δ 7.12 (d, J = 7.9 Hz, 1H), 6.96 (d, J = 7.9 Hz, 1H), 2.39 (dq, J = 9.5, 6.7 Hz, 1H), 2.11 (td, J = 8.0, 4.2 Hz, 1H), 1.19 (ddd, J = 11.1, 7.9, 3.6 Hz, 1H), 1.07 (d, J = 6.7 Hz, 3H), 0.65 (dddd, J = 13.0, 9.6, 8.1, 4.9 Hz, 1H), 0.42 (ddd, J = 10.4, 8.1, 6.1 Hz, 1H), 0.35 - 0.25 (m, 2H), 0.18 (dt, J = 6.2, 3.9 Hz, 1H), 0.05 -0.06 (m, 2H). **MS (ESI)** *m*/*z* (M+ H)⁺=304.7.

### Using 16H-ab as the starting material and the same synthetic preparation method as that of 16-a, 17.88 mg of 16b was obtained:

**¹H NMR (400 MHz, Methanol-d4)** δ 7.13 (d, J = 7.9 Hz, 1H), 6.97 (d, J = 7.9 Hz, 1H), 2.40 (dq, J = 9.5, 6.7 Hz, 1H), 2.11 (td, J = 8.0, 4.1 Hz, 1H), 1.19 (ddd, J = 10.4, 7.9, 3.6 Hz, 1H), 1.08 (d, J = 6.7 Hz, 3H), 0.71 - 0.61 (m, 1H), 0.42 (ddd, J = 10.4, 8.1, 6.2 Hz, 1H), 0.34 - 0.24 (m, 2H), 0.19 (dt, J = 6.2, 3.9 Hz, 1H), -0.00 (ddt, J = 6.4, 4.9, 2.2 Hz, 2H). MS (ESI) *m*/*z* (M+ H)⁺=304.7.

### Using 16H-ba as the starting material and the same synthetic preparation method as that of 16-a, 15.4 mg of 16-c was obtained:

**¹H NMR (400 MHz, Methanol-d4)** δ 7.12 (d, J = 7.9 Hz, 1H), 6.96 (d, J = 7.9 Hz, 1H), 2.39 (dq, J = 9.5, 6.7 Hz, 1H), 2.11 (td, J = 8.0, 4.1 Hz, 1H), 1.22 - 1.17 (m, 1H), 1.07 (d, J = 6.7 Hz, 3H), 0.69 - 0.61 (m, 1H), 0.45 - 0.39 (m, 1H), 0.34 - 0.26 (m, 2H), 0.18 (dt, J = 6.2, 3.9 Hz, 1H), 0.04 - -0.04 (m, 2H). MS (ESI) m/z (M+ H)+=304.7.

### Using 16H-bb as the starting material and the same synthetic preparation method as that of 16-a, 21.66 mg of 16-d was obtained:

**¹H NMR (400 MHz, Methanol-d4)** δ 7.13 (d, J = 7.9 Hz, 1H), 6.97 (d, J = 7.9 Hz, 1H), 2.40 (dq, J = 9.5, 6.7 Hz, 1H), 2.11 (td, J = 8.0, 4.1 Hz, 1H), 1.20 (ddd, J = 10.5, 8.0, 3.6 Hz, 1H), 1.08 (d, J = 6.7 Hz, 3H), 0.70 - 0.62 (m, 1H), 0.42 (ddd, J = 10.4, 8.1, 6.1 Hz, 1H), 0.34 - 0.26 (m, 2H), 0.19 (dt, J = 6.2, 3.9 Hz, 1H), -0.00 (tdd, J = 4.9, 2.8, 1.6 Hz, 2H). MS (ESI) m/z (M+ H)+=304.7.

### Example 17: Synthesis of compound 17

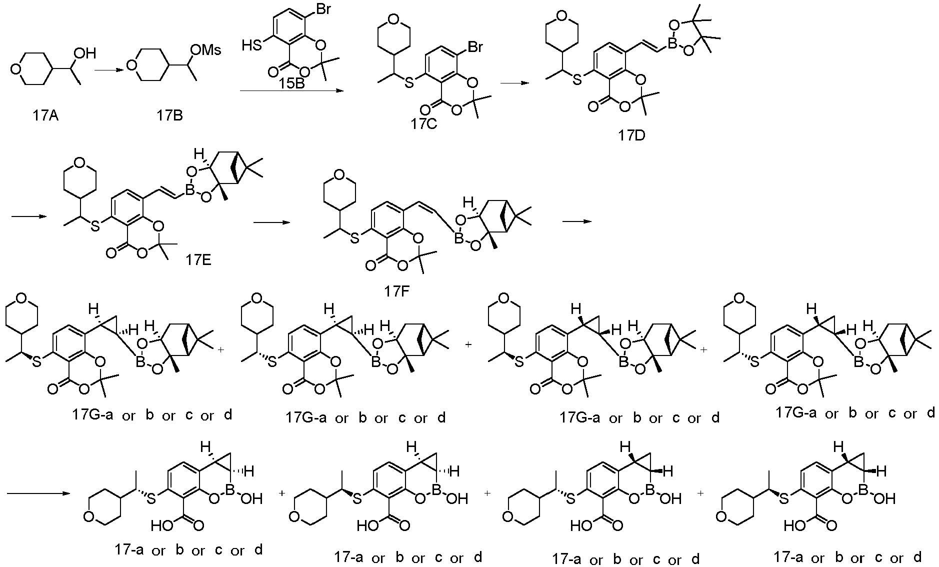

### Step 1: Preparation of compound 17B

At -10°C, methanesulfonyl chloride (210 mg, 1.84mmol) was slowly added dropwise to a solution of compound **17A** (200 mg, 1.54 mmol) and triethylamine (233 mg, 2.31 mmol) in dichloromethane (4 mL), and the reaction mixture was stirred for 1 hour. After the reaction was completed, the reaction mixture was returned to room temperature, washed with water (10 mL), extracted with dichloromethane (20 mL × 3), and the organic phase was collected, dried with anhydrous sodium sulfate, filtered and concentrated under reduced pressure to obtain compound **17B** (319 mg, yield: 99%).

### Step 2: Preparation of compound 17C

At room temperature, compound **15B** (70 mg, 0.24 mmol) and potassium carbonate (50.0 mg, 0.36 mmol) were dissolved in N,N-dimethylformamide (1.5 mL), and a DMF solution of **17B** (60.0 mg, 0.28 mmol) was added dropwise thereto under nitrogen atmosphere. After the addition, the reaction mixture was heated to 100°C and stirred for 1 hour. After the reaction was completed, the reaction mixture was washed with water, extracted with ethyl acetate (5 mL × 3). The organic phase was collected, washed with saturated brine, dried with anhydrous sodium sulfate, filtered, concentrated under reduced pressure to obtain a crude product, which was purified by column chromatography (petroleum ether: ethyl acetate = 5:1) to obtain compound **17C** (45 mg, yield: 46%). MS(ESI)m/z:(M+H)⁺= 401.0.

### Step 3: Preparation of compound 17D

At room temperature, **17C** (40.0 mg, 0.1 mmol), pinacol vinylboronate (46.0 mg, 0.3 mmol), tris[dibenzylideneacetone]dipalladium (0) (9.15 mg, 0.1 mmol), tri-tert-butylphosphine tetrafluoroborate (6.0 mg, 0.02 mmol) and N,N-diisopropylethylamine (40 mg, 0.3 mmol) were dissolved in toluene (1.5 mL), and the system was replaced with nitrogen for three times, heated to 115°C and stirred for two hours. After the reaction was completed, the reaction mixture was returned to room temperature, washed with water, and extracted with ethyl acetate (3 mL × 3). The organic phases were combined, washed with saturated brine, dried with anhydrous sodium sulfate, filtered and concentrated directly under reduced pressure to obtain a crude product, which was purified by column chromatography (petroleum ether: ethyl acetate = 10:1) to obtain compound **17D** (26 mg, yield: 55%). MS(ESI)m/z:(M+H)⁺= 475.2.

### Step 4: Preparation of compound 17E

At room temperature, (1S,2S,3R,5S)-(+)-2,3-pinanediol (591.0 mg, 3.48 mmol) was added to a tetrahydrofuran (6 mL) solution of compound **17D** (550.0 mg, 1.16 mmol), and the reaction mixture was stirred for 2 hours. After the reaction was completed, the reaction mixture was concentrated under reduced pressure to obtain a crude product, which was purified by column chromatography (petroleum ether: ethyl acetate = 10:1) to obtain compound **17E** (520.0 mg, yield: 85.2%). MS (ESI) m/z (M- H) + = 527.2.

### Step 5: Preparation of compound 17F

Compound **17E** (100.0 mg, 0.19 mmol) was dissolved in dichloromethane (9 mL), and tris(2-phenylpyridine)iridium (5.00 mg) was added thereto at room temperature. After being irradiated by LED blue light for 1.5 hours under nitrogen atmosphere, the reaction mixture was concentrated under reduced pressure to obtain a crude product, which was purified by column chromatography (petroleum ether: ethyl acetate =5:1) to obtain compound **17F** (55 mg, yield: 55%). MS (ESI) m/z (M+ H) ⁺ = 527.2.

### Step 6: Preparation of compound 17G

In an ice bath and under nitrogen atmosphere, an ether solution (2.5 mL, 2.5 mmol) of diazomethane was added into a solution of compound **17F** (130.0 mg, 0.25 mmol) and palladium acetate (5.50 mg, 0.024 mmol) in tetrahydrofuran (6 mL), and the reaction mixture was stirred for 10 minutes. After the reaction was completed, the reaction mixture was filtered and concentrated under reduced pressure to obtain a crude product, which was purified by column chromatography (petroleum ether: ethyl acetate = 10:1) to obtain compound **A3-7** (105.0 mg, yield: 79.0%). MS (ESI) m/z (M+ H) ⁺ = 540.2.

**17G** (220 mg, 0.41 mmol) was resolved by a chiral preparative column to obtain four compounds, **17G-a** (30.5 mg, retention time t = 8.843), **17G-b** (35.2 mg, retention time t = 10.024), **17G-c** (26.5 mg, retention time t = 11.146) and **17G-d** (36.5 mg, retention time t = 13.983).

Chiral resolution conditions: chromatographic column: Daicel CHIRALPAK^{®} IB250 * 30 mm, 10 µm; Mobile phase A: n-hexane; Mobile phase B: ethanol; Detection wavelength: 254 nm; Flow rate: 25 mL/min; Isocratic elution procedure: mobile phase A: mobile phase B = 95:05 (v/v).

Chiral analysis conditions: chromatographic column: Daicel CHIRALPAK^{®} IB 250 * 4.6 mm, 5 µm: mobile phase A: n-hexane; Mobile phase B: ethanol; Detection wavelength: 254 nm; Flow rate: 1 mL/min; Column temperature: 30°C; Time: 20 min; Isocratic elution procedure: mobile phase A: mobile phase B = 95:05 (v/v).

### Step 7: Preparation of compounds 17-a, b, c, d

At room temperature, NaOH solution (3 M, 150 µL) was added to a mixed solution of acetonitrile (0.6 mL) and water (0.2mL) in which compound **17G-a** (25 mg, 46.3 umol) was dissolved, and then the reaction mixture was heated to 60°C and stirred for 1.5 hours. In an ice bath, the reaction mixture was added with triethylsilane (0.2 mL), trifluoroacetic acid (1 mL) and isobutylboronic acid (9.41 mg, 92.34 µmol) in sequence and stirred at room temperature for 1 hour. After the reaction was completed, the reaction mixture was concentrated under reduced pressure to obtain a crude product, which was purified by HPLC to obtain **17-a** (10.42 mg).

**¹H NMR (400 MHz, Methanol-d4)** δ 7.23 (d, J = 8.0 Hz, 1H), 7.04 (d, J = 8.0 Hz, 1H), 3.98 - 3.72 (m, 2H), 3.33 - 3.23 (m, 2H), 3.11-3.00 (m, 1H), 2.26-2.15 (m, 1H), 1.71 - 1.53 (m, 3H), 1.49 - 1.23 (m, 3H), 1.11 (d, J = 6.8 Hz 3H), 0.55-0.45 (m, 1H), 0.31-0.25 (m, 1H). MS (ESI) m/z (M-1)-=347.0.

HPLC 100.00% (254 nM); retention time: t = 2.70 min.

**Separation conditions:** Chromatographic column: Welch Xtimate ^{®} C18 21.2 × 250 mm; Column temperature: 25°C; Mobile phase A. Water (10.0 mM/L ammonium bicarbonate solution); Mobile phase B: Acetonitrile; Gradient: 0-45%, 15 min; flow rate 18.0 mL/min.

**Analysis conditions:** Chromatographic column: Waters X Bridge 4.6 * 100 mm, 3.5 µm; Column temperature: 40°C; Mobile phase A: Water (10.0 mM/L ammonium bicarbonate solution); Mobile phase B: Acetonitrile; Gradient: 5%-95%, 7 min; flow rate 1.0 mL/min.

**20.46 mg of 17-b was prepared using 17G-b as raw material, and the method was the same as that of 17-a.**

**¹H NMR (400MHz, Methanol -d4)** δ 7.21 (d, J = 8.0 Hz, 1H), 7.00 (d, J = 8.0 Hz, 1H), 3.93 - 3.73 (m, 2H), 3.35 - 3.23 (m, 2H), 3.12-3.01 (m, 1H), 2.18-2.07(m, 1H), 1.71-1.55 (m, 3H), 1.46 - 1.17 (m, 3H), 1.11 (d, J = 6.8 Hz, 3H), 0.53-0.39 (m, 1H), 0.33-0.24 (m, 1H).

**7.91 mg of 17-c was prepared using 17G-c as raw material, and the method was the same as that of 17-a.**

**¹H NMR (400 MHz, Methanol-d4)** δ 7.23 (d, J = 7.9 Hz, 1H), 7.04 (d, J = 8.0 Hz, 1H), 3.88 - 3.82 (m, 2H), 3.29 - 3.23 (m, 2H), 3.07 - 3.02 (m, 1H), 2.19 (dt, J = 8.0, 4.0 Hz, 1H), 1.62 (tt, J = 8.6, 3.2 Hz, 3H), 1.37 (dtd, J = 11.0, 3.3, 1.7 Hz, 2H), 1.31 - 1.26 (m, 1H), 1.12 (d, J = 6.9 Hz, 3H), 0.50 (ddd, J = 10.4, 8.1, 6.1 Hz, 1H), 0.28 (dt, J = 6.2, 3.9 Hz, 1H). **MS (ESI)** *m*/*z* (M- H)⁻=306.8.

**9.32 mg of 17-d was prepared using 17G-d as raw material, and the method was the same as that of 17-a.**

**¹H NMR (400 MHz, Methanol-d4)** δ 7.23 (d, J = 7.9 Hz, 1H), 7.04 (d, J = 7.8 Hz, 1H), 3.88 - 3.82 (m, 2H), 3.27 (dtd, J = 11.9, 5.9, 2.0 Hz, 2H), 3.06 (dd, J = 7.0, 5.0 Hz, 1H), 2.19 (td, J = 8.0, 4.1 Hz, 1H), 1.67 - 1.58 (m, 3H), 1.43 - 1.33 (m, 2H), 1.31 - 1.25 (m, 1H), 1.12 (d, J = 6.9 Hz, 3H), 0.50 (ddd, J = 10.5, 8.1, 6.2 Hz, 1H), 0.28 (dt, J = 6.2, 3.8 Hz, 1H). MS (ESI) m/z (M- H)-=306.8.

### Example 18: Synthesis of compound 18

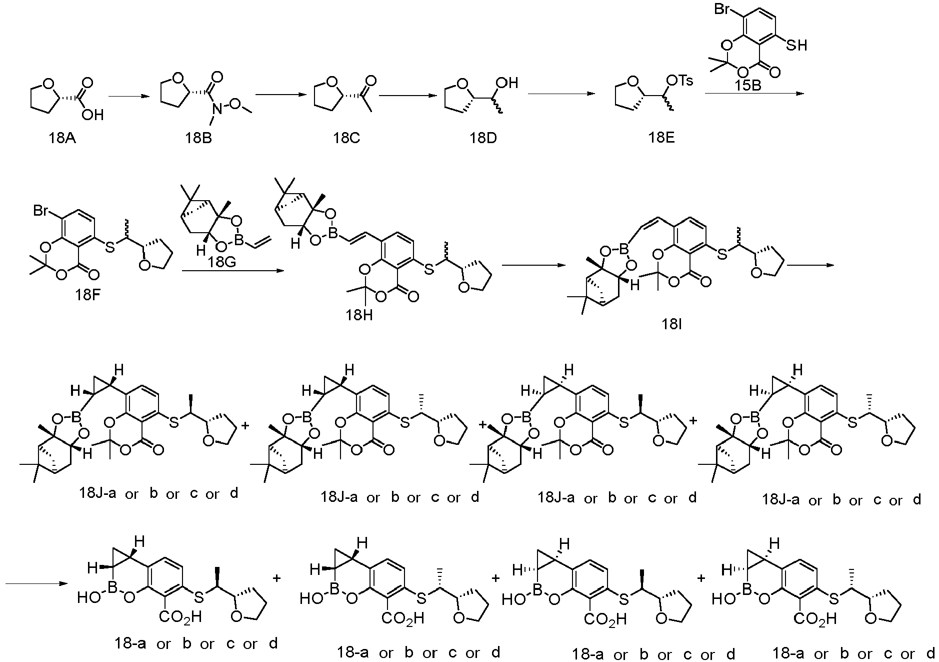

### Step 1: Preparation of compound 18B

Compound **18A** (1.0 g, 8.62 mmol) and dimethylhydroxylamine hydrochloride (930.0 mg, 9.48 mmol, 1.1 equiv) were dissolved in a solution of dichloromethane (20.0 mL), and N,N'-carbonyldiimidazole (1536.0 mg, 9.48 mmol, 1.1 equiv) was added to the above reaction mixture in batches, and the reaction mixture was stirred at room temperature for 16.0 hours. After the reaction was completed, the reaction mixture was quenched with hydrochloric acid (1.0 N) and extracted with ethyl acetate (40 mL × 3). The organic phase was collected, washed with sodium hydroxide aqueous solution (100.0 mL, 1.0N), dried, filtered and concentrated to obtain crude product **18B** (1.0 g, yield: 73.0%, colorless liquid), which was directly used in the next step without further purification.

**¹H NMR (400 MHz, Chloroform-*d*)** δ 4.75 - 4.62 (m, 1H), 3.85 - 3.72 (m, 2H), 3.67 (s, 3H), 3.09 (s, 3H), 2.15 - 2.03 (m, 1H), 1.92 - 1.73 (m, 3H).

### Step 2: Preparation of compound 18C

Methylmagnesium bromide (2.3 mL, 3.0 M in THF) was slowly added dropwise to a solution of compound **18B** (1.0 g, 6.28 mmol) in tetrahydrofuran (10.0 mL) at 0°C under argon atmosphere, and the reaction mixture was stirred for 1 hour at 0°C. After the reaction was completed, the reaction mixture was quenched with saturated ammonium chloride aqueous solution, extracted with ethyl acetate (40 mL × 3), dried, filtered, and concentrated under reduced pressure to obtain crude product **18C** (380.0 mg, yield: 53.0%, yellow liquid), which was directly used in the next step without further purification.

**¹H NMR (400 MHz, Chloroform-*d*)** δ 4.27 - 4.22 (m, 1H), 3.84 - 3.75 (m, 2H), 2.10 (s, 3H), 2.09 - 2.01 (m, 1H), 1.86 - 1.73 (m, 3H).

### Step 3: Preparation of compound 18D

Sodium borohydride (253.0 mg, 6.67 mmol, 2.0 equiv) was added to a solution of compound **18C** (380.0 mg, 3.33 mmol) in tetrahydrofuran (5.0 mL) in batches at 0°C, and the reaction mixture was slowly warmed to room temperature and stirred overnight. After the reaction was completed, the reaction mixture was quenched with hydrochloric acid (1.0 N), extracted with ethyl acetate (10 mL × 3), and the organic phase was collected, dried, filtered, and concentrated under reduced pressure to obtain crude product **18D** (126.0 mg, yield: 33.0%, yellow liquid), which was directly used in the next step without further purification.

**¹H NMR (400 MHz, Chloroform-*d*)** δ 4.57 - 4.39 (m, 1H), 3.76 - 3.67 (m, 1H), 3.65 - 3.58 (m, 1H), 3.58 - 3.39 (m, 2H), 1.87 - 1.65 (m, 4H), 1.04 - 0.97 (m, 3H).

### Step 4: Preparation of compound 18E

Compound **18D** (6.0 g, 51.72 mmol) and triethylamine (14.35 mL, 103.44 mmol, 2.0 equiv) were dissolved in dichloromethane (50.0 mL), and then 4-dimethylaminopyridine (634.0 mg, 5.2 mmol, 0.1 equiv) and p-toluenesulfonyl chloride (19.757 g, 103.44 mmol, 2.0 equiv) were added thereto in sequence, and the reaction mixture was stirred at room temperature for 16 hours. After the reaction was completed, the reaction mixture was concentrated under reduced pressure to obtain a crude product, which was purified by column chromatography (ethyl acetate/petroleum ether= 0 to 10%) to obtain compound **18E** (7.0 g, yield: 50.1%, colorless liquid). **MS (ESI)** m/z (M+ H)⁺ = 271.

### Step 5: Preparation of compound 18F

A solution of compound **15B** (1.0 g, 3.46 mmol) and cesium carbonate (2256.0 mg, 6.92 mmol, 2.0 equiv) in N,N-dimethylformamide (10.0 mL) was stirred at room temperature for 5.0 min under argon atmosphere, then **18E** (1.4 g, 5.19 mmol, 1.5 equiv) was added thereto and stirred at room temperature for 1.0 hour. After the reaction was completed, the reaction mixture was quenched with saturated ammonium chloride aqueous solution (100.0 mL) and extracted with ethyl acetate (30 mL × 3). The organic phase was collected, dried with anhydrous sodium sulfate, filtered and concentrated to obtain a crude product, which was purified by column chromatography (ethyl acetate/petroleum ether = 0 to 10%) to obtain compound **18F** (1.0 g, yield: 75.0%, yellow liquid). **MS (ESI)** m/z (M+ H)⁺ = 388.8.

### Step 6: Preparation of compound 18G

At room temperature, (1S,2S,3R,5S)-(+)-2,3-pinanediol (1.3 g, 7.79 mmol) was added to a solution of pinacol vinylboronate (1 g, 6.49 mmol) in tetrahydrofuran (10 mL) and stirred for 20 hours. After the reaction was completed, the reaction mixture was concentrated under reduced pressure to obtain a crude product, which was purified by column chromatography (PE: EA = 9:1) to obtain compound **18G** (1.3 g, yield: 97.15%).

### Step 7: Preparation of compound 18H

A solution of compounds **18F** (1.0 g, 2.58 mmol), **18G** (738 mg, 3.1 mmol, 1.2 equiv), tris(dibenzylideneacetone)dipalladium (473.0 mg, 0.517 mmol, 0.2 equiv), tri-tert-butylphosphine tetrafluoroborate (297.0 mg, 1.03 mmol, 0.4 equiv), and diisopropylethylamine (0.674 mL, 3.87 mmol, 1.5 equiv) in acetonitrile (20.0 mL) was replaced with argon four times and then heated to 100°C and stirred for 1.0 hour. After the reaction was completed, the reaction mixture was filtered and concentrated under reduced pressure to obtain a crude product, which was purified by column chromatography (ethyl acetate/petroleum ether = 0 to 15%) to obtain compound **18H** (1.2 g, yield: 90.8%). **MS (ESI)** m/z (M+ H) ⁺ = 513.0.

### Step 8: Preparation of compound 18I

Tris(2-phenylpyridine)iridium (15.0 mg) was added to a solution of compound **18H** (300.0 mg) in dichloromethane (6.0 mL), and the system was replaced with argon four times, and then the reaction mixture was stirred at room temperature for 2.0 hours under blue light irradiation at a wavelength of 460 to 465 nm. After the reaction was completed, the reaction mixture was concentrated under reduced pressure to obtain a crude product, which was purified by column chromatography (ethyl acetate/petroleum ether = 0 to 15%) to obtain compound **18I** (175.0 mg, yield: 58%). MS (ESI) m/z (M+H) ⁺ = 513.0.

### Step 10: Preparation of compounds 18J-a & 18J-b & 18J-c & 18J-d

Ether solution of diazomethane (40 mmol) was added to a solution of compound **18I** (2.1 g, 4.1 mmol) and palladium acetate (92 mg, 0.41 mmol, 0.1 equiv) in tetrahydrofuran (60.0 mL), and the reaction mixture was stirred at room temperature for 15 min. After the reaction was completed, the reaction mixture was concentrated under reduced pressure to obtain a residue, which was purified by column chromatography (ethyl acetate/petroleum ether (v/v) = 0 to 5%) to obtain a crude product, which was then purified by chiral separation to obtain **18J-a** (324.0 mg) and **18J- b** (167.0 mg).
Chiral separation conditions: Chromatographic column: Daicel CHIRALPAK^{®} IE250 * 30 mm, 10 µm; mobile phase: [n-hexane (0.2% DEA) - ethanol (0.2% DEA); Ratio of n-hexane to ethanol in mobile phase: 90:10 (V/V); flow rate 25.0 mL/min]
**18J-c** (232.0 mg), **18J-d** (284.0 mg)
Chiral separation conditions: Chromatographic column: Daicel CHIRALPAK^{®} IE250 * 30 mm, 10 µm; mobile phase: [n-hexane (0.2% DEA) - ethanol (0.2% DEA)]; Ratio of n-hexane to ethanol in mobile phase: 95:05 (V/V); flow rate 25.0 mL/min.
**18J-a:** retention time: t = 25.930 min, HPLC: 99.72%;
**18J-b:** retention time: t = 29.544 min, HPLC: 97.48%;
**18J-c:** retention time: t = 5.123 min, HPLC: 91.2%;
**18J-d:** retention time: t = 5.650 min, HPLC: 98.83%;
**MS (ESI)** m/z (M+ H) ⁺ = 527.0.

### Step 11: Preparation of compounds 18-a, b, c, d

Aqueous sodium hydroxide solution (0.3 mL, 3.0 N) was added to a solution of compound **18J-a** (50.0 mg) in acetonitrile (0.5 mL), and the reaction mixture was stirred for 3.0 hours in a 65°C oil bath. After the reaction was completed, the reaction mixture was returned to room temperature, added with hydrochloric acid to adjust the pH to 1-2, then added with isobutylboronic acid (30.0 mg) and stirred for another 10 min. After the reaction was completed, the mixture was purified by high performance preparative liquid chromatography to obtain compound **18-a** (16.7 mg).

**HPLC separation conditions:** Chromatographic column: Welch Xtimate ^{®} C18 21.2 × 250 mm; Column temperature: 25°C; Mobile phase: [Water (10.0 mM/L formic acid solution)-acetonitrile]; acetonitrile ratio in mobile phase: 40%-45%, 15 min; flow rate 18.0 mL/min.

**¹H NMR (400 MHz, DMSO-d6)** δ 12.97 (s, 1H), 9.33 (s, 1H), 7.33 (d, J = 8.0 Hz, 1H), 7.10 (d, J = 7.9 Hz, 1H), 3.84 - 3.70 (m, 2H), 3.61 (dt, J = 8.0, 6.6 Hz, 1H), 3.32 - 3.29 (m, 1H), 2.28 (td, J = 8.0, 4.0 Hz, 1H), 1.96 - 1.86 (m, 1H), 1.86 - 1.77 (m, 2H), 1.73 - 1.60 (m, 1H), 1.33 (ddd, J = 10.9, 7.9, 3.3 Hz, 1H), 1.14 (d, J = 6.9 Hz, 3H), 0.53 (ddd, J = 10.4, 8.0, 6.0 Hz, 1H), 0.32 (dt, J = 6.1, 3.7 Hz, 1H). **MS (ESI)** m/z (M+ H-18) ⁺ = 317.0.

**Compound 18-b was synthesized using 18J-b as starting material, and the preparation process was the same as that of 18-a to obtain 15.9 mg of 18-b.**

**¹H NMR (400 MHz, DMSO-d6)** δ 12.96 (s, 1H), 9.33 (s, 1H), 7.33 (d, J = 7.9 Hz, 1H), 7.11 (d, J = 7.9 Hz, 1H), 3.74 (dq, J = 14.2, 6.9 Hz, 2H), 3.63 (td, J = 7.6, 5.9 Hz, 1H), 3.24 (p, J = 6.7 Hz, 2H), 2.28 (td, J = 8.0, 4.0 Hz, 1H), 1.93 (dtd, J = 11.9, 7.1, 4.9 Hz, 1H), 1.85 - 1.74 (m, 2H), 1.66 - 1.56 (m, 1H), 1.33 (ddd, J = 11.0, 7.9, 3.4 Hz, 1H), 1.15 (d, J = 6.8 Hz, 3H), 0.54 (ddd, J = 10.5, 8.1, 6.1 Hz, 1H), 0.31 (dt, J = 6.4, 3.7 Hz, 1H). **MS (ESI)** m/z (M+H-18) ⁺ = 317.0.

**Compound 18-c was synthesized using 18J-c as starting material, and the preparation process was the same as that of 18-a to obtain 15.4 mg of 18-c.**

**¹H NMR (400 MHz, DMSO-d6)** δ 12.97 (s, 1H), 9.33 (s, 1H), 7.33 (d, J = 8.0 Hz, 1H), 7.11 (d, J = 8.0 Hz, 1H), 3.80 - 3.69 (m, 2H), 3.63 (td, J = 7.7, 5.9 Hz, 1H), 3.25 (t, J = 6.7 Hz, 1H), 2.28 (td, J = 7.9, 4.0 Hz, 1H), 2.00 - 1.85 (m, 1H), 1.86 - 1.72 (m, 3H), 1.65 - 1.55 (m, 1H), 1.33 (ddd, J = 10.9, 7.9, 3.4 Hz, 1H), 1.15 (d, J = 6.8 Hz, 3H), 0.53 (ddd, J = 10.5, 8.0, 6.1 Hz, 1H), 0.32 (dt, J = 6.0, 3.7 Hz, 1H). **MS (ESI)** m/z (M+H-18) ⁺ = 317.0.

**Compound 18-b was synthesized using 18J-d as starting material, and the preparation process was the same as that of 18-a to obtain 17.3 mg of 18-d.**

**¹H NMR (400 MHz, DMSO-d6)** δ 12.97 (s, 1H), 9.33 (s, 1H), 7.33 (d, J = 8.0 Hz, 1H), 7.10 (d, J = 8.0 Hz, 1H), 3.88 - 3.69 (m, 2H), 3.61 (dt, J = 8.0, 6.6 Hz, 1H), 3.33 (s, 1H), 2.28 (td, J = 7.9, 4.0 Hz, 1H), 1.97 - 1.87 (m, 1H), 1.81 (dtt, J = 10.1, 7.3, 4.9 Hz, 2H), 1.73 - 1.62 (m, 1H), 1.33 (ddd, J = 11.0, 7.9, 3.4 Hz, 1H), 1.13 (d, J = 6.9 Hz, 3H), 0.53 (ddd, J = 10.5, 8.1,6.1 Hz, 1H), 0.31 (dt, J = 6.0, 3.7 Hz, 1H). **MS (ESI)** m/z (M+H-18) ⁺ = 317.0.

### Example 19: Synthesis of compound 19

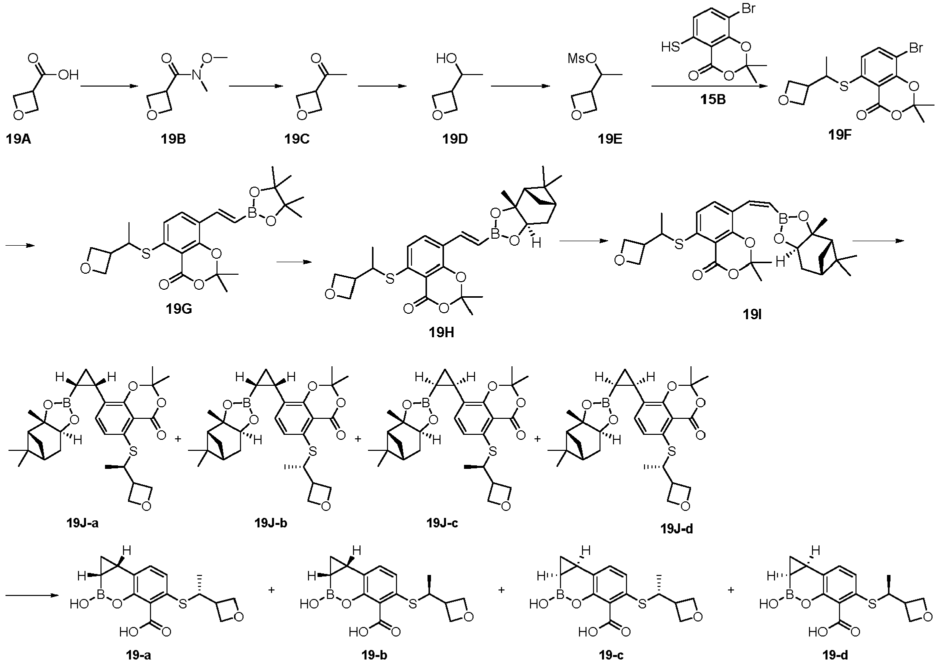

### Step 1: Preparation of compound 19B

At room temperature, N,N-carbonyldiimidazole (8.74 g, 54 mmol) was added to a solution of compound **19A** (5.00 g, 49 mmol) and N,O-dimethylhydroxylamine hydrochloride (4.78 g, 49 mmol) in dichloromethane (100 mL), and the reaction mixture was stirred at room temperature for 16 hours. After the reaction was completed, the reaction mixture was quenched by adding water (100 mL), extracted with ethyl acetate (80 mL × 3), and the organic phase was collected, washed with saturated brine, dried with anhydrous sodium sulfate, filtered and concentrated under reduced pressure to obtain a crude product, which was purified by column chromatography (petroleum ether: ethyl acetate = 0-100%) to obtain compound **19B** (6 g, yield: 84.5%).

### Step 2: Preparation of compound 19C

At 0°C, a solution of methylmagnesium bromide (13.1 mL, 1N, 13.1 mmol) was slowly added dropwise to a solution of compound **19B** (950 mg, 6.55 mmol) in tetrahydrofuran (20 mL) under nitrogen atmosphere, and the reaction mixture was stirred for 2 hours. After the reaction was completed, the reaction mixture was returned to room temperature, quenched with water (20 mL), extracted with ethyl acetate (30 mL × 3), and the organic phase was collected, washed with saturated brine, dried with anhydrous sodium sulfate, filtered and concentrated under reduced pressure to obtain a crude product, which was purified by column chromatography (petroleum ether: ethyl acetate = 1:1) to obtain compound **19C** (650 mg, yield: 90%).

**¹H NMR (400 MHz, CDCl3-d)** δ 4.78 (m, 4H), 3.9 (m, 1H), 2.15 (s, 3H).

### Step 3: Preparation of compound 19D

At 0°C, sodium borohydride (451 mg, 11.88 mmol) was added to a solution of compound **19C** in methanol (15 mL) and the reaction mixture was stirred for one hour. After the reaction was completed, the reaction mixture was quenched with water (5 mL), extracted with ethyl acetate (30 mL × 3), and the organic phases were combined, dried, filtered and concentrated under reduced pressure to obtain a crude product, which was purified by column chromatography (dichloromethane: methanol = 10:1) to obtain compound **19D** (1.1 g, yield: 70.5%).

**¹H NMR (400 MHz, DMSO-d6)** δ 4.72-4.70 (d, 1H), 4.57-4.54 (m, 2H), 4.45-4.42 (t, 1H), 4.30-4.26 (t, 1H), 3.86-3.78 ((m, 1H), 2.85-2.76 (m, 1H), 0.97-0.95 (d, 3H).

### Step 4: Preparation of compound 19E

In an ice bath, methanesulfonyl chloride (1.09 g, 9.56 mmol) was added to dichloromethane (6 mL) in which compound **19D** (750 mg, 7.35 mmol) and triethylamine (1.18g, 11.76 mmol) were dissolved, and the reaction mixture was stirred for 2 hours. After the reaction was completed, the reaction mixture was quenched with water, extracted with ethyl acetate (50 mL × 3), washed with saturated brine, dried with anhydrous sodium sulfate, filtered and concentrated under reduced pressure to obtain a crude product **19E** (1.14 g, yield: 86.4%).

**¹H NMR (400 MHz, DMSO-d6)** δ 5.00 (m, 1H). 4.57-4.61 (m, 2H), 4.44-4.47 (t, 1H), 4.36-4.39 (t, 1H), 3.22 (s, 3H), 3.24-3.15 ((m, 1H), 1.28-1.26 (d, 3H).

### Step 5: Preparation of compound 19F

Under nitrogen atmosphere, a solution of compounds **19E** (1.11 g, 6.2 mmol), **15B** (1.78 g, 6.2 mmol), potassium carbonate (1.71 g, 12.4 mmol) and potassium iodide (103 mg, 0.62 mmol) in N,N-dimethylformamide (30 mL) was heated to 90°C and stirred continuously for 2 hours. After the reaction was completed, the reaction mixture was cooled to room temperature, poured into ice water, extracted with ethyl acetate (150 mL × 3), and the organic phases were combined and washed with saturated brine, dried with anhydrous sodium sulfate, filtered and concentrated under reduced pressure to obtain a crude product, which was purified by column chromatography (petroleum ether/ethyl acetate = 5:1) to obtain compound **19F** (1.67 g, yield: 72.2%). MS (ESI) m/z (M+ H) ⁺ = 375.0.

### Step 6: Preparation of compound 19G

A solution of compound **19F** (1.5 g,4.03 mmol), pinacol vinylboronate (3.09 g, 20.2 mmol), tris(dibenzylideneacetone)dipalladium (735 mg, 0.804 mmol), tri-tert-butylphosphine tetrafluoroborate (465 mg, 1.608 mmol) and diisopropylethylamine (777 mg, 6.06 mmol) in acetonitrile (30 mL) was stirred at 90°C for 1 hour under nitrogen atmosphere. After the reaction was completed, the reaction mixture was returned to room temperature, filtered and concentrated under reduced pressure to obtain a crude product, which was purified by column chromatography (petroleum ether/ethyl acetate = 4:3) to obtain compound 19G (1.36 g, yield: 75.9%). MS (ESI) m/z (M+ H) ⁺ = 447.0.

### Step 7: Preparation of compound 19H

At room temperature, (1R,2R,3S,5S)-(-)-2,3-pinanediol (2.06 g, 12.1 mmol) was added to a tetrahydrofuran (30 mL) solution of compound **19G** (1.35 g, 3.03 mmol), and the reaction mixture was stirred for 16 hours. After the reaction was completed, the reaction mixture was concentrated under reduced pressure to obtain a crude product, which was purified by (petroleum ether/ethyl acetate = 5:1) to obtain compound **19H** (900 mg, yield: 59.6%). MS(ESI)m/z:(M+H)⁺ = 499.0.

### Step 8: Preparation of compound 19I

Tris(diphenylpyridine)iridium (24 mg, 0.36 mmol) was added to dichloromethane (7 mL) in which compound **19H** (900 mg,1.8 mmol) was dissolved, and was irradiated using blue light (wavelength: 460-465 nm, 10 W) for 70 min under nitrogen atmosphere. After the reaction was completed, the reaction mixture was concentrated under reduced pressure to obtain a crude product, which was purified by column chromatography (petroleum ether/ethyl acetate = 3:1) to obtain **19I** (550 mg, yield: 61 %). MS(ESI)m/z:(M+H)⁺ = 499.0.

### Step 9: Preparation of compounds 19J-a, 19J- b, 19J- c, and 19J-d

At -20°C, an ether solution (20 mmol) of diazomethane was added to a solution of compound **19I** (500 mg, 1mmol) and Pd(OAc)₂ (22 mg, 0.1mmol) in THF (3 mL) under nitrogen atmosphere, and the reaction mixture was stirred for 16 hours. After the reaction was completed, the reaction mixture was concentrated under reduced pressure to obtain a crude product, which was purified by column chromatography (ethyl acetate/petroleum ether =0 to 40%) to obtain compound **19J**-mixture (500 mg, yield: 97.6%), a yellow oil, which was purified by SFC to obtain four compounds.
**19J-a,** 60 mg, yellow solid, retention time: t = 8.889 min, MS(ESI)M/z:(M+H)⁺= 513.0;
**19J-b,** 69 mg, yellow solid, retention time: t = 9.890 min, MS(ESI)M/z:(M+H)⁺= 513.0;
**19J-c,** 71 mg, yellow solid, retention time: t = 10.979 min, MS(ESI)M/z:(M+H)⁺= 513.0;
**19J-d,** 66 mg, yellow solid, retention time: t = 12.86 min, MS(ESI)M/z:(M+H)⁺= 513.0;

**Chiral resolution conditions:** chromatographic column: Daicel CHIRALPAK^{®} IB250 * 30 mm, 10 µm; Mobile phase A: n-hexane; Mobile phase B: isopropanol; Detection wavelength: 254 nm; Flow rate: 25 mL/min; Isocratic elution procedure: mobile phase A: mobile phase B = 80:20 (V/V).

**Chiral analysis conditions:** chromatographic column: Daicel CHIRALPAK^{®} IB 250 * 4.6 mm, 5 µm: mobile phase A: n-hexane; Mobile phase B: ethanol; Detection wavelength: 254 nm; Flow rate: 1 mL/min; Column temperature: 30°C; Time: 20 min; Isocratic elution procedure: mobile phase A: mobile phase B = 80:20 (V/V).

### Step 13: Preparation of compounds 19-a, 19-b, 19-c and 19-d

At room temperature, sodium hydroxide aqueous solution (3 M, 330 µL) was added to a mixed solvent of acetonitrile (1 mL) and water (0.2 mL) in which compound **19J-a** (40 mg, 78.1 µmol) was dissolved, and then the reaction mixture was heated to 60°C and stirred for 3 hours. The reaction was cooled to room temperature, and triethylsilane (0.2 mL), trifluoroacetic acid (1.2 mL) and isobutylboronic acid (24 mg, 0.234 mmol) were added to the above reaction mixture, and stirred at room temperature for another 1 hour. After the reaction was completed, the reaction mixture was concentrated under reduced pressure to obtain a crude product, which was purified by HPLC to obtain compound **19-a** (18 mg, yield: 72%).

**¹H NMR (400 MHz, Methanol-d4)** δ 7.25 (d, J = 7.9 Hz, 1H), 7.07 (d, J = 7.9 Hz, 1H), 4.63 (dd, J = 8.1, 6.2 Hz, 1H), 4.55 (dd, J = 7.9, 6.3 Hz, 1H), 4.37 (dt, J = 13.1, 6.5 Hz, 2H), 3.45 (dd, J = 9.9, 6.7 Hz, 1H), 3.05 - 2.91 (m, 1H), 2.20 (td, J = 8.0, 4.1 Hz, 1H), 1.28 (ddd, J = 10.3, 8.0, 3.6 Hz, 1H), 1.10 (d, J = 6.7 Hz, 3H), 0.51 (ddd, J = 10.4, 8.1, 6.2 Hz, 1H), 0.33 - 0.24 (m, 1H). HPLC 100.00% (254nM); Retention time: t = 5.379 min. **MS (ESI) m/z (M-1)**-=321.3.

**HPLC separation conditions:** Chromatographic column: Welch Xtimate ^{®} C18 21.2 × 250 mm; Column temperature: 25°C; Mobile phase: [Water-acetonitrile]; acetonitrile ratio in mobile phase: 30%-35%, 15 min; flow rate 18.0 mL/min.

Analysis conditions: Chromatographic column: Waters X Bridge 4.6 * 100 mm, 3.5 µm; Column temperature: 40°C; Mobile phase A: Water (10.0 mM/L ammonium bicarbonate solution); Mobile phase B: Acetonitrile; Gradient: 5%-95%, 7 min; flow rate 1.0 mL/min.

**Compound 19-b was synthesized using 19J-b as starting material, and the preparation process was the same as that of 19-a to obtain 22 mg of 19-b.**

**¹H NMR (400 MHz, Methanol-d4)** δ 7.26 (d, J = 7.9 Hz, 1H), 7.08 (d, J = 7.9 Hz, 1H), 4.64 (dd, J = 8.1, 6.2 Hz, 1H), 4.56 (dd, J = 7.9, 6.3 Hz, 1H), 4.37 (dt, J = 13.2, 6.5 Hz, 2H), 3.45 (dd, J = 9.9, 6.7 Hz, 1H), 3.05 - 2.94 (m, 1H), 2.21 (td, J = 8.0, 4.1 Hz, 1H), 1.30 (ddd, J = 10.4, 7.9, 3.6 Hz, 1H), 1.10 (d, J = 6.7 Hz, 3H), 0.52 (ddd, J = 10.4, 8.1, 6.2 Hz, 1H), 0.29 (dt, J = 6.2, 3.8 Hz, 1H). MS (ESI) m/z (M+ H-18) + = 321.3.

**Compound 19-c was synthesized using 19J-c as starting material, and the preparation process was the same as that of 19-a to obtain 12 mg of 19-c.**

**¹H NMR (400 MHz, Methanol-d4)** δ 7.25 (d, J = 7.9 Hz, 1H), 7.08 (d, J = 7.9 Hz, 1H), 4.63 (dd, J = 8.1, 6.2 Hz, 1H), 4.55 (dd, J = 7.9, 6.3 Hz, 1H), 4.36 (dt, J = 18.3, 6.4 Hz, 2H), 3.45 (dd, J = 9.9, 6.7 Hz, 1H), 2.98 (dddd, J = 9.8, 8.0, 6.6, 1.4 Hz, 1H), 2.21 (td, J = 8.0, 4.2 Hz, 1H), 1.29 (ddd, J = 11.1, 8.0, 3.6 Hz, 1H), 1.10 (d, J = 6.7 Hz, 3H), 0.52 (ddd, J = 10.4, 8.1, 6.2 Hz, 1H), 0.28 (dt, J = 6.2, 3.9 Hz, 1H). MS (ESI) m/z (M+ H-18) + = 321.3.

**Compound 19-d was synthesized using 19J-d as starting material, and the preparation process was the same as that of 19-a to obtain 7 mg of 19-d.**

**¹H NMR (400 MHz, Methanol-d4)** δ 7.24 (d, J = 7.9 Hz, 1H), 7.05 (d, J = 7.9 Hz, 1H), 4.63 (dd, J = 8.1, 6.2 Hz, 1H), 4.55 (dd, J = 7.9, 6.3 Hz, 1H), 4.36 (dt, J = 17.6, 6.5 Hz, 2H), 3.45 (dd, J = 9.9, 6.7 Hz, 1H), 3.01 - 2.91 (m, 1H), 2.18 (td, J = 8.0, 4.0 Hz, 1H), 1.26 (ddd, J = 11.2, 7.9, 3.5 Hz, 1H), 1.10 (d, J = 6.7 Hz, 3H), 0.50 (ddd, J = 10.4, 8.2, 6.2 Hz, 1H), 0.28 (dt, J = 6.2, 3.8 Hz, 1H). MS (ESI) m/z (M+ H-18) + = 321.3.

### Example 20: Synthesis of compound 20

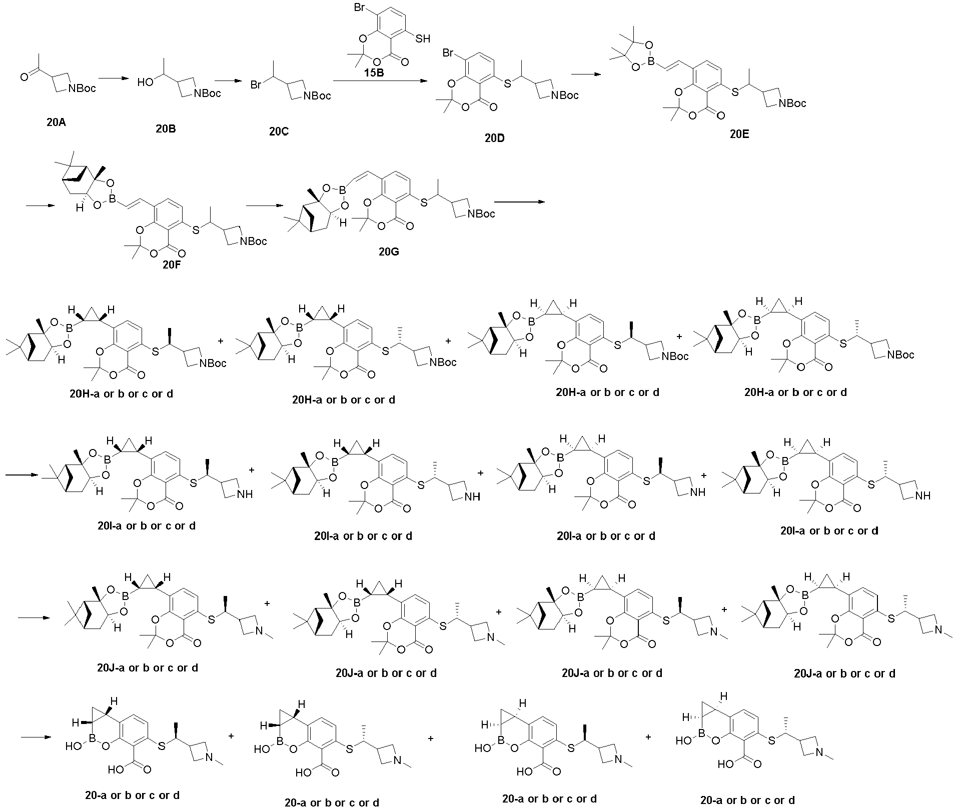

### Step 1: Preparation of compound 20B

At 0°C, sodium borohydride (782 mg, 21 mmol) was added to a solution of compound **20A** (4 g, 20 mmol) in methanol (500 mL), and the reaction mixture was stirred for 2 hours. After the reaction was completed, the reaction mixture was concentrated under reduced pressure to remove methanol, added with potassium hydroxide solution, and extracted with ethyl acetate (100 mL * 3). The combined organic phases were washed with saturated brine, dried with anhydrous sodium sulfate, filtered, and concentrated under reduced pressure to obtain a crude product, which was purified by column chromatography (petroleum ether/ethyl acetate = 4:1) to obtain compound **20B** (3.7 g, yield: 91.57%).

### Step 2: Preparation of compound 20C

At 0°C, carbon tetrabromide (7.3 g, 22.1 mmol) was added to a solution of compound **20B** (3.7 g, 18.4 mmol) and triphenylphosphine (5.8 g, 22.1 mmol)in dichloromethane (50 mL) in batches, and then the reaction mixture was slowly warmed to room temperature and stirred for 16 hours. After the reaction was completed, the reaction mixture was concentrated under reduced pressure to obtain a crude product, which was purified by column chromatography (petroleum ether/ethyl acetate = 5:1) to obtain **20C** (3.3g, yield: 67.95%).

### Step 3: Preparation of compound 20D

Potassium carbonate (5.2 g, 37.5 mmol) was added to a solution of compounds **20C** (3.3 g, 12.5 mmol) and **15B** (3.6 g, 12.5 mmol) in *N,N-*dimethylformamide (50 mL) , and the system was replaced with nitrogen, heated to 60°C, and stirred for 6 hours. After the reaction was completed, the reaction mixture was diluted with ethyl acetate, filtered and concentrated under reduced pressure to obtain a crude product, which was purified by column chromatography (petroleum ether/ethyl acetate = 2:1) to obtain compound **20D** (3.9 g, yield: 66.09%). MS (ESI) m/z (M- H) ⁺ = 472.0.

### Step 4: Preparation of compound 20E

The acetonitrile (9 mL) solution in which compound **20D** (870 mg, 1.8 mmol), N,N-diisopropylethylamine (250 mg, 1.9 mmol), tri-tert-butylphosphine tetrafluoroborate (213 mg, 0.7 mmol), tri-(dibenzylideneacetone) palladium (337 mg, 0.4 mmol) and pinacol vinylboronate (1.42 g, 9.2 mmol) were dissolved was stirred at 90°C for 1 h under nitrogen atmosphere. After the reaction was completed, the crude product (1 g) of compound **20E** was obtained by diluting with ethyl acetate, filtering and concentrating under reduced pressure, which was directly used in the next step without purification. MS (ESI) m/z (M- H) ⁺ = 546.2.

### Step 5: Preparation of compound 20F

A solution of compound **20E** (1.5 g, 2.8 mmol) and (1S,2S,3R,5S)-(+)-2,3-pinanediol (2.3 g, 13.8 mmol) in tetrahydrofuran (20 mL) was stirred at room temperature for 4 hours. After the reaction was completed, the reaction mixture was concentrated under reduced pressure to obtain the crude compound **20F,** which was purified by C18 reverse column (MeCN: 0.2% NH₄HCO₃ = 5% to 95%) to obtain a crude product, which was then purified by forward column chromatography (petroleum ether/ethyl acetate = 4:1) to obtain compound **20F** (1 g, yield: 60.86%). MS (ESI) m/z (M- H) ⁺ = 598.2.

### Step 6: Preparation of compound 20G

Compound **20F** (200 mg, 0.33 mmol) and tris(2-phenylpyridine)iridium (11 mg, 0.02 mmol) were dissolved in acetonitrile (6 mL), the system was replaced with nitrogen and stirred at room temperature for 1 hour under the irradiation of 10 W blue light. After the reaction was completed, the reaction mixture was concentrated under reduced pressure to obtain a crude product, which was purified by column chromatography (petroleum ether/ethyl acetate = 4:1) to obtain compound **20G** (105 mg, yield: 52.50%). MS (ESI) m/z (M- H) ⁺ = 598.2

### Step 7: Preparation of compounds 20H-a, 8b, 8c and 8d

Compound **20G** (645 mg, 1.1 mmol) and palladium acetate (24 mg, 0.1 mmol) were dissolved in tetrahydrofuran (15 mL), and the system was replaced with nitrogen, then an ether solution (7.2 mL, 21.6 mmol) of diazomethane was added thereto at 0 °C. The reaction mixture was stirred at room temperature for 1 hour. After the reaction was completed, the reaction mixture was diluted with dichloromethane and filtered to remove the solid. The filtrate was concentrated under reduced pressure to obtain a crude product, which was purified by column chromatography (petroleum ether/ethyl acetate = 4.5:1) to obtain a crude product (550 mg, yield: 83.32%), which was then subjected to chiral separation to obtain 94 mg of **20H-a;** 50 mg of **20H-b;** 54 mg of **20H-c;** 112 mg of **20H-d;** MS (ESI) m/z (M- H) ⁺ = 612.2. Retention time: **20H-a:** t = 10.620 min; **20H-b:** t = 9.075 min; **20H-c:** t = 10.741 min; **20H-d:** t = 14.542 min.

Chiral resolution conditions of **20H-a** and **20H-d:** chromatographic column: Daicel CHIRALPAK^{®} IE 250 * 30 mm, 10 µm; Mobile phase A: n-hexane (0.2% DEA); Mobile phase B: isopropanol (0.2% DEA); Detection wavelength: 254 nm; Flow rate: 2 5mL/min; Isocratic elution procedure: mobile phase A: mobile phase B = 85:15 (V/V).

Chiral analysis conditions: chromatographic column: Daicel CHIRALPAK^{®} IE 250 * 4.6 mm, 5 µm; Mobile phase A: n-hexane (0.2% DEA); Mobile phase B: isopropanol (0.2% DEA); Detection wavelength: 254 nm; Flow rate: 1 mL/min; Isocratic elution procedure: mobile phase A: mobile phase B = 85:15 (V/V).

Chiral resolution conditions of **20H-b** and **20H-c:** chromatographic column: Daicel CHIRALPAK IC 250 * 30 mm, 10 µm; Mobile phase A: n-hexane; Mobile phase B: isopropanol; Detection wavelength: 254 nm; Flow rate: 25 mL/min; Isocratic elution procedure: mobile phase A: mobile phase B = 60:40(V/V).

Chiral analysis conditions: chromatographic column: Daicel CHIRALPAK^{®} IE 250 * 4.6 mm, 5 µm; Mobile phase A: n-hexane (0.2% DEA); Mobile phase B: isopropanol (0.2% DEA); Detection wavelength: 254 nm; Flow rate: 1 mL/min; Isocratic elution procedure: mobile phase A: mobile phase B = 60:40 (V/V).

### Step 8: Preparation of compounds 20I-a, b, c and d

At 0°C, 2,6-dimethylpyridine (42 mg, 0.39 mmol) and trimethylsilyl trifluoromethanesulfonate (106 mg, 0.59 mmol) were added to a solution of compound **20H-a** (60 mg, 0.1 mmol) in dichloromethane (3 mL), and the reaction mixture was stirred for 2 hours. After the reaction was completed, the reaction mixture was concentrated under reduced pressure to obtain a crude product, which was purified by column chromatography (DCM: MeOH (0.1% NH₃) = 9:1) to obtain compound **20I-a** (50 mg, yield: 99.64%).

### Step 9: Preparation of compounds 20I-a, b, c and d

An aqueous formaldehyde solution (0.3 mL) was added to a solution of compound **20I-a** (50 mg, 0.1 mmol) in dichloromethane (1 mL) at room temperature. After stirring for 1 hour at room temperature, sodium triacetylborohydride (41 mg, 0.2 mmol) was added to the above reaction mixture and stirred for another 1 hour. After the reaction was completed, the reaction mixture was quenched with saturated sodium bicarbonate solution and extracted with ethyl acetate (10 mL × 3), and the organic phases were combined, washed with saturated brine, dried with anhydrous sodium sulfate, filtered, and concentrated under reduced pressure to obtain a crude product, which was purified by column chromatography (DCM: MeOH(0.1% NH₃) = 9:1) to obtain compound **20I-a** (33 mg, yield: 64.24%). MS (ESI) m/z (M- H) ⁺ = 526.0.

### Step 10: Preparation of compounds 20-a, b, c and d

Water (0.1 mL) and 3N sodium hydroxide (188 uL, 0.57 mmol) solution were sequentially added to a solution of compound **20I-a** (33 mg, 0.06 mmol) in acetonitrile (1 mL), and the reaction mixture was stirred for 4 hours at 60 °C. TFA (0.5 mL), triethylsilane (0.1 mL) and isobutylboronic acid (19 mg, 0.19 mmol) were added to the reaction system in sequence, and stirred for another 1 hour. After the reaction was completed, the pH was adjusted to about 6, and the reaction mixture was concentrated under reduced pressure to obtain a crude product, which was separated by HPLC to prepare the crude compound (12 mg, yield: 72. 6%). 0.5 mL of water was added thereto, followed by 3M sodium hydroxide (19.8 µL, 0.059 mmol), and the reaction mixture was stirred for 1 hour at 60 °C. The reaction mixture was purified by C18 reverse phase column chromatography (separation conditions: chromatographic column: Redi-GOLD ^{®} C18 10 µm 33 * 147 mm; Mobile phase: [water-acetonitrile]; Flow rate: 30 mL/min) to obtain compound **20-a** (6.59 mg, yield: 26.6%). Retention time: t = 1.798 min. **¹H NMR (400 MHz, Deuterium Oxide)** δ 7.10 (d, J = 7.6 Hz, 1H), 6.90 (d, J = 7.8 Hz, 1H), 4.25 - 4.10 (m, 1H), 4.06 - 3.96 (m, 1H), 3.84 - 3.77 (m, 1H), 3.73 - 3.62 (m, 1H), 3.34 (dt, J = 21.6, 7.5 Hz, 1H), 2.97 - 2.84 (m, 1H), 2.74 (d, J = 25.9 Hz, 3H), 1.96 (s, 1H), 1.07 (dd, J = 15.2, 6.8 Hz, 4H), 0.31 (d, J = 26.1 Hz, 2H). MS (ESI) m/z (M- H) ⁺ = 334.2.

**Similar to the synthesis of Example 20-a, Examples 20-b to 34-b were synthesized as shown in Table 1 below:**

**Table 1**

| | |
|---|---|
| **Example 20-b** | 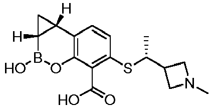 |
| | ¹H NMR (400 MHz, Deuterium Oxide) δ 7.07 (d, J = 7.7 Hz, 1H), 6.80 (d, J = 8.0 Hz, 1H), 3.69 - 3.61 (m, 2H), 3.37 - 3.25 (m, 2H), 2.66 (q, J = 8.3 Hz, 1H), 2.45 (s, 3H), 1.79 (q, J = 7.3 Hz,1H), 1.11 (d, J = 6.6 Hz, 3H), 0.84 (t, J = 8.5 Hz, 1H), 0.33 (m, 1H), 0.25 (m, 1H). |
| | MS (ESI) m/z (M+1)+=334.2. Retention time: t = 3.120 min |
| **Example 20-c** | 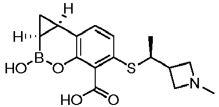 |
| | ¹H NMR (400 MHz, Deuterium Oxide) δ 7.08 (d, J = 7.8 Hz, 1H), 6.81 (d, J = 7.8 Hz, 1H), 3.82 - 3.63 (m, 2H), 3.35 (m, 2H), 2.73 (q, J = 8.5 Hz, 1H), 2.52 (s, 3H), 1.79 ((td, J = 8.3, 3.7 Hz, 1H), 1.12 (d, J = 6.8 Hz, 3H), 0.84 (m, 1H), 0.34 (m, 1H), 0.30 - 0.19 (m, 1H). |
| | MS (ESI) m/z (M+1)+=334.2. Retention time: t = 3.031 min |
| **Example 20-d** | 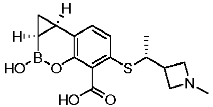 |
| | **¹H NMR (400 MHz, Methanol-d4)** δ 7.12 (d, J = 7.8 Hz, 1H), 6.97 (d, J = 11.2 Hz, 1H), 3.81-3.74 (m, 2H), 3.52-3.43 (m, 2H), 2.91-2.84 (m, 1H), 2.76 (s, 3H), 2.05-1.99 (m, 1H), 1.14 (d, J = 6.9 Hz, 3H), 0.81 (d, J = 6.6 Hz, 1H), 0.42 (q, J = 8.3 Hz, 1H), 0.30 (dt, J = 6.6, 3.6 Hz, 1H). |
| | MS (ESI) m/z (M+1)+=334.4. Retention time: t = 2.936 min |
| **Example 21-a** | 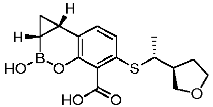 |
| | ¹H NMR (400 MHz, Methanol-*d*₄) δ 7.24 (d, *J* = 8.0 Hz, 1H), 7.05 (d, *J* = 8.0 Hz, 1H), 3.77 (td, *J* = 8.3, 4.4 Hz, 2H), 3.62 (q, *J=* 8.0 Hz, 1H), 3.52 - 3.46 (m, 1H), 3.17 - 3.10 (m, 1H), 2.31 - 2.15 (m, 2H), 2.01 (dtd, *J* = 12.3, 7.6, 4.3 Hz, 1H), 1.76 - 1.67 (m, 1H), 1.28 (td, *J* = 7.8, 4.0 Hz, 1H), 1.11 (d, *J* = 6.7 Hz, 3H), 0.50 (ddd, *J* = 10.4, 8.1, 6.1 Hz, 1H), 0.28 (dt, *J* = 6.2, 3.9 Hz, 1H). |
| | MS (ESI) m/z (M-1)-=333.0. Retention time: t = 2.934 min |
| **Example 21-b** | 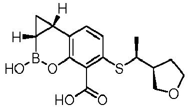 |
| | ¹H NMR (400 MHz, Methanol-*d*₄) δ 7.24 (d, *J* = 8.0 Hz, 1H), 7.05 (d, *J* = 7.9 Hz, 1H), 3.84 (dd, *J* = 8.7, 7.7 Hz, 1H), 3.78 (td, *J* = 8.4, 3.9 Hz, 1H), 3.63 (td, *J* = 8.4, 6.9 Hz, 1H), 3.47 (dd, *J* = 8.7, 7.8 Hz, 1H), 3.08 (dq, *J* = 8.9, 6.7 Hz, 1H), 2.30 - 2.12 (m, 2H), 1.98 (dddd, *J* = 12.5, 8.5, 7.1, 4.1 Hz, 1H), 1.63 (dq, *J* = 12.3, 8.4 Hz, 1H), 1.28 (ddd, *J* = 10.4, 8.0, 3.6 Hz, 1H), 1.16 (d, *J* = 6.7 Hz, 3H), 0.50 (ddd, *J* = 10.4, 8.1, 6.2 Hz, 1H), 0.28 (dt, *J* = 6.2, 3.9 Hz, 1H). |
| | MS (ESI) m/z (M-1)-=333.0. Retention time: t = 2.903 min |
| **Example 21-c** | 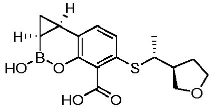 |
| | ¹H NMR (400 MHz, Methanol-d4) δ 7.24 (d, J = 8.0 Hz, 1H), 7.05 (d, J = 8.0 Hz, 1H), 3.77 (td, J = 8.4, 4.4 Hz, 2H), 3.62 (td, J = 8.4, 7.2 Hz, 1H), 3.52 - 3.39 (m, 1H), 3.13 - 2.98 (m, 1H), 2.33 - 2.15 (m, 2H), 2.06 - 1.86 (m, 2H), 1.71 - 1.55 (m, 1H), 1.16 (d, J = 6.4 Hz, 3H), 0.59 - 0.38 (m, 1H), 0.30 - 0.26 (m, 1H). MS (ESI) m/z (M-1)- = 333.0. Retention time: t = 2.832 min |
| **Example 21-d** | 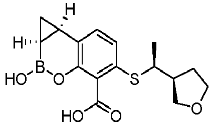 |
| | ¹H NMR (400 MHz, Methanol-*d*₄) δ 7.24 (d, *J* = 8.0 Hz, 1H), 7.05 (d, *J* = 8.0 Hz, 1H), 3.77 (td, *J* = 8.4, 4.4 Hz, 2H), 3.62 (td, *J* = 8.4, 7.2 Hz, 1H), 3.49 (dd, *J* = 8.4, 7.2 Hz, 1H), 3.19 - 3.08 (m, 1H), 2.36 - 2.15 (m, 2H), 2.07 - 1.93 (m, 1H), 1.75 -1.65 (m, 1H), 1.34 - 1.24 (m, 1H), 1.16 (d, *J* = 6.4 Hz, 3H), 0.54 - 0.48 (m, 1H), 0.30 - 0.26 (m, 1H). |
| | MS (ESI) m/z (M-1)⁻=333.0. Retention time: t = 2.355 min |
| **Example 22-a** | 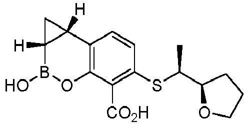 |
| | ¹H NMR (400 MHz, DMSO-d6) δ 12.97 (s, 1H), 9.33 (s, 1H), 7.33 (d, J = 8.0 Hz, 1H), 7.11 (d, J = 7.9 Hz, 1H), 3.82 - 3.69 (m, 2H), 3.63 (td, J = 7.8, 6.1 Hz, 1H), 3.25 (d, J = 6.7 Hz, 1H), 2.28 (td, J = 7.9, 4.0 Hz, 1H), 1.99 - 1.88 (m, 1H), 1.83 - 1.73 (m, 2H), 1.67 - 1.57 (m, 1H), 1.34 (td, J = 7.7, 4.0 Hz, 1H), 1.15 (d, J = 6.8 Hz, 3H), 0.53 (ddd, J = 10.5, 8.0, 6.0 Hz, 1H), 0.31 (dt, J = 6.0, 3.7 Hz, 1H). **MS (ESI)** m/z (M+ H-18) ⁺ = 317.0. Retention time: t = 2.196 min; Retention time of precursor before deprotection: t = 25.930 min |
| **Example 22-b** | 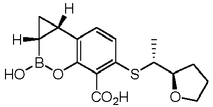 |
| | ¹H NMR (400 MHz, DMSO-d6) δ 12.97 (s, 1H), 9.33 (s, 1H), 7.33 (d, J = 8.0 Hz, 1H), 7.10 (d, J = 8.0 Hz, 1H), 3.85 - 3.71 (m, 2H), 3.61 (dt, J = 8.0, 6.6 Hz, 1H), 2.28 (q, J = 3.9 Hz, 1H), 1.89 (dd, J = 11.8, 7.2 Hz, 1H), 1.84 - 1.76 (m, 2H), 1.75 - 1.66 (m, 1H), 1.33 (ddd, J = 10.9, 7.9, 3.4 Hz, 1H), 1.14 (d, J = 6.9 Hz, 3H), 0.53 (ddd, J = 10.4, 8.0, 6.1 Hz, 1H), 0.32 (dt, J = 6.1, 3.7 Hz, 1H). **MS (ESI)** m/z (M+ H-18) ⁺ = 317.0. Retention time: t = 2.187 min; Retention time of precursor before deprotection: t = 29.544 min |
| **Example 22-c** | 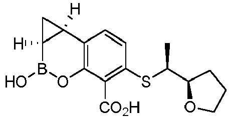 |
| | ¹H NMR (400 MHz, Methanol-d4) δ 7.33 (d, J = 8.0 Hz, 1H), 7.17 (d, J = 8.0 Hz, 1H), 3.93 (td, J = 7.1, 5.0 Hz, 1H), 3.85 (dt, J = 8.4, 6.5 Hz, 1H), 3.73 (ddd, J = 8.2, 7.1, 6.0 Hz, 1H), 3.39 - 3.33 (m, 1H), 2.29 (td, J = 8.0, 4.1 Hz, 1H), 2.01 (dd, J = 9.4, 2.0 Hz, 1H), 1.94 - 1.81 (m, 3H), 1.37 (ddd, J = 10.4, 7.9, 3.5 Hz, 1H), 1.23 (d, J = 6.9 Hz, 3H), 0.60 (ddd, J = 10.4, 8.1, 6.2 Hz, 1H), 0.37 (dt, J = 6.2, 3.9 Hz, 1H). **MS (ESI)** m/z (M+ H-18) ⁺ = 317.0. Retention time: t = 2.506 min; Retention time of precursor before deprotection: t = 5.123 min |
| **Example 22-d** | 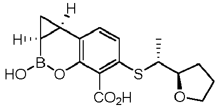 |
| | ¹H NMR (400 MHz, DMSO-d6) δ 12.97 (s, 1H), 9.33 (s, 1H), 7.33 (d, J = 8.0 Hz, 1H), 7.11 (d, J = 7.9 Hz, 1H), 3.80 - 3.69 (m, 2H), 3.66 - 3.60 (m, 1H), 3.25 (t, J = 6.7 Hz, 1H), 2.28 (d, J = 4.0 Hz, 1H), 1.98 - 1.90 (m, 1H), 1.85 - 1.75 (m, 2H), 1.60 (dd, J = 11.9, 8.3 Hz, 1H), 1.33 (s, 1H), 1.15 (d, J = 6.8 Hz, 3H), 0.53 (ddd, J = 10.3, 8.0, 6.0 Hz, 1H), 0.35 - 0.29 (m, 1H). **MS (ESI) m/z** (M+ H-18)⁺ = 317.0. Retention time: t = 2.506 min; |
| | Retention time of precursor before deprotection: t = 5.650 min |
| **Example 23-a** | 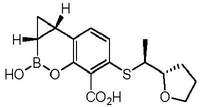 |
| | **¹H NMR** (400 MHz, DMSO-d6) δ 12.97 (s, 1H), 9.33 (s, 1H), 7.33 (d, J = 8.0 Hz, 1H), 7.10 (d, J = 7.9 Hz, 1H), 3.84 - 3.70 (m, 2H), 3.61 (dt, J = 8.0, 6.6 Hz, 1H), 3.32 - 3.29 (m, 1H), 2.28 (td, J = 8.0, 4.0 Hz, 1H), 1.96 - 1.86 (m, 1H), 1.86 - 1.77 (m, 2H), 1.73 - 1.60 (m, 1H), 1.33 (ddd, J = 10.9, 7.9, 3.3 Hz, 1H), 1.14 (d, J = 6.9 Hz, 3H), 0.53 (ddd, J = 10.4, 8.0, 6.0 Hz, 1H), 0.32 (dt, J = 6.1, 3.7 Hz, 1H). **MS (ESI)** m/z (M+ H-18) ⁺ = 317.0. |
| | Retention time: t = 3.334 min |
| **Example 23-b** | 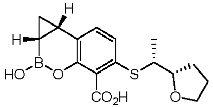 |
| | **¹H NMR** (400 MHz, DMSO-d6) δ 12.96 (s, 1H), 9.33 (s, 1H), 7.33 (d, J = 7.9 Hz, 1H), 7.11 (d, J = 7.9 Hz, 1H), 3.74 (dq, J = 14.2, 6.9 Hz, 2H), 3.63 (td, J = 7.6, 5.9 Hz, 1H), 3.24 (p, J = 6.7 Hz, 2H), 2.28 (td, J = 8.0, 4.0 Hz, 1H), 1.93 (dtd, J = 11.9, 7.1, 4.9 Hz, 1H), 1.85 - 1.74 (m, 2H), 1.66 - 1.56 (m, 1H), 1.33 (ddd, J = 11.0, 7.9, 3.4 Hz, 1H), 1.15 (d, J = 6.8 Hz, 3H), 0.54 (ddd, J = 10.5, 8.1, 6.1 Hz, 1H), 0.31 (dt, J = 6.4, 3.7 Hz, 1H). |
| | **MS (ESI)** m/z (M+ H-18)⁺ = 317.0. Retention time: t = 3.306 min |
| **Example 23-c** | 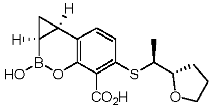 |
| | **¹H NMR** (400 MHz, DMSO-d6) δ 12.97 (s, 1H), 9.33 (s, 1H), 7.33 (d, J = 8.0 Hz, 1H), 7.11 (d, J = 8.0 Hz, 1H), 3.80 - 3.69 (m, 2H), 3.63 (td, J = 7.7, 5.9 Hz, 1H), 3.25 (t, J = 6.7 Hz, 1H), 2.28 (td, J = 7.9, 4.0 Hz, 1H), 2.00 - 1.85 (m, 1H), 1.86 - 1.72 (m, 3H), 1.65 - 1.55 (m, 1H), 1.33 (ddd, J = 10.9, 7.9, 3.4 Hz, 1H), 1.15 (d, J = 6.8 Hz, 3H), 0.53 (ddd, J = 10.5, 8.0, 6.1 Hz, 1H), 0.32 (dt, J = 6.0, 3.7 Hz, 1H). |
| | **MS (ESI)** m/z (M+ H-18)⁺ = 317.0. Retention time: t = 3.305 min |
| **Example 23-d** | 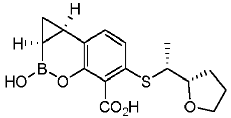 |
| | **¹H NMR** (400 MHz, DMSO-d6) δ 12.97 (s, 1H), 9.33 (s, 1H), 7.33 (d, J = 8.0 Hz, 1H), 7.10 (d, J = 8.0 Hz, 1H), 3.88 - 3.69 (m, 2H), 3.61 (dt, J = 8.0, 6.6 Hz, 1H), 3.33 (s, 1H), 2.28 (td, J = 7.9, 4.0 Hz, 1H), 1.97 - 1.87 (m, 1H), 1.81 (dtt, J = 10.1, 7.3, 4.9 Hz, 2H), 1.73 - 1.62 (m, 1H), 1.33 (ddd, J = 11.0, 7.9, 3.4 Hz, 1H), 1.13 (d, J = 6.9 Hz, 3H), 0.53 (ddd, J = 10.5, 8.1, 6.1 Hz, 1H), 0.31 (dt, J = 6.0, 3.7 Hz, 1H). |
| | **MS (ESI)** m/z (M+ H-18)⁺ = 317.0. Retention time: t = 2.284 min |
| **Example 24-a** | 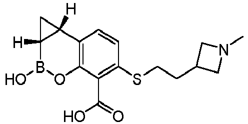 |
| | **¹H NMR (400 MHz, Deuterium Oxide)** δ 7.12 (d, J = 7.9 Hz, 1H), 6.81 (d, J = 7.8 Hz, 1H), 4.23 (t, J = 10.8 Hz, 1H), 4.05-3.88 (m, 2H), 3.70 (t, J = 9.8 Hz, 1H), 3.02 (t, J = 9.2 Hz, 2H), 2.89-2.74 (m, 5H), 1.94-1.75 (m, 3H), 0.91 (t, J = 7.9 Hz, 1H) 0.37-0.24 (m, 2H). |
| | **MS (ESI)** m/z (M+ H) ⁺ = 334.2. Retention time: t = 8.916 min; Retention time of precursor before deprotection: t = 2.990 min |
| **Example 24-b** | 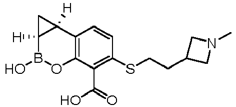 |
| | **¹H NMR (400 MHz, Deuterium Oxide)** δ 7.04 (d, J = 7.9 Hz, 1H), 6.75 (d, J = 7.8 Hz, 1H), 4.17 (q, J = 8.7 Hz, 1H), 3.97 - 3.82 (m, 2H), 3.63 (t, J = 9.7 Hz, 1H), 2.97 (p, J = 8.1 Hz, 1H), 2.82 - 2.69 (m, 5H), 1.88 - 1.71 (m, 3H), 0.89 (dd, J = 9.2, 5.0 Hz, 1H), 0.31 - 0.16 (m, 2H). |
| | **MS (ESI)** m/z (M+ H) ⁺ = 334.2. Retention time: t = 7.725 min; Retention time of precursor before deprotection: t = 2.223 min |
| **Example 25-a** | 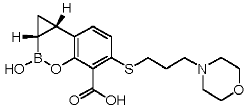 |
| | **¹H NMR (400 MHz, Deuterium Oxide)** δ 7.08 (d, J = 7.8 Hz, 1H), 6.77 (d, J = 7.8 Hz, 1H), 3.72 (s, 4H), 2.81 (t, J = 7.0 Hz, 2H), 2.64 - 2.47 (m, 6H), 1.77 (m, 3H), 0.82 (t, J = 8.5 Hz, 1H), 0.31 (dd, J = 5.7, 2.9 Hz, 1H), 0.27 - 0.20 (m, 1H). |
| | **MS (ESI)** m/z (M+ H) ⁺ = 364.3. Retention time: t = 8.077 min; Retention time of precursor before deprotection: t = 2.849 min |
| **Example 25-b** | 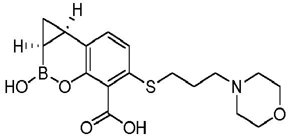 |
| | **¹H NMR (400 MHz, Deuterium Oxide)** δ 7.07 (d, J = 7.8 Hz, 1H), 6.76 (d, J = 7.8 Hz, 1H), 3.69 (t, J = 4.8 Hz, 4H), 2.80 (t, J = 7.2 Hz, 2H), 2.45 (dd, J = 15.2, 7.4 Hz, 6H), 1.82 - 1.66 (m, 3H), 0.87 - 0.77 (m, 1H), 0.31 (dt, J = 6.4, 3.1 Hz, 1H), 0.23 (td, J = 9.1, 6.7 Hz, 1H). |
| | **MS (ESI)** m/z (M+ H) ⁺ = 364.3. Retention time: t = 9.601 min; Retention time of precursor before deprotection: t = 2.849 min |
| **Example 26-a** | 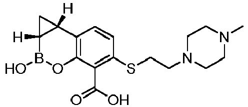 |
| | **¹H NMR (400 MHz, Deuterium Oxide)** δ 7.13 (d, J = 7.8 Hz, 1H), 6.85 (d, J = 7.8 Hz, 1H), 2.98 (d, J = 57.3 Hz, 12H), 2.49 (s, 3H), 1.81 (td, J = 8.3, 3.6 Hz, 1H), 0.85 (ddd, J = 10.2, 8.0, 2.6 Hz, 1H), 0.34 - 0.23 (m, 2H). |
| | **MS (ESI)** *m*/*z* (M+ H)⁺= 363.4. Retention time: t = 1.402 min |
| **Example 26-b** | 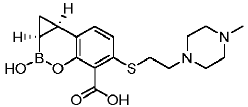 |
| | **¹H NMR (400 MHz, Deuterium Oxide)** δ 7.12 (d, J = 7.8 Hz, 1H), 6.85 (d, J = 7.8 Hz, 1H), 3.10 - 2.64 (m, 12H), 2.48 (s, 3H), 1.80 (dt, J = 8.2, 4.1 Hz, 1H), 0.88 - 0.82 (m, 1H), 0.34 - 0.24 (m, 2H). **MS (ESI)** *m*/*z* (M+ H)⁺= 363.4. Retention time: t = 1.425 min |
| **Example 27-a** | 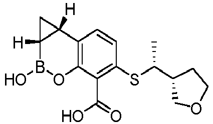 |
| | **¹H NMR (400 MHz, Methanol-d4)** δ7.11 (d, J = 7.9 Hz, 1H), 6.82 (d, J = 7.9 Hz, 1H), 3.97 (dd, J = 8.7, 7.7 Hz, 1H), 3.88 (td, J = 8.3, 3.9 Hz, 1H), 3.73 (td, J = 8.4, 7.0 Hz, 1H), 3.59 (t, J = 8.4 Hz, 1H), 3.24 (dd, J = 8.7, 6.7 Hz, 1H), 2.36 (q, J = 8.2 Hz, 1H), 2.07 (ddd, J = 12.1, 8.4, 4.3 Hz, 1H), 1.93-1.84 (m, 1H), 1.82-1.71 (m, 1H), 1.29 (d, J = 6.6 Hz, 3H), 0.95 (t, J = 8.2 Hz, 1H), 0.45-0.34 (m, 2H). **MS (ESI)** *m*/*z* (M-18)⁺= 317.4. Retention time: t = 2.467 min |
| **Example 27-b** | 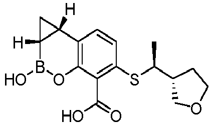 |
| | **¹H NMR (400 MHz, Methanol-d4)** δ7.34 (d, J = 8.0 Hz, 1H), 7.15 (d, J = 7.9 Hz, 1H), 3.92-3.83 (m, 2H), 3.78-3.67 (m, 1H), 3.59 (dd, J = 8.6, 7.5 Hz, 1H), 3.24 (dd, J = 7.8, 6.7 Hz, 1H), 2.37 (q, J = 7.8 Hz, 1H), 2.29 (td, J = 8.1, 4.2 Hz, 1H), 2.16-2.06 (m, 1H), 1.80 (dd, J = 12.5, 8.2 Hz, 1H), 1.38 (ddd, J = 10.3, 8.0, 3.6 Hz, 1H), 1.21 (d, J = 6.7 Hz, 3H), 0.60 (ddd, J = 10.5, 8.2, 6.2 Hz, 1H), 0.38 (dt, J = 6.3, 3.9 Hz, 1H). **MS (ESI)** *m*/*z* (M-18)⁺= 317.2. Retention time: t = 2.052 min |
| **Example 27-c** | 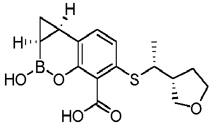 |
| | **¹H NMR (400 MHz, Methanol-d4)** δ 7.33 (d, J = 7.9 Hz, 1H), 7.13 (d, J = 7.9 Hz, 1H), 3.90 - 3.84 (m, 2H), 3.72 (d, J = 7.4 Hz, 1H), 3.58 (dd, J = 8.6, 7.4 Hz, 1H), 3.25 - 3.22 (m, 1H), 2.36 (q, J = 7.8 Hz, 1H), 2.27 (d, J = 4.1 Hz, 1H), 2.11 (d, J = 7.6 Hz, 1H), 1.81 (dd, J = 12.5, 8.1 Hz, 1H), 1.38 - 1.32 (m, 1H), 1.21 (d, J = 6.7 Hz, 3H), 0.59 (ddd, J = 10.4, 8.2, 6.2 Hz, 1H), 0.38 (dt, J = 6.3, 3.8 Hz, 1H). **MS (ESI)** *m*/*z* (M-18)⁺= 317.2. |
| | Retention time: t = 2.053 min |
| **Example 27-d** | 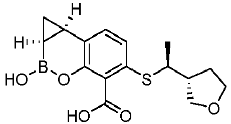 |
| | **¹H NMR (400 MHz, Methanol-d4)** δ 7.14 (d, J = 7.9 Hz, 1H), 6.83 (d, J = 7.9 Hz, 1H), 3.98 (dd, J = 8.7, 7.7 Hz, 1H), 3.88 (td, J = 8.3, 3.9 Hz, 1H), 3.73 (td, J = 8.4, 6.9 Hz, 1H), 3.59 (t, J = 8.3 Hz, 1H), 3.25 (dd, J = 8.8, 6.6 Hz, 1H), 2.35 (q, J = 8.2 Hz, 1H), 2.14 - 2.03 (m, 1H), 1.90 (d, J = 4.1 Hz, 1H), 1.77 (dd, J = 12.3, 8.5 Hz, 1H), 1.28 (d, J = 6.6 Hz, 3H), 0.97 (t, J = 8.7 Hz, 1H), 0.47 - 0.33 (m, 2H). **MS (ESI)** *m*/*z* (M-18)⁺= 317.2. |
| | Retention time: t = 2.560 min |
| **Example 28-a** | 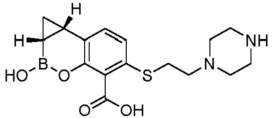 |
| | **¹H NMR (400 MHz, Methanol-d4)** δ 7.14 (d, J = 8.0 Hz, 1H), 6.85 (d, J = 8.1 Hz, 1H), 3.09 (s, 2H), 2.97 (t, J = 6.9 Hz, 3H), 2.70 (q, J = 6.4, 5.6 Hz, 6H), 1.96 (td, J = 8.2, 3.9 Hz, 1H), 1.09 - 0.99 (m, 1H), 0.45 - 0.25 (m, 2H). **MS (ESI)** *m*/*z* (M+ H)⁺= 349.4. Retention time: t = 2.147 min |
| **Example 28-b** | 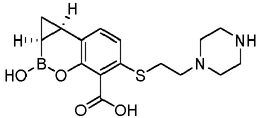 |
| | **¹H NMR (400 MHz, Deuterium Oxide)** δ 7.26 (d, J = 8.0 Hz, 1H), 7.11 (d, J = 7.9 Hz, 1H), 3.40 (d, J = 5.7 Hz, 4H), 3.28 (s, 4H), 3.19 (d, J = 5.7 Hz, 2H), 3.15 (d, J = 5.7 Hz, 2H), 2.16 (td, J= 8.2, 4.3 Hz, 1H), 1.27 - 1.21 (m, 1H), 0.52 - 0.45 (m, 1H), 0.26 (dt, J = 6.1, 3.8 Hz, 1H). |
| | **MS (ESI)** *m*/*z* (M+H)⁺=349.4. Retention time: t = 2.120 min |
| **Example 29-a** | 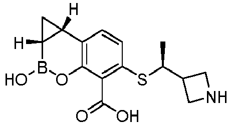 |
| | **¹H NMR (400 MHz, Methanol-d4)** δ 8.10 (s, 1H), 7.11 (d, J = 7.9 Hz, 1H), 6.94 (d, J = 8.0 Hz, 1H), 3.95 - 3.87 (m, 3H), 3.69 (dd, J = 11.0, 7.5 Hz, 1H), 3.43 (dd, J = 8.5, 6.6 Hz, 1H), 2.92 - 2.86 (m, 1H), 2.01 (td, J = 8.2, 4.0 Hz, 1H), 1.13 (d, J = 6.8 Hz, 3H), 1.08 (s, 1H), 0.43 - 0.37 (m, 1H), 0.28 (dt, J = 6.8, 3.7 Hz, 1H). **MS (ESI)** m/z (M+ H)⁺ = 320.2. Retention time: t = 3.001 min |
| **Example 29-b** | 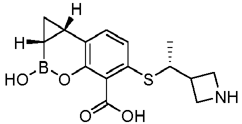 |
| | ¹H NMR (400 MHz, Methanol-*d*₄) δ 7.20 (d, *J* = 7.9 Hz, 1H), 7.03 (d, *J* = 7.9 Hz, 1H), 4.02 (dd, *J* = 11.3, 9.4 Hz, 3H), 3.78 (dd, *J* = 10.9, 7.5 Hz, 1H), 3.53 (dd, *J* = 8.4, 6.7 Hz, 1H), 2.99 (q, *J* = 8.2 Hz, 1H), 2.11 (dt, *J* = 8.0, 4.2 Hz, 1H), 1.23 (d, *J* = 6.8 Hz, 3H), 1.18 (d, *J* = 11.8 Hz, 1H), 0.55 - 0.48 (m, 1H), 0.38 (dt, *J* = 6.7, 3.7 Hz, 1H). **MS (ESI)** *m*/*z* (M+ H)⁺= 320.2. Retention time: t = 3.364 min |
| **Example 29-c** | 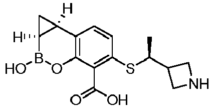 |
| | ¹H NMR (400 MHz, Methanol-*d*₄) δ 7.19 (d, *J* = 7.9 Hz, 1H), 7.04 (d, *J* = 7.9 Hz, 1H), 4.05 - 3.99 (m, 3H), 3.81 - 3.75 (m, 1H), 3.58 - 3.53 (m, 1H), 3.00 (q, *J* = 8.3 Hz, 1H), 2.12 (s, 1H), 1.23 (d, *J* = 6.8 Hz, 3H), 1.19 (s, 1H), 0.51 (q, *J* = 9.1, 8.6 Hz, 1H), 0.39 (dt, *J* = 6.6, 3.7 Hz, 1H). **MS (ESI)** *m*/*z* (M+ H)⁺= 320.2. Retention time: t = 3.288 min |
| **Example 29-d** | 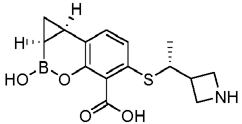 |
| | **¹H NMR (400 MHz, Methanol-d4)** δ 7.11 (d, J = 7.9 Hz, 1H), 6.93 (d, J = 7.8 Hz, 1H), 3.94 - 3.86 (m, 3H), 3.69 (dd, J = 10.8, 7.6 Hz, 1H), 3.47 - 3.42 (m, 1H), 2.94 - 2.88 (m, 1H), 2.01 (td, J = 8.0, 3.9 Hz, 1H), 1.13 (d, J = 6.9 Hz, 3H), 1.09 - 1.04 (m, 1H), 0.44 - 0.37 (m, 1H), 0.29 (dt, J = 6.7, 3.7 Hz, 1H). **MS (ESI)** *m*/*z* (M+ H)⁺= 320.2. Retention time: t = 2.922 min |
| **Example 30-a** | 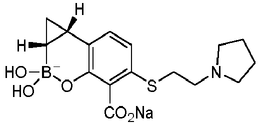 |
| | **¹H NMR (400 MHz, Deuterium Oxide)** δ 6.83 (d, J = 7.8 Hz, 1H), 6.52 (d, J = 7.9 Hz, 1H), 3.66 (ddd, J = 11.4, 4.3, 1.7 Hz, 2H), 3.18 (td, J = 11.8, 2.0 Hz, 2H), 2.64 - 2.56 (m, 2H), 1.54 (ddd, J = 9.1, 7.8, 3.6 Hz, 1H), 1.45 (ddt, J = 10.6, 6.8, 3.7 Hz, 1H), 1.38 (ddd, J = 13.2, 3.6, 1.6 Hz, 2H), 1.24 (q, J = 6.9 Hz, 2H), 0.95 (dtd, J = 13.5, 11.4, 4.5 Hz, 2H), 0.58 (ddd, J = 10.1, 7.9, 2.6 Hz, 1H), 0.11 - 0.05 (m, 1H), -0.01 (td, J = 9.2, 6.6 Hz, 1H). **MS (ESI)** m/z (M+ H)⁺ = 349.4. Retention time: t = 3.516 min |
| **Example 30-b** | 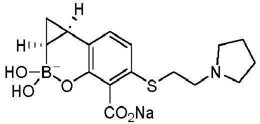 |
| | **¹H NMR (400 MHz, Deuterium Oxide)** δ 6.78 (dd, J = 7.8, 1.6 Hz, 1H), 6.49 (dd, J = 7.7, 1.5 Hz, 1H), 3.59 (dd, J = 11.6, 4.2 Hz, 2H), 3.11 (t, J = 11.7 Hz, 2H), 2.54 (t, J = 7.5 Hz, 2H), 1.54 (td, J = 8.1, 3.7 Hz, 1H), 1.42 - 1.26 (m, 3H), 1.17 (q, J = 7.2 Hz, 2H), 0.88 (qd, J = 12.3, 4.4 Hz, 2H), 0.58 (t, J = 8.3 Hz, 1H), 0.04 - -0.11 (m, 2H). **MS (ESI)** m/z (M+ H)⁺ = 349.4. Retention time: t = 3.510 min |
| **Example 31-a** | 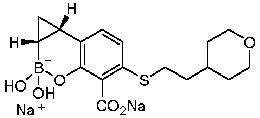 |
| | **¹H NMR (400 MHz, Deuterium Oxide)** δ 6.89 (d, J = 7.9 Hz, 1H), 6.61 (d, J = 7.8 Hz, 1H), 3.26 - 2.89 (m, 4H), 2.86 (dd, J = 6.4, 4.6 Hz, 2H), 2.69 (s, 2H), 1.68 (s, 4H), 1.57 - 1.48 (m, 1H), 0.58 (ddd, J = 10.2, 8.1, 2.5 Hz, 1H), 0.08 - 0.03 (m, 1H), 0.03 - -0.05 (m, 1H). |
| | **MS (ESI)** m/z (M+H)⁺ = 334.4. Retention time: t = 2.149 min |
| **Example 31-b** | 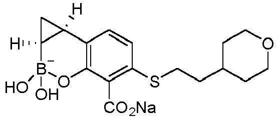 |
| | **¹H NMR (400 MHz, Deuterium Oxide)** δ 6.89 (d, J = 7.9 Hz, 1H), 6.61 (d, J = 7.8 Hz, 1H), 3.22 - 2.89 (m, 4H), 2.86 (dd, J = 6.4, 4.6 Hz, 2H), 2.69 (s, 2H), 1.68 (s, 4H), 1.57 - 1.48 (m, 1H), 0.58 (ddd, J = 10.2, 8.1, 2.5 Hz, 1H), 0.08 - 0.03 (m, 1H), 0.03 - -0.05 (m, 1H). |
| | **MS (ESI)** m/z (M+H)⁺=334.4. Retention time: t = 3.919 min |
| **Example 32-a** | 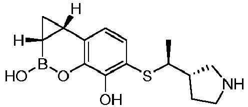 |
| | ¹H NMR (400 MHz, Methanol-*d*₄) δ 7.16 (d, *J* = 7.9 Hz, 1H), 6.97 (d, *J* = 7.9 Hz, 1H), 3.44 (dd, *J* = 12.0, 8.0 Hz, 1H), 3.33 (d, *J* = 4.5 Hz, 1H), 3.25 - 3.11 (m, 3H), 2.39 (q, *J* = 8.4 Hz, 1H), 2.15 (td, *J* = 12.0, 7.1 Hz, 1H), 2.07 (td, *J* = 8.2, 3.9 Hz, 1H), 1.83 - 1.70 (m, 1H), 1.33 (d, *J* = 6.7 Hz, 3H), 1.20 - 1.08 (m, 1H), 0.53 - 0.43 (m, 1H), 0.37 (dt, *J* = 6.6, 3.6 Hz, 1H). Retention time: t = 2.253 min |
| **Example 32-b** | 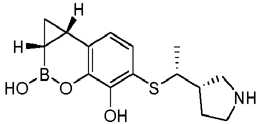 |
| | ¹H NMR (400 MHz, Methanol-*d*₄) δ 7.17 (d, *J* = 8.0 Hz, 1H), 6.98 (d, *J* = 7.9 Hz, 1H), 3.48 - 3.34 (m, 2H), 3.26 - 3.11 (m, 3H), 2.41 (q, *J* = 8.2 Hz, 1H), 2.15 (dt, *J* = 11.6, 6.0 Hz, 1H), 2.07 (d, *J* = 4.0 Hz, 1H), 1.84 - 1.71 (m, 1H), 1.33 (d, *J* = 6.7 Hz, 3H), 1.15 (t, *J* = 9.6 Hz, 1H), 0.47 (q, *J* = 9.2, 8.1 Hz, 1H), 0.39 (dd, *J* = 6.4, 3.4 Hz, 1H). Retention time: t = 2.316 min |
| **Example 32-c** | 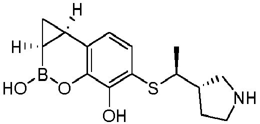 |
| | **¹H NMR (400 MHz, Methanol-d4)** δ 7.09 (d, J = 7.9 Hz, 1H), 6.91 (d, J = 7.9 Hz, 1H), 3.43 - 3.25 (m, 3H), 3.09 - 3.00 (m, 1H), 2.95 (dd, J = 11.9, 8.4 Hz, 1H), 2.50 (td, J = 8.5, 5.2 Hz, 1H), 2.02 (dddd, J = 27.9, 12.2, 7.8, 3.9 Hz, 2H), 1.94 - 1.82 (m, 1H), 1.24 (d, J = 6.9 Hz, 3H), 1.10 - 1.01 (m, 1H), 0.39 (ddd, J = 10.0, 8.5, 6.3 Hz, 1H), 0.26 (dt, J = 6.8, 3.7 Hz, 1H). |
| | **MS (ESI)** m/z (M+ H-18) + = 334.4. Retention time: t = 2.023 min |
| **Example 32-d** | 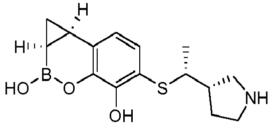 |
| | **¹H NMR (400 MHz, Methanol-d4)** δ 6.90 (d, J = 7.9 Hz, 1H), 6.70 (d, J = 7.8 Hz, 1H), 3.46 - 3.34 (m, 1H), 3.34 - 3.24 (m, 1H), 2.87 (t, J = 11.0 Hz, 2H), 2.65 (dd, J = 12.0, 6.7 Hz, 1H), 2.50 (s, 1H), 2.03 (dd, J = 20.1, 9.3 Hz, 2H), 1.67 (td, J = 8.3, 3.7 Hz, 1H), 1.18 (d, J = 6.9 Hz, 3H)0.80 - 0.67 (m, 1H), 0.30 (dt, J = 6.4, 3.3 Hz, 1H), 0.23 (td, J = 9.2, 6.7 Hz, 1H). |
| | **MS(ESI) m/z** (M+ H-18) + = 334.4. Retention time: t = 2.015 min |
| **Example 33-a** | 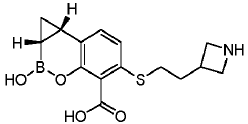 |
| | **¹H NMR (400 MHz, Deuterium Oxide)** δ 7.30 - 7.25 (d,J=8.0 Hz, 1H), 7.09 - 7.02 (m, 1H), 4.10 - 4.05 (m, 2H), 3.81 - 3.76 (m, 2H), 3.12 - 3.03 (m, 1H), 2.88 - 2.82 (m, 2H), 2.29 - 2.22 (m, 1H), 1.87 (q, J = 7.1 Hz, 2H), 1.33 (ddd, J = 11.0, 7.9, 3.4 Hz, 1H), 0.60 - 0.54 (m, 1H), 0.43 - 0.34 (m, 1H). **MS (ESI)** m/z (M+ H)⁺ = 334.4. Retention time: t = 1.520 min |
| **Example 33-b** | 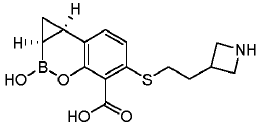 |
| | **¹H NMR (400 MHz, Deuterium Oxide)** δ 7.26 (d, J = 8.0 Hz, 1H), 7.04 (d, J = 8.0 Hz, 1H), 4.08 (d, J = 11.0 Hz, 2H), 3.80 (d, J = 8.1 Hz, 2H), 3.11 - 3.04 (m, 1H), 2.85 (t, J = 7.0 Hz, 2H), 2.24 (td, J = 8.0, 4.1 Hz, 1H), 1.90 - 1.83 (m, 2H), 1.31 (ddd, J = 11.0, 8.0, 3.4 Hz, 1H), 0.58 - 0.51 (m, 1H), 0.41 - 0.36 (m, 1H). **MS (ESI)** m/z (M+ H)⁺ = 334.4. Retention time: t = 1.513 min |
| **Example 34-a** | 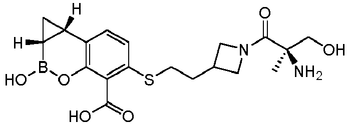 |
| | **¹H NMR (400 MHz, Deuterium Oxide)** δ 7.37 (d, J = 8.2 Hz, 1H), 6.97 (d, J = 8.3 Hz, 1H), 4.17 - 4.04 (m, 1H), 3.86 (d, J = 12.3 Hz, 1H), 3.75 - 3.38 (m, 5H), 3.14 (m, 1H), 2.86 - 2.76 (m, 1H), 2.67 - 2.53 (m, 1H), 2.11 (m, 1H), 1.88 (m, 1H), 1.44 (d, J = 7.8 Hz, 3H), 0.95 (t, J = 8.3 Hz, 1H), 0.44 (s, 1H), 0.42 - 0.25 (m, 1H). **MS (ESI)** m/z (M+ H)⁺ = 421.4. Retention time: t = 1.101 min |
| **Example 34-b** | 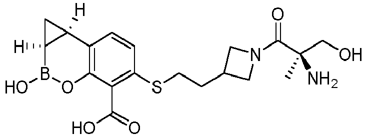 |
| | **¹H NMR (400 MHz, Deuterium Oxide)** δ 7.38 (d, J = 8.2 Hz, 1H), 6.98 (d, J = 8.3 Hz, 1H), 4.14 (m, 1H), 3.88 - 3.66 (m, 3H), 3.58 - 3.44 (m, 3H), 3.23 (m, 1H), 2.88 - 2.48 (m, 2H), 2.28 - 2.00 (m, 1H), 1.89 (td, J = 8.3, 3.4 Hz, 1H), 1.29 (d, J = 14.2 Hz, 3H), 0.95 (t, J = 7.8 Hz, 1H), 0.45 (t, J = 3.8 Hz, 1H), 0.37 (q, J = 8.7 Hz, 1H). **MS (ESI)** m/z (M+ H)⁺ = 421.4. Retention time: t = 1.288 min |
| **Example 35** | 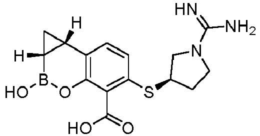 |
| | **¹H NMR (400 MHz, Deuterium Oxide)** δ 7.17 (d, J = 7.8 Hz, 1H), 6.87 (d, J = 7.6 Hz, 1H), 3.95 (s, 1H), 3.67 (dd, J = 16.1, 9.9 Hz, 2H), 3.55 - 3.41 (m, 2H), 2.36 (dd, J = 13.6, 6.5 Hz, 1H), 2.11 (s, 1H), 1.86 (s, 1H), 0.92 (t, J = 7.7 Hz, 1H), 0.41-0.35 (m, 2H). |
| | **MS (ESI)** m/z (M+ H)⁺ = 348.0. |
| **Example 36** | 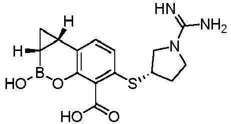 |
| | **¹H NMR (400 MHz, Deuterium Oxide)** δδ 7.17 (d, *J* = 7.2 Hz, 1H), 6.94 (s, 1H), 3.89 (s, 1H), 3.64 - 3.54 (m, 2H), 3.42 (s, 1H), 3.34 (d, *J* = 11.7 Hz, 1H), 2.26 (s, 1H), 2.02 (s, 2H), 1.08 (s, 1H), 0.35 (s, 2H). |
| | **MS (ESI)** m/z (M+ H)⁺ = 348.0. |
| **Example 37** | 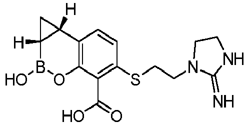 |
| | **¹H NMR (400 MHz, Deuterium Oxide)** δ 7.05 (d, J = 7.8Hz, 1H), 6.83 (d, J = 7.8Hz, 1H), 3.50 (t, J = 8.8Hz, 2H), 3.40 - 3.24 (m, 4H), 2.97 (t, J = 6.3Hz, 2H), 1.90 (d, J = 3.8Hz, 1H), 0.96 (t, J = 7.2Hz, 1H), 0.24 (s, 2H). |
| | **MS (ESI)** m/z (M+ H)⁺ = 347.0. |
| **Example 38** | 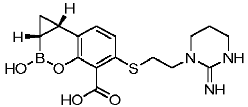 |
| | **¹H NMR (400 MHz, Deuterium Oxide)** δ 7.15 (d, J = 7.2 Hz, 1H), 6.87 (d, J = 6.6 Hz, 1H), 3.51 (s, 2H), 3.34 (s, 2H), 3.21 (s, 2H), 3.14 (s, 2H), 1.89 (s, 3H), 0.92 (s, 1H), 0.35 (d, J = 13.3 Hz, 2H). |
| | **MS (ESI)** m/z (M+ H)⁺ = 362.0. |

### Test Example 1: Enzyme activity test

**Compound stock solution:** the compound was prepared as a 50 mM DMSO stock solution. The positive control compound VNRX-5133 was prepared as a 50 mM DMSO stock solution. After aliquoting, the compound stock solutions were stored at -20°C.

**Preparation and storage of antibiotic substrate stock solution:** antibiotic fluorescent substrate FC5 was synthesized internally and prepared as a 2 mM stock solution, which was stored at -20°C after aliquoting.

**Storage of β-lactamases:** each β-lactamase was synthesized by GenScript Biotech Co., Ltd., and stored at -80°C after aliquoting to avoid repeated freezing and thawing.

**Preparation of enzyme activity reaction buffer:** SBL buffer: PBS pH 7.4, 0.01% (v/v) Triton X-100; MBL buffer: 50 Mm HEPES in PBS pH 7.2, 1 µM ZnSO₄, 1 µg/mL BSA, 0.01% v/v Triton X-100

The buffers were stored at 4°C.

### Experimental steps:

a) The compound to be tested and buffer were taken out and warmed to room temperature.
b) Each compound was diluted with DMSO and buffer to the initial experimental concentration (Table 2), and then the compound was diluted 3-fold for a total of 10 concentrations.
c) Diluted compounds were added to wells 1 to 20 of a 384-well black plate at 2 replicate wells per concentration, 5 µL per well. 5 µL of DMSO was added to wells 21 to 24.
d) Each enzyme was diluted to the experimental concentration (Table 2) in SBL and MBL buffers, and finally 35 µL/ well was added, with a total of 22 wells. 35 µL buffer was added to wells 23 to 24. Wells 21 to well 22 served as the maximum test values (Max), while wells 23 to 24 were designated as the minimum test values (Min).
e) The 384-well plate was centrifuged quickly and incubated for 10 minutes at room temperature.
f) The antibiotic substrate FC5 was diluted in buffer to the experimental concentration of each enzyme (Table 2).
g) The incubated 384-well plate was taken out and FC-5 was added to wells 1-24 at 10 µL per well. The 384-well plate was centrifuged rapidly and promptly transferred to a microplate reader for reading.
h) The fluorescence intensity was measured at 355 nm excitation light / 460nm emission light in the microplate reader, and the fluorescence intensity was measured every three minutes for a total of 10 measurements.

**The test concentrations are shown in Table 2 below.**

**Table 2**

| **Beta-lactamase** | **Enzyme concentration (pM)** | **Substrate concentration (µM)** | **Initial concentration of compound (µM)** |
|---|---|---|---|
| NDM-1 | 6 | 15 | 10 |
| NDM-5 | 15 | 25 | 10 |
| IMP-1 | 20 | 15 | 100 |
| VIM-1 | 70 | 10 | 1 |
| VIM-2 | 25 | 20 | 1 |
| TEM-116 | 300 | 210 | 1 |
| KPC-2 | 45 | 25 | 1 |
| CTX-M-15 | 45 | 50 | 1 |
| AMPC | 25 | 15 | 100 |
| OXA-10 | 350 | 50 | 100 |
| OXA-48 | 650 | 125 | 100 |
| OXA-23 | 650 | 50 | 100 |

**Initial velocity of enzyme reaction V:** Subtract the background reading (Min wells) without enzyme from each fluorescence intensity reading, and then perform on a linear regression analysis on each compound concentration group. The slope is the initial velocity of enzyme reaction (V) at each compound concentration.

**Dose-effect curve and IC₅₀ calculation of compounds:** The initial velocity of enzyme reaction under the effect of each concentration of compounds was subjected to nonlinear regression fitting. The IC₅₀ (half-maximal inhibitory concentration) of each compound was calculated using the formula IC₅₀ = (1 - Vi/V0) × 100 (Vi: the initial velocity of enzyme reaction at each compound concentration; V0: initial velocity of enzyme reaction without the compound (Max wells). The software used was Graphpad prism 8.

**Table 3: IC₅₀ values (nM) of selected compounds in enzyme activity test experiments**

| Compound number | ND M-1 | ND M-5 | IMP -1 | VIM -1 | VIM -2 | TE M-116 | KPC -2 | CTX -M-15 | Amp c | OX A-10 | OX A-48 | OX A-23 |
|---|---|---|---|---|---|---|---|---|---|---|---|---|
| **1** | 13.1 | 15.3 | < 5.1 | 1.5 | 0.1 | 9.4 | 1.2 | 1.3 | 17.2 | 10 | 0.2 | 0.8 |
| **2** | 1.6 | 2.9 | < 5.1 | 0.1 | 0.1 | 18.1 | 3.3 | 3.2 | 42.7 | 6.8 | 0.4 | 0.6 |
| **4** | 1.9 | 2.3 | < 5.1 | < 0.05 | < 0.05 | 11.9 | 1.5 | 1.1 | 33.8 | 3.7 | 0.07 | 1.3 |
| **24-a** | 19.7 | 14.8 | < 5.1 | 7.7 | 6.0 | 34.4 | 3.4 | 3.1 | 63.8 | 39.4 | 1.0 | 1.4 |
| **Avibactam** | > 10⁴ | N/A | > 10⁴ | > 10⁴ | > 10⁴ | 3.0 | N/A | N/A | 331 | 5760 | 382 | N/A |
| **VNRX-5133** | 94.0 | 177 | > 10⁴ | 33.0 | 4.4 | 6.2 | 64.2 | 12.4 | 857 | 463 | 343 | > 10⁴ |

As shown in Table 3, the compounds of the present disclosure can effectively inhibit four classes of β-lactamase compounds, A, B, C, and D, and exhibit significant advantage in inhibitory activity compared to compound VNRX-5133. The drug avibactam on the market has good activity against class A and class C serine β-lactamases **(SBL),** but has no inhibitory effect on class B metal-zinc ion β-lactamase **(MBL),** and low inhibitory activity against the class D serine β-lactamase.

### Test Example 2: Minimum Inhibitory Concentration (MIC) Test of Compounds on Bacterial Growth

### (1) Major reagents and consumables:

**(2) Table 4**

| **Reagents and Consumables** | **Brand** | **Cat. No.** | **Batch** |
|---|---|---|---|
| Mueller Hinton II Broth (Cation-Adjusted)/CAMHB | BD | 212322 | 8190586 |
| Mueller Hinton II Agar (Cation-Adjusted)/CAMHA | BD | 211438 | 9039673 |
| Dimethyl sulfoxide (DMSO) | SIGMA | D4540-1L | BCCB6907 |
| Ceftazidime | TCI | C2225 | AT2DJ-MI |
| Cefoperazone | Selleck Chemicals | S1611 | lot 02 |
| Meropenem Trihydrate | TCI | M2279 | YCY8L-KS |
| Cefepime | Ochem Incorporation | 040C237 | 807165A |
| Avibactam | Shanghai Send Pharmaceutical Technology Co., Ltd | - | - |
| NaCl | Richjoint Chemical | - | 20151101 |
| 96-deep well plate | Corning | 3960 | - |
| 96-U bottom plate | NEST | 701111 | - |
| Polyethylene round bottom test tube | Falcon | 352001 | - |

### (2) Strain:

**Table 5**

| **Strain name** | **Strain number** | **Strain source** |
|---|---|---|
| *Klebsiella pneumoniae* | ARLG 1196 | ATCC |
| *Klebsiella pneumoniae* | ATCC BAA-2470 | ATCC |
| *Escherichia coli* | ARLG 2829 | ATCC |
| *Klebsiella pneumoniae* | ATCC BAA-1705 | ATCC |
| *Klebsiella pneumoniae* | ARLG-1019 | ARLG |
| *Klebsiella pneumoniae* | NCTC-13439 | NCTC |
| *Escherichia coli* | NCTC-13476 | NCTC |

### (3) Experimental methods

**Strain preparation:** the strains to be tested were streaked from glycerol tube at -80°C onto Mueller Hinton II Agar plates. The plates were then placed in a 37°C incubator and cultured for 18-24 hours.

**Media preparation:** CAMHB (Mueller Hinton II Broth): 22 g of powder was dissolved in 1 L of purified water and sterilized at 121°C for 10 min for use. Saline: 8.5 g NaCl was dissolved in 1 L of pure water and sterilized at 121°C for 30 min for use.

**Preparation of compound and antibiotic stock solutions:** the compound and antibiotic were dissolved in DMSO or water to 12.8 mg/mL.

**2-Fold gradient dilution of antibiotics:** 300 µL of CAMHB medium was added to columns 2-12 of a 96-deep well plate. In column 1, 24 µL of antibiotic stock solution and 576 µL of CAMHB medium were added and mixed well, with a concentration of 512 µg/mL. 300 µL of antibiotic solution was transferred from the column 1 to the second column and mixed well. The process was repeated until column 11, and the column 12 was a blank control.

**Dilution of combined compounds:** the compound was diluted 400-fold to 32 ug/ml with CAMHB medium.

**Working solution aliquoting:** 25 µL of antibiotics was aliquoted into the 96-U bottom plate with a pipette, and 25 µL of combined compound was added to each well. If antibiotics were used alone, 25 µL of CAMHB medium was added.

**Inoculum solution preparation:** 5-6 single colonies were selected from the plate and suspended in normal saline. The bacterial suspension was adjusted to 0.2 (i.e., 0.5 McFarland turbidity, with a bacterial concentration was about 1 to 2 × 10⁸ CFU/mL) with a turbidity meter. The bacterial suspension was diluted 100-fold with CAMHB medium for later use.

**Inoculation:** 50 µL of inoculum solution was transferred to the above 50 µL compound plate with a pipette.

**Culture:** The 96-U bottom plate was placed in an incubator at 37°C for 18 to 20 h.

### (4) MIC evaluation:

After the incubation, bacterial growth was visually observed, and the test plate was photographed and recorded. The minimum compound concentration at which bacterial growth is completely inhibited is defined as the Minimum Inhibitory Concentration (MIC).

**Table 6: Determination of MIC values (µg/mL) of selected compounds in combination with antibiotics meropenem/biapenem against different drug-resistant strains**

| Compound number | Klebsiella pneumoniae | | | Escherichia coli | *Acinetobacter baumannii* | | | | | |
|---|---|---|---|---|---|---|---|---|---|---|
| | ATCC BAA-1705 | ARLG -1196 | ATCC BAA-2470 | ARLG-2829 | ARL G-1791 | ARL G-1809 | ARL G-1852 | ARL G-1853 | WX-AB0 05 | WX AB 013 |
| | KPC-2 | CTM-1 group, SHV, NDM, TEM | NDM-1 | NDM-5, OXA-1 | OXA -23 | OXA -23 | OXA -23 | OXA -23 | | |
| Meropenem + **1** (8 µg/mL) | ≤ 0.125 | ≤ 0.125 | ≤ 0.125 | ≤ 0.125 | 4 | 4 | 2 | 2 | 2 | 4 |
| Biapenem + **1** (8 µg/mL) | ≤ 0.125 | ≤ 0.125 | ≤ 0.125 | ≤ 0.125 | 0.5 | 1 | 0.5 | 0.25 | 2 | 1 |
| Meropenem + **2** (8 µg/mL) | 0.5 | 0.25 | 0.25 | 0.25 | 4 | 4 | 2 | 2 | 16 | 2 |
| Biapenem + **2** (8 µg/mL) | ≤ 0.125 | ≤ 0.125 | 0.25 | ≤ 0.125 | 0.5 | 1 | 0.25 | 0.5 | 2 | 1 |
| Meropenem + **3** (8 µg/mL) | 0.5 | 32 | 32 | 128 | 8 | 16 | 4 | 4 | 16 | 4 |
| Biapenem + **3** (8 µg/mL) | ≤ 0.125 | 0.5 | 0.25 | 0.5 | 1 | 1 | 0.25 | 0.25 | 2 | 1 |
| Meropenem + **4** (8 µg/mL) | 1 | ≤ 0.125 | 1 | 0.25 | 4 | 2 | 2 | 2 | 8 | 4 |
| Biapenem + **4** (8 µg/mL) | 0.25 | ≤ 0.125 | 0.5 | ≤ 0.125 | 0.5 | 1 | 0.25 | 0.25 | 2 | 1 |
| Meropenem + **5** (8 µg/mL) | 0.25 | ≤ 0.125 | 0.25 | ≤ 0.125 | 4 | 4 | 2 | 4 | 8 | 2 |
| Biapenem + **5** (8 µg/mL) | ≤ 0.125 | ≤ 0.125 | 0.25 | ≤ 0.125 | 0.5 | 1 | 0.25 | 0.25 | 2 | 0.5 |
| Meropenem + **6** (8 µg/mL) | ≤ 0.125 | 0.5 | 0.5 | 2 | 4 | 4 | 2 | 2 | 8 | 4 |
| Biapenem + **6** (8 µg/mL) | ≤ 0.125 | ≤ 0.125 | 0.5 | ≤ 0.125 | 0.25 | 0.25 | ≤ 0.125 | 0.25 | 2 | 0.5 |
| Meropenem + **7** (8 µg/mL) | ≤ 0.125 | 16 | 8 | 64 | 2 | 4 | 2 | 2 | 8 | 4 |
| Biapenem + **7** (8 µg/mL) | ≤ 0.125 | 0.25 | 0.5 | 0.5 | 0.25 | 0.5 | 0.25 | 0.25 | 2 | 0.5 |
| Meropenem + **8** (8 µg/mL) | ≤ 0.125 | 2 | 0.5 | 0.5 | 2 | 1 | 0.5 | 1 | 4 | 1 |
| Biapenem + **8** (8 pg/mL) | ≤ 0.125 | 0.25 | 0.25 | ≤ 0.125 | ≤ 0.125 | 0.25 | ≤ 0.125 | ≤ 0.125 | 1 | 0.2 5 |
| Meropenem + **9** (8 µg/mL) | ≤ 0.125 | 8 | 8 | 32 | 4 | 4 | 2 | 2 | 8 | 4 |
| Biapenem + 9 (8 µg/mL) | 0.25 | 0.25 | 0.5 | 0.25 | 0.25 | 0.5 | 0.25 | 0.25 | 2 | 2 |
| Meropenem + **10_P1** (8 µg/mL) | ≤ 0.125 | 2 | ≤ 0.125 | 1 | 4 | 2 | 1 | 2 | 4 | 2 |
| Biapenem + **10_P1** (8 µg/mL) | ≤ 0.125 | 0.25 | ≤ 0.125 | ≤ 0.125 | 0.5 | 1 | 0.25 | 0.5 | 2 | 0.5 |
| Meropenem + **10_P2** (8 µg/mL) | ≤ 0.125 | 1 | 0.25 | 0.25 | 4 | 2 | 1 | 1 | 4 | 1 |
| Biapenem + **10_P2** (8 µg/mL) | ≤ 0.125 | 0.25 | 0.5 | ≤ 0.125 | 0.25 | 0.25 | ≤ 0.125 | ≤ 0.125 | 1 | 0.5 |
| Meropenem + **11_P1** (8 µg/mL) | ≤ 0.125 | 0.5 | ≤ 0.125 | ≤ 0.125 | 2 | 2 | 1 | 2 | 4 | 2 |
| Biapenem + **11_P1** (8 µg/mL) | ≤ 0.125 | 0.25 | 0.25 | 0.25 | 0.25 | 0.5 | 0.25 | ≤ 0.125 | 1 | 0.5 |
| Meropenem + **11_P2** (8 µg/mL) | ≤ 0.125 | 2 | 0.5 | ≤ 0.125 | 2 | 2 | 1 | 1 | 4 | 2 |
| Biapenem + **11_P2** (8 µg/mL) | 0.25 | 0.25 | 0.25 | ≤ 0.125 | 0.25 | 0.25 | ≤ 0.125 | ≤ 0.125 | 1 | 0.5 |
| Meropenem + **13** (8 µg/mL) | ≤ 0.125 | 16 | 8 | 32 | 8 | 4 | 2 | 4 | 4 | 4 |
| Biapenem + **13** (8 µg/mL) | ≤ 0.125 | 0.5 | 0.25 | 0.25 | 1 | 1 | 0.5 | 0.5 | 2 | 2 |
| Meropenem + **14_P1** (8 µg/mL) | ≤ 0.125 | 64 | 32 | 128 | 8 | 8 | 4 | 4 | 8 | 8 |
| Biapenem + **14_P1** (µg/mL) | 0.5 | 1 | 1 | 2 | 2 | 4 | 1 | 1 | 4 | 2 |
| Meropenem + **14_P2** (8 µg/mL) | ≤ 0.125 | 64 | 64 | 128 | 8 | 8 | 4 | 4 | 16 | 8 |
| Biapenem + **14_P2** (8 µg/mL) | ≤ 0.125 | 0.5 | 1 | 2 | 2 | 2 | 1 | 1 | 8 | 2 |
| Meropenem + **15-a**(8 µg/mL) | ≤ 0.125 | 2 | 8 | 16 | 8 | 8 | 4 | 16 | 8 | 4 |
| Biapenem + **15-a**(8 µg/mL) | ≤ 0.125 | 1 | 0.25 | 1 | 2 | 2 | 1 | 1 | 4 | 2 |
| Meropenem + **15-b** (8 µg/mL) | ≤ 0.125 | ≤ 0.125 | 1 | 1 | 4 | 2 | 2 | 4 | 4 | 4 |
| Biapenem + **15-b** (8 µg/mL) | ≤ 0.125 | ≤ 0.125 | ≤ 0.125 | 0.25 | 0.5 | 1 | 0.25 | 0.25 | 2 | 1 |
| Meropenem | 4 | 1 | 2 | 2 | 4 | 2 | 2 | 2 | 8 | 4 |
| + **16-a** (8 µg/mL) | | | | | | | | | | |
| Biapenem + **16-a** (8 µg/mL) | 0.5 | 0.5 | 1 | 0.25 | 0.5 | 1 | 0.25 | 0.5 | 2 | 0.5 |
| Meropenem + **16-b** (8 µg/mL) | 2 | 8 | 16 | 16 | 8 | 4 | 4 | 8 | 8 | 4 |
| Biapenem + **16-b** (8 µg/mL) | 0.25 | 0.5 | 1 | 0.5 | 1 | 2 | 0.5 | 1 | 4 | 2 |
| Meropenem + **16-c** (8 µg/mL) | 8 | 2 | 8 | 4 | 8 | 4 | 4 | 4 | 8 | 4 |
| Biapenem + **16-c** (8 µg/mL) | 1 | 1 | 0.5 | ≤ 0.125 | 1 | 2 | 1 | 1 | 4 | 2 |
| Meropenem + **16-d** (8 µg/mL) | 2 | 0.25 | 0.5 | 0.5 | 8 | 4 | 2 | 2 | 8 | 4 |
| Biapenem + **16-d** (8 µg/mL) | 0.5 | ≤ 0.125 | 0.25 | ≤ 0.125 | 0.5 | 0.5 | 0.25 | 0.25 | 2 | 0.5 |
| Meropenem + 17-a (8 µg/mL) | 8 | 8 | 32 | 8 | 16 | 32 | 4 | 8 | 32 | 8 |
| Biapenem + 17-a (8 µg/mL) | 0.5 | 0.5 | 0.25 | 0.25 | 2 | 4 | 0.5 | 1 | 4 | 1 |
| Meropenem + 17-b (8 µg/mL) | 8 | 8 | 64 | 4 | 16 | 32 | 8 | 16 | 32 | 16 |
| Biapenem + 17-b (8 µg/mL) | 2 | 0.5 | 0.25 | 0.25 | 2 | 4 | 0.5 | 1 | 4 | 2 |
| Meropenem + 17-c (8 µg/mL) | 4 | 8 | 64 | 8 | 16 | 16 | 4 | 8 | 16 | 8 |
| Biapenem + 17-c (8 µg/mL) | 0.5 | 1 | 0.5 | 0.5 | 2 | 2 | 0.5 | 1 | 4 | 2 |
| Meropenem + 17-**d** (8 µg/mL) | 16 | 16 | 64 | 32 | 16 | 16 | 8 | 8 | 16 | 8 |
| Biapenem + 17-**d** (8 µg/mL) | 1 | 1 | 0.5 | 0.5 | 2 | 4 | 1 | 1 | 4 | 4 |
| Meropenem + 18-**a** (8 µg/mL) | 1 | 2 | 8 | 2 | 8 | 4 | 2 | 2 | 8 | 4 |
| Biapenem + 18-**a** (8 µg/mL) | 0.5 | ≤ 0.125 | 0.5 | ≤ 0.125 | 0.5 | 2 | 0.5 | 0.5 | 4 | 0.5 |
| Meropenem + 18-**b** (8 µg/mL) | 0.5 | 1 | 8 | 2 | 8 | 8 | 4 | 8 | 8 | 4 |
| Biapenem + 18-**b** (8 | ≤ 0.125 | 0.25 | ≤ 0.125 | 0.25 | 2 | 4 | 1 | 1 | 8 | 2 |
| µg/mL) | | | | | | | | | | |
| Meropenem + 18-**c** (8 µg/mL) | 2 | 1 | 1 | 0.5 | 4 | 4 | 2 | 4 | 2 | 2 |
| Biapenem + **4c** (8 µg/mL) | 0.5 | ≤ 0.125 | 0.25 | ≤ 0.125 | 0.5 | 2 | 0.5 | 0.5 | 2 | 1 |
| Meropenem + 18-**d** (8 µg/mL) | 0.5 | 4 | 16 | 8 | 8 | 8 | 8 | 4 | 8 | 4 |
| Biapenem + 18-**d** (8 µg/mL) | 0.5 | ≤ 0.125 | 0.5 | 0.5 | 1 | 4 | 1 | 1 | 4 | 1 |
| Meropenem + **19-a** (8 µg/mL) | 4 | 2 | 32 | 2 | 8 | 4 | 2 | 4 | 8 | 2 |
| Biapenem + **19-a** (8 µg/mL) | 0.5 | ≤ 0.125 | 0.5 | 0.5 | 1 | 4 | 0.5 | 0.5 | 2 | 1 |
| Meropenem + **19-b** (8 µg/mL) | 1 | 2 | 32 | 4 | 16 | 8 | 4 | 4 | 8 | 8 |
| Biapenem + **19-b** (8 µg/mL) | 0.25 | 0.5 | 0.5 | ≤ 0.125 | 2 | 4 | 1 | 1 | 4 | 2 |
| Meropenem + **19-c** (8 µg/mL) | 8 | 1 | 16 | 1 | 8 | 8 | 4 | 4 | 8 | 4 |
| Biapenem + | 0.5 | 0.25 | 0.5 | ≤ 0.125 | 2 | 2 | 0.5 | 1 | 8 | 2 |
| **19-c** (8 µg/mL) | | | | | | | | | | |
| Meropenem + **19-d** (8 µg/mL) | 4 | 8 | 32 | 16 | 16 | 8 | 4 | 16 | 32 | 4 |
| Biapenem + **19-d** (8 µg/mL) | 0.5 | 0.25 | 1 | 0.25 | 4 | 4 | 1 | 2 | 4 | 2 |
| Meropenem + **20-a** (8 µg/mL) | ≤ 0.125 | 2 | 4 | 16 | 2 | 2 | 2 | 2 | 4 | 2 |
| Biapenem + **20-a** (8 µg/mL) | 0.5 | ≤ 0.125 | ≤ 0.125 | 0.25 | 0.25 | 0.5 | 0.25 | 0.25 | 2 | 0.5 |
| Meropenem + **20-b** (8 µg/mL) | ≤ 0.125 | 32 | 16 | 64 | 8 | 4 | 4 | 4 | 8 | 4 |
| Biapenem + **20-b** (8 µg/mL) | 0.5 | 0.25 | 1 | 0.5 | 2 | 2 | 1 | 1 | 4 | 4 |
| Meropenem + **20-c** (8 µg/mL) | ≤ 0.125 | 128 | 64/> 128 | >128 | 8 | 8 | 2 | 2 | 8 | 4 |
| Biapenem + **20-c** (8 µg/mL) | ≤ 0.125 | 2 | 2 | 2 | 2 | 2 | 0.5 | 0.5 | 4 | 2 |
| Meropenem + **20-d** (8 µg/mL) | ≤ 0.125 | 0.5 | 1 | 2 | 4 | 8 | 2 | 4 | 16 | 4 |
| Biapenem + **20-d** (8 µg/mL) | 0.25 | ≤ 0.125 | 0.5 | ≤ 0.125 | 0.25 | 1 | ≤ 0.125 | ≤ 0.125 | 2 | 1 |
| Meropenem + **21-a** (8 µg/mL) | 2 | 2 | 16 | 1 | 4 | 4 | 2 | 4 | 8 | 4 |
| Biapenem + **21-a** (8 µg/mL) | 0.5 | 0.25 | 0.25 | ≤ 0.125 | 1 | 2 | 0.5 | 0.5 | 2 | 1 |
| Meropenem + **21-b** (8 µg/mL) | 0.5 | 4 | 16 | 2 | 8 | 8 | 4 | 4 | 8 | 4 |
| Biapenem + **21-b** (8 µg/mL) | 0.5 | 0.25 | 0.25 | 0.25 | 2 | 4 | 0.5 | 2 | 8 | 2 |
| Meropenem + **21-c** (8 µg/mL) | 2 | 0.5 | 8 | 1 | 8 | 4 | 8 | 8 | 8 | 4 |
| Biapenem + **21-c** (8 µg/mL) | 0.5 | 0.25 | 0.25 | 0.25 | 1 | 2 | 0.5 | 1 | 4 | 1 |
| Meropenem + **21-d** (8 µg/mL) | 2/8 | 8 | 32 | 16 | 8 | 8 | 4 | 8 | 16 | 4 |
| Biapenem + **21-d** (8 µg/mL) | 0.5 | 0.5 | 1 | 0.5 | 2 | 4 | 1 | 1 | 4 | 4 |
| Meropenem + **22-a** (8 | 1 | 2 | 8 | 2 | 8 | 4 | 2 | 2 | 8 | 4 |
| µg/mL) | | | | | | | | | | |
| Biapenem + **22-a** (8 µg/mL) | 0.5 | ≤ 0.125 | 0.5 | ≤ 0.125 | 0.5 | 2 | 0.5 | 0.5 | 4 | 0.5 |
| Meropenem + **22-b** (8 µg/mL) | 0.5 | 1 | 8 | 2 | 8 | 8 | 4 | 8 | 8 | 4 |
| Biapenem + **22-b** (8 µg/mL) | ≤ 0.125 | 0.25 | ≤ 0.125 | 0.25 | 2 | 4 | 1 | 1 | 8 | 2 |
| Meropenem + **22-c** (µg/mL) | 2 | 1 | 1 | 0.5 | 4 | 4 | 2 | 4 | 2 | 2 |
| Biapenem + **22-c** (8 µg/mL) | 0.5 | ≤ 0.125 | 0.25 | ≤ 0.125 | 0.5 | 2 | 0.5 | 0.5 | 2 | 1 |
| Meropenem + **22-d** (8 µg/mL) | 0.5 | 4 | 16 | 8 | 8 | 8 | 8 | 4 | 8 | 4 |
| Biapenem + **22-d** (8 µg/mL) | 0.5 | ≤ 0.125 | 0.5 | 0.5 | 1 | 4 | 1 | 1 | 4 | 1 |
| Meropenem + **23-a** (8 µg/mL) | 1 | 8 | 8 | 2 | 4 | 4 | 2 | 4 | 8 | 4 |
| Biapenem + **23-a** (8 µg/mL) | 0.5 | 0.25 | 0.25 | 0.5 | 1 | 1 | 0.5 | 0.5 | 2 | 1 |
| Meropenem | 1 | 8 | 8 | 2 | 4 | 4 | 2 | 4 | 8 | 4 |
| + **23-b** (8 µg/mL) | | | | | | | | | | |
| Biapenem + **23-b** (8 µg/mL) | 1 | 0.5 | 1 | ≤ 0.125 | 1 | 2 | 0.5 | 0.5 | 4 | 2 |
| Meropenem + **23-c** (8 µg/mL) | 2 | 1 | 4 | 0.25 | 2 | 2 | 1 | 2 | 4 | 4 |
| Biapenem + **23-c** (8 µg/mL) | 0.25 | 0.5 | 0.25 | ≤ 0.125 | 0.5 | 2 | 0.25 | 0.25 | 2 | 1 |
| Meropenem + **23-d** (8 µg/mL) | 1 | 16 | 16 | 8 | 4 | 4 | 2 | 4 | 8 | 4 |
| Biapenem + **23-d** (8 µg/mL) | 0.25 | 0.5 | 0.25 | 0.25 | 2 | 2 | 0.5 | 0.5 | 4 | 2 |
| Meropenem + **24-a** (8 µg/mL) | ≤ 0.125 | 0.25 | 0.5 | 0.5 | 2 | 2 | 1 | 2 | 8 | 2 |
| Biapenem + **24-a** (8 µg/mL) | ≤ 0.125 | 0.25 | 0.5 | ≤ 0.125 | 0.25 | 0.5 | 0.25 | 0.25 | 2 | 1 |
| Meropenem + **24-b** (8 µg/mL) | ≤ 0.125 | 16 | 16 | 64 | 16 | 8 | 8 | 8 | 16 | 8 |
| Biapenem + **24-b** (8 µg/mL) | ≤ 0.125 | 0.5 | 0.25 | 0.5 | 1 | 4 | 1 | 1 | 8 | 2 |
| Meropenem + **25-a** (8 µg/mL) | ≤ 0.125 | 0.25 | 0.25 | 1 | 4 | 4 | 2 | 4 | 8 | 4 |
| Biapenem + **25-a** (8 µg/mL) | 0.25 | ≤ 0.125 | ≤ 0.125 | 0.25 | 0.5 | 1 | 0.5 | 0.25 | 4 | 1 |
| Meropenem + **25-b** (8 µg/mL) | 0.25 | 16 | 16 | 64 | 32 | 16 | 8 | 16 | 16 | 8 |
| Biapenem + **25-b** (8 µg/mL) | ≤ 0.125 | 0.25 | 0.25 | 0.5 | 4 | 8 | 2 | 2 | 16 | 8 |
| Meropenem + **26-a** (8 µg/mL) | ≤ 0.125 | 0.25 | 0.5 | 1 | 4 | 8 | 2 | 2 | 8 | 2 |
| Biapenem + **26-a** (8 µg/mL) | 0.25 | ≤ 0.125 | ≤ 0.125 | ≤ 0.125 | 0.5 | 4 | 0.5 | 0.5 | 2 | 0.5 |
| Meropenem + **26-b** (8 µg/mL) | ≤ 0.125 | 4 | 4 | 32 | 16 | 16 | 16 | 16 | 16 | 8 |
| Biapenem + **26-b** (8 µg/mL) | 0.25 | 0.25 | 0.5 | 0.25 | 2 | 8 | 2 | 2 | 8 | 4 |
| Meropenem + **27-a** (8 µg/mL) | 8 | 1 | 16 | 1 | 8 | 4 | 2 | 4 | 8 | 4 |
| Biapenem + **27-a** (8 µg/mL) | 1 | ≤ 0.125 | 0.25 | ≤ 0.125 | 1 | 1 | 0.25 | 0.5 | 2 | 1 |
| Meropenem + **27-b** (8 µg/mL) | 4 | 1 | 8 | 1 | 4 | 4 | 2 | 4 | 16 | 2 |
| Biapenem + **27-b** (8 µg/mL) | 1 | 0.25 | 0.5 | 0.5 | 1 | 2 | 0.25 | 0.5 | 2 | 1 |
| Meropenem + **27-c** (8 µg/mL) | 16 | 8 | 32 | 16 | 16 | 8 | 8 | 8 | 32 | 8 |
| Biapenem + **27-c** (8 µg/mL) | 2 | ≤ 0.125 | 1 | 0.5 | 4 | 4 | 2 | 2 | 8 | 4 |
| Meropenem + **27-d** (8 µg/mL) | 2 | 8 | 32 | 16 | 8 | 8 | 8 | 4 | 16 | 4 |
| Biapenem + **27-d** (8 µg/mL) | 0.5 | 0.25 | 0.5 | 0.25 | 2 | 4 | 1 | 1 | 4 | 2 |
| Meropenem + **28-a** (8 µg/mL) | ≤ 0.125 | ≤ 0.125 | 0.25 | 0.25 | 2 | 2 | 1 | 2 | 8 | 2 |
| Biapenem + **28-a** (8 µg/mL) | ≤ 0.125 | 0.25 | 0.5 | ≤ 0.125 | 0.25 | 0.5 | 0.25 | 0.25 | 2 | 0.5 |
| Meropenem + **28-b** (8 µg/mL) | ≤ 0.125 | 4 | 4 | 32 | 8 | 16 | 8 | 8 | 32 | 16 |
| Biapenem + **28-b** (8 µg/mL) | ≤ 0.125 | 0.25 | 0.5 | 0.25 | 1 | 4 | 0.5 | 1 | 8 | 4 |
| Meropenem + 29-a (8 µg/mL) | ≤ 0.125 | 2 | 4 | 8 | 4 | 8 | 1 | 2 | 16 | 4 |
| Biapenem + 29-a (8 µg/mL) | 0.25 | ≤ 0.125 | 0.25 | ≤ 0.125 | ≤ 0.125 | 0.5 | ≤ 0.125 | ≤ 0.125 | 2 | 0.2 5 |
| Meropenem + 29-d (8 µg/mL) | ≤ 0.125 | 0.25 | 0.5 | 0.5 | 4 | 8 | 2 | 2 | 16 | 2 |
| Biapenem + 29-d (8 µg/mL) | 0.25 | ≤ 0.125 | 0.5 | ≤ 0.125 | 0.5 | 0.5 | ≤ 0.125 | ≤ 0.125 | 2 | 0.5 |
| Meropenem + **30-a** (8 µg/mL) | ≤ 0.125 | 32 | 16 | 128 | 8 | 16 | 2 | 4 | 16 | 4 |
| Biapenem + **30-a** (8 µg/mL) | ≤ 0.125 | 0.25 | 0.5 | 1 | 0.5 | 0.5 | ≤ 0.125 | ≤ 0.125 | 2 | 0.5 |
| Meropenem + **30-b** (8 µg/mL) | ≤ 0.125 | >128 | 128 | >128 | 16 | 16 | 4 | 8 | 16 | 8 |
| Biapenem + **30-b** (8 µg/mL) | 0.5 | 1 | 2 | 4 | 1 | 2 | 0.5 | 0.5 | 4 | 2 |
| Meropenem + **31-a** (8 µg/mL) | 4 | 2 | 16 | 2 | 4 | 4 | 2 | 4 | 8 | 2 |
| Biapenem + **31-a** (8 µg/mL) | 1 | 0.5 | 0.5 | ≤ 0.125 | 1 | 2 | 0.25 | 0.5 | 4 | 1 |
| Meropenem + **31-b** (8 µg/mL) | 4 | 16 | 64 | 32 | 16 | 16 | 16 | 8 | 32 | 8 |
| Biapenem + **31-b** (8 µg/mL) | 0.25 | 0.5 | 1 | ≤ 0.125 | 1 | 4 | 0.5 | 1 | 8 | 2 |
| Meropenem + **32-c** (8 µg/mL) | ≤ 0.125 | 1 | 1 | 4 | 4 | 8 | 2 | 4 | 16 | 4 |
| Biapenem + **32-c** (8 µg/mL) | ≤ 0.125 | ≤ 0.125 | 1 | 0.25 | 0.25 | 1 | ≤ 0.125 | ≤ 0.125 | 2 | 0.2 5 |
| Meropenem + **32-d** (8 µg/mL) | ≤ 0.125 | 64 | 64 | >128 | 8 | 16 | 8 | 4 | 16 | 4 |
| Biapenem + **32-d** (8 µg/mL) | 0.25 | 0.25 | 0.25 | 1 | 0.5 | 2 | 0.25 | 0.25 | 4 | 1 |
| Meropenem + **33-a** (8 µg/mL) | ≤ 0.125 | 0.25 | 0.5 | 0.5 | 8 | 16 | 4 | 4 | 16 | 2 |
| Biapenem + **33-a** (8 µg/mL) | ≤ 0.125 | ≤ 0.125 | 0.5 | ≤ 0.125 | 0.25 | 0.5 | 0.25 | ≤ 0.125 | 1 | 0.2 5 |
| Meropenem + **33-b** (8 µg/mL) | ≤ 0.125 | 4 | 4 | 8 | 8 | 8 | 4 | 8 | 16 | 8 |
| Biapenem + **33-b** (8 µg/mL) | ≤ 0.125 | ≤ 0.125 | 0.5 | ≤ 0.125 | 0.5 | 2 | 0.25 | ≤ 0.125 | 4 | 1 |
| Meropenem + **34-a** (8 µg/mL) | 0.25 | 1 | 4 | 4 | 32 | 32 | 32 | 32 | 32 | 32 |
| Biapenem + **34-a** (8 µg/mL) | 0.5 | 0.5 | ≤ 0.125 | 0.5 | 8 | 8 | 4 | 4 | 16 | 16 |
| Meropenem + **34-b** (8 µg/mL) | 0.5 | >128 | 128 | >128 | 128 | 128 | 64 | 32 | 128 | 64 |
| Biapenem + **34-b** (8 µg/mL) | 0.5 | 2 | 2 | 8 | 8 | 16 | 8 | 8 | 16 | 16 |
| Meropenem + **35** (8 µg/mL) | ≤ 0.125 | 0.12 | 0.5 | 0.5 | 8 | 16 | 2 | 4 | 16 | 4 |
| Biapenem + **35** (8 µg/mL) | ≤ 0.125 | ≤ 0.125 | 0.25 | 0.25 | 0.5 | 1 | ≤ 0.125 | 0.25 | 2 | 1 |
| Meropenem + **36** (8 µg/mL) | ≤ 0.125 | 0.5 | 1 | 2 | 8 | 8 | 2 | 4 | 16 | 4 |
| Biapenem + **36** (8 µg/mL) | ≤ 0.125 | ≤ 0.125 | 0.25 | ≤ 0.125 | ≤ 0.125 | 0.25 | ≤ 0.125 | ≤ 0.125 | 1 | 0.2 5 |
| Cefepime + **VNRX5133** (8 µg/mL) | 0.25 | 1 | 1 | 1 | 16 | 32 | 32 | 32 | 128 | 64 |
| Meropenem + **VNRX5133** (8 µg/mL) | 0.125 | 1 | 1 | 1 | 128 | 128 | 128 | 128 | 128 | 128 |
| Cefepime | >128 | >64 | >128 | >64 | 128 | 128 | 128 | 128 | 64 | 32 |
| Meropenem | 128 | 128 | 128 | >128 | >128 | 32 | 128 | 128 | >128 | 64 |
| Biapenem | 32 | 8 | 4 | 16 | 16 | 32 | 32 | 32 | 32 | 64 |

As shown in Table 6, for carbapenem-resistant Acinetobacter baumannii strains, the compounds of the present disclosure can effectively restore the activity of antibiotics cefepime/meropenem/biapenem. The combination of VNRX5133 and cefepime/meropenem is essentially ineffective against all resistant strains of Acinetobacter baumannii (MIC > 32 µg/mL); the combination of the compounds of the present disclosure and meropenem/biapenem is effective against all resistant strains, demonstrating significant advantages in terms of both activity and inhibition spectrum compared to the combination of VNRX5133 and cefepime.

## Claims

1. A compound of formula (I), an optical isomer thereof, and a pharmaceutically acceptable salt thereof, 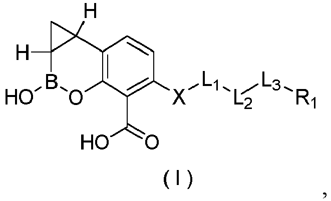 wherein,
X is selected from S, S(=O), and S(=O)₂;
L₁ is selected from a single bond, C₁₋₆ alkyl, and C₁₋₆ heteroalkyl, and the C₁₋₆ alkyl or C₁₋₆ heteroalkyl is optionally substituted by 1, 2, or 3 R;
L₂ is selected from a single bond, C₃₋₆ cycloalkyl, and 4- to 9-membered heterocycloalkyl, and the C₃₋₆ cycloalkyl or 4- to 9-membered heterocycloalkyl is optionally substituted by 1, 2, or 3 R;
L₃ is selected from a single bond, C₁₋₆ alkyl, C₁₋₆ heteroalkyl, and 4- to 9-membered heterocycloalkyl, and the C₁₋₆ alkyl, C₁₋₆ heteroalkyl, or 4- to 9-membered heterocycloalkyl is optionally substituted by 1, 2, or 3 R;
Ri is selected from NH₂, 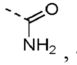 -NRₐC(=NH)R_{b}, -NRₐC(=NH)NR_{b1}R_{b2}, -C(=NH)R_{b}, Cl-₆ alkyl, C₃₋₆ cycloalkyl, 4- to 8-membered heterocycloalkyl, partially unsaturated 4- to 8-membered heterocycloalkyl, and 5- to 6-membered heteroaryl, and the C₁₋₆ alkyl, C₃₋₆ cycloalkyl, 4- to 8-membered heterocycloalkyl, partially unsaturated 4- to 8-membered heterocycloalkyl, or 5- to 6-membered heteroaryl is optionally substituted by 1, 2, or 3 R;
R, Rₐ, R_{b}, R_{b1}, and R_{b2} are each independently selected from H, F, Cl, Br, OH, NH₂, CN, C₁₋₆ alkyl, and C₃₋₆ cycloalkyl, and the C₁₋₆ alkyl or C₃₋₆ cycloalkyl is optionally substituted by 1, 2, or 3 R';
each R' is independently selected from F, Cl, Br, I, NH₂, OH, and Me;
the C₁₋₆ heteroalkyl, 4- to 9-membered heterocycloalkyl, partially unsaturated 4- to 8-membered heterocycloalkyl, or 5- to 6-membered heteroaryl comprises 1, 2, or 3 heteroatoms or heteroatom group independently selected from O, NH, S, C(=O), C(=NH), C(=O)O, S(=O), S(=O)₂, and N.

2. The compound of formula (I), the optical isomer thereof, and the pharmaceutically acceptable salt thereof according to claim 1, selected from compounds of formulas (I-A) and (I-B), optical isomers thereof, and pharmaceutically acceptable salts thereof, 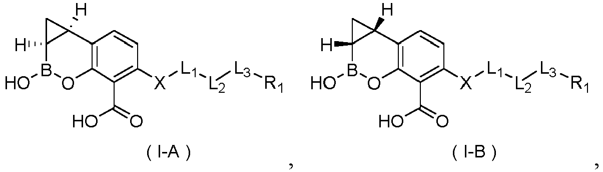 wherein
X, L₁, L₂, L₃, and Ri are as defined in claim 1.

3. The compound, the optical isomer thereof, and the pharmaceutically acceptable salt thereof according to claim 1 or 2, wherein each R is independently selected from R selected from F, Cl, Br, OH, NH₂, CH₃, 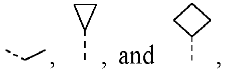 and the CH₃, 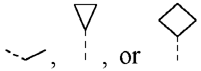 is optionally substituted by 1, 2, or 3 R'.

4. The compound, the optical isomer thereof, and the pharmaceutically acceptable salt thereof according to claim 3, wherein each R is independently selected from F, Cl, Br, OH, NH₂, CH₃, 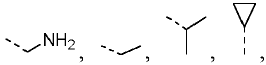 and

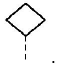

5. The compound, the optical isomer thereof, and the pharmaceutically acceptable salt thereof according to claim 1 or 2, wherein L₁ is selected from a single bond, C₁₋₃ alkyl, -C₁₋₃ alkyl-O-, -C₁₋₃ alkyl-S-, -C₁₋₃ alkyl-NH-, and -C₁₋₃ alkyl-C(=O)-, and the C₁₋₃ alkyl, -C₁₋₃ alkyl-O-, -C₁₋₃ alkyl-S-, -C₁₋₃ alkyl-NH-, or -C₁₋₃ alkyl-C(=O) - is optionally substituted by 1, 2, or 3 R.

6. The compound, the optical isomer thereof, and the pharmaceutically acceptable salt thereof according to claim 5, wherein L₁ is selected from a single bond, CH₂, 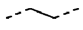 , 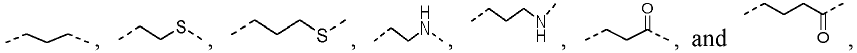 and the CH₂, 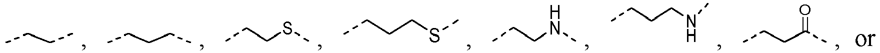 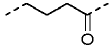 is optionally substituted by 1, 2, or 3 R.

7. The compound, the optical isomer thereof, and the pharmaceutically acceptable salt thereof according to claim 6, wherein L₁ is selected from a single bond, CH₂, 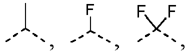 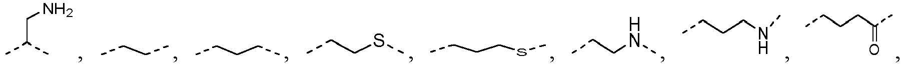 and

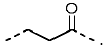

8. The compound, the optical isomer thereof, and the pharmaceutically acceptable salt thereof according to claim 1 or 2, wherein L₂ is selected from a single bond, cyclobutyl, cyclopentyl, azetidinyl, piperidinyl, piperazinyl, morpholinyl, and 2,6-diazaspiro[3.3]heptyl, and the cyclobutyl, cyclopentyl, azetidinyl, piperidinyl, piperazinyl, morpholinyl, or 2,6-diazaspiro[3.3]heptyl is optionally substituted by 1, 2, or 3 R.

9. The compound, the optical isomer thereof, and the pharmaceutically acceptable salt thereof according to claim 8, wherein L₂ is selected from a single bond,

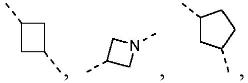

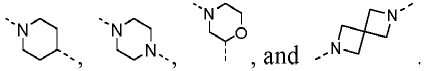

10. The compound, the optical isomer thereof, and the pharmaceutically acceptable salt thereof according to claim 1 or 2, wherein L₃ is selected from a single bond, C(=O), C₁₋₃ alkyl, -C₁₋₃ alkyl-C(=O)-, and -C₁₋₃ alkyl-C(=O)NH-, and the C₁₋₃ alkyl, -C₁₋₃ alkyl-C(=O)-, or -C₁₋₃ alkyl-C(=O)NH- is optionally substituted by 1, 2, or 3 R.

11. The compound, the optical isomer thereof, and the pharmaceutically acceptable salt thereof according to claim 10, wherein L₃ is selected from a single bond, C(=O),

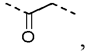

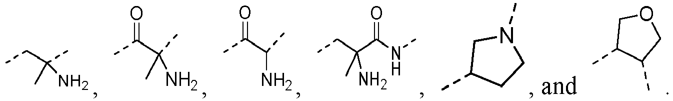

12. The compound, the optical isomer thereof, and the pharmaceutically acceptable salt thereof according to claim 1 or 2, wherein Ri is selected from NH₂, 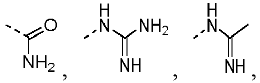 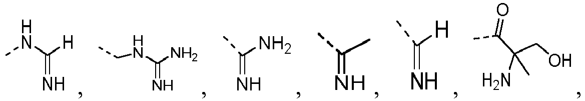 C₁₋₃ alkyl, cyclopropyl, cyclobutyl, oxetanyl, azetidinyl, pyrrolidinyl, tetrahydrofuranyl, tetrahydro-2H-pyranyl, morpholinyl, piperidinyl, piperazinyl, octahydrocyclopentadieno[c]pyrrolyl, 4,5-dihydro-1H-imidazolyl, imidazolyl, 4,5-dihydro-1H-imidazolyl, imidazolin-2-imino, tetrahydropyrimidin-2(1H)-imino, 2,6-diazaspiro[3.3]heptyl, 2-oxa-6-azaspiro[3.3]heptyl, 2,6-diazaspiro[3.4]octyl, 6-oxa-2-azaspiro[3.4]octyl, 2,6-diazaspiro[3.4]octyl, 3,6-diazabicyclo[3.1.1]heptyl, and 2,5-diazabicyclo[2.2.1]heptyl, and the C₁₋₃ alkyl, cyclopropyl, cyclobutyl, oxetanyl, azetidinyl, pyrrolidinyl, tetrahydrofuranyl, tetrahydro-2H-pyranyl, morpholinyl, piperidinyl, piperazinyl, octahydrocyclopentadieno[c]pyrrolyl, 4,5-dihydro-1H-imidazolyl, imidazolyl, 4,5-dihydro-1H-imidazolyl, imidazolin-2-imino, tetrahydropyrimidin-2(1H)-imino, 2,6-diazaspiro[3.3]heptyl, 2-oxa-6-azaspiro[3.3]heptyl, 2,6-diazaspiro[3.4]octyl, 6-oxa-2-azaspiro[3.4]octyl, 2,6-diazaspiro[3.4]octyl, 3,6-diazabicyclo[3.1.1]heptyl, or 2,5-diazabicyclo[2.2.1]heptyl is optionally substituted by 1, 2, or 3 R.

13. The compound, the optical isomer thereof, and the pharmaceutically acceptable salt thereof according to claim 12, wherein Ri is selected from NH₂, 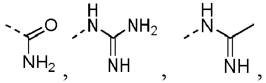 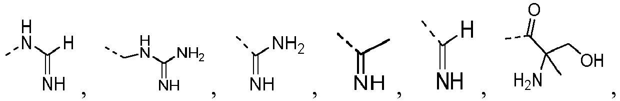 Me, 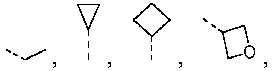 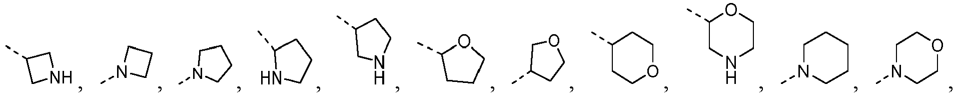 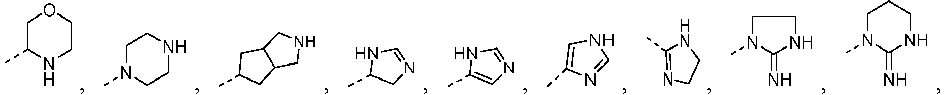 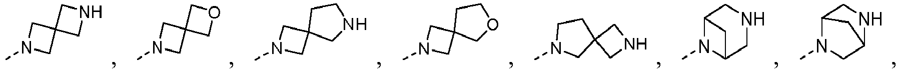 and 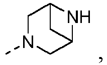 and the Me, 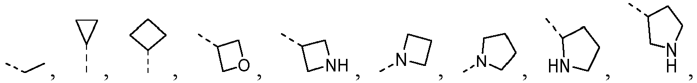 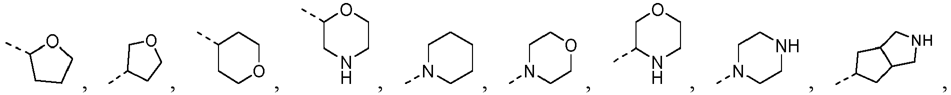 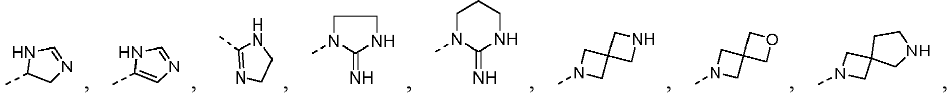 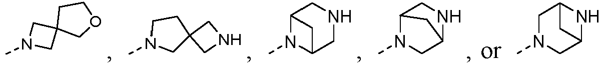 is optionally substituted by 1, 2, or 3 R.

14. The compound, the optical isomer thereof, and the pharmaceutically acceptable salt thereof according to claim 13, wherein Ri is selected from NH₂, Me, CF₃, 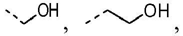 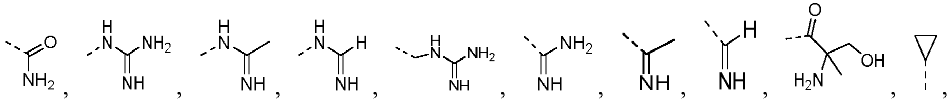 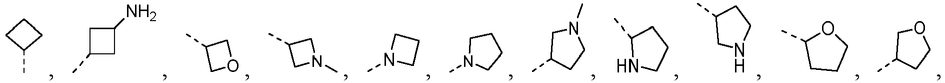 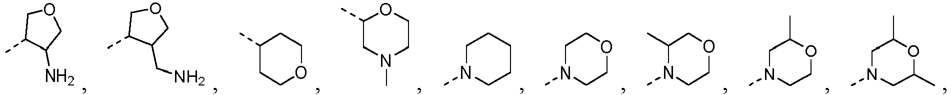 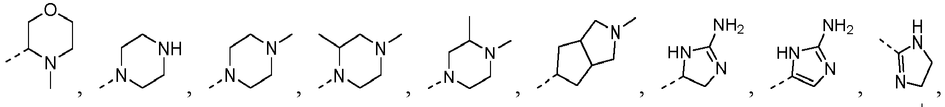 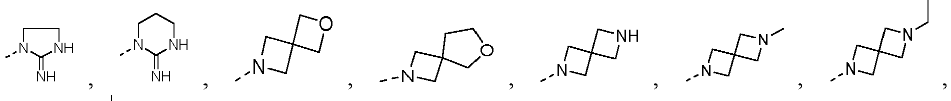 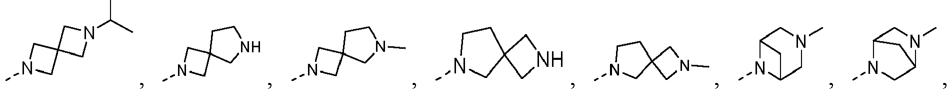 and

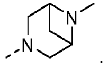

15. The compound, the optical isomer thereof, and the pharmaceutically acceptable salt thereof according to any one of claims 7, 9, 11, or 14, wherein the structural moiety 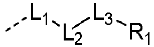 is selected from Me, CF₃,

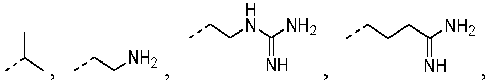

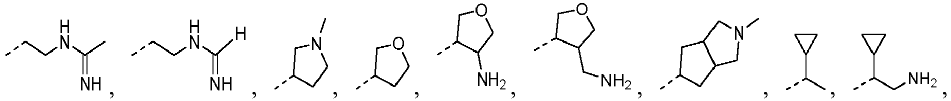

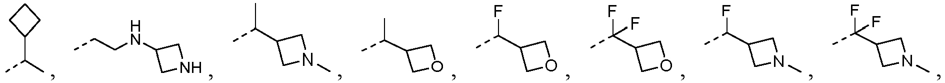

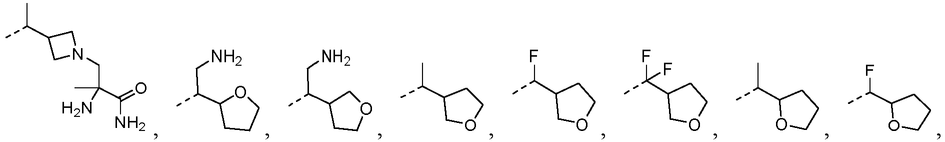

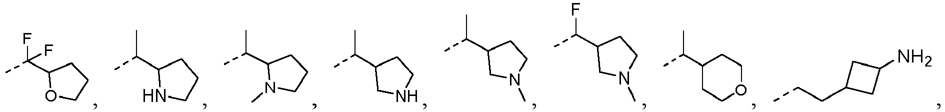

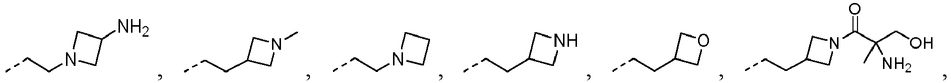

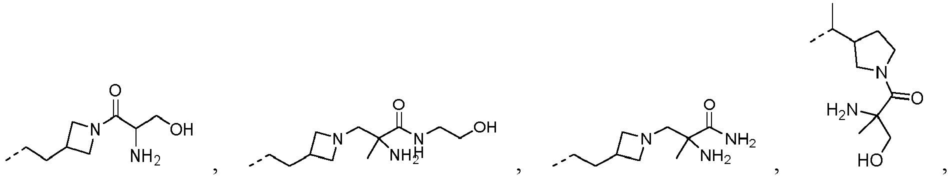

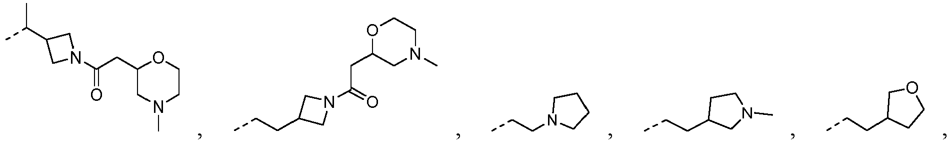

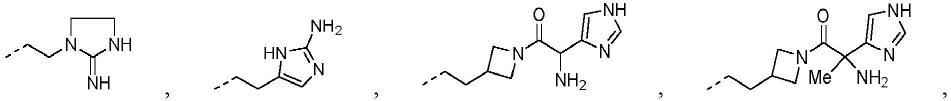

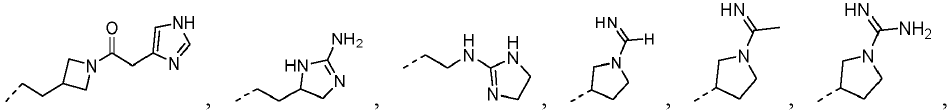

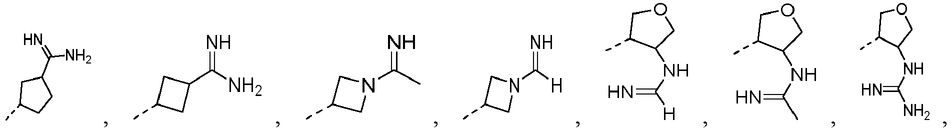

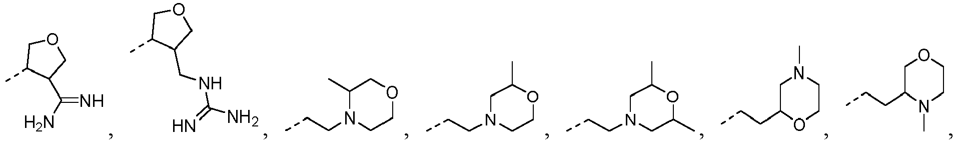

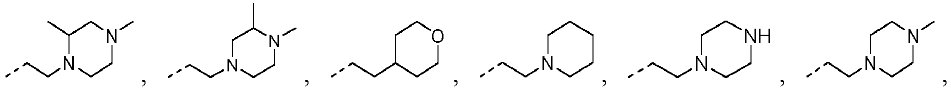

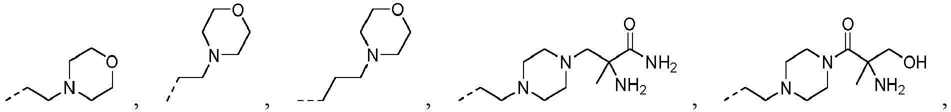

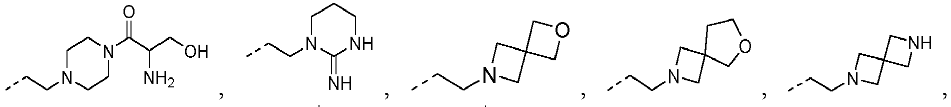

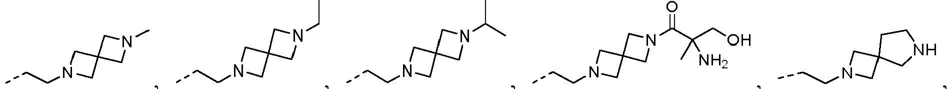

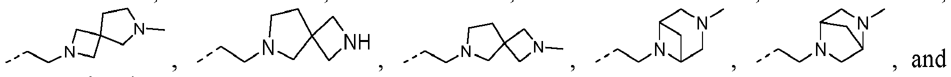

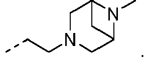

16. A compound of the following formula, an optical isomer thereof, and a pharmaceutically acceptable salt thereof, selected from:

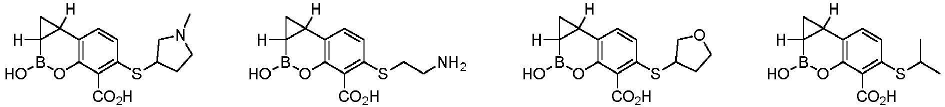

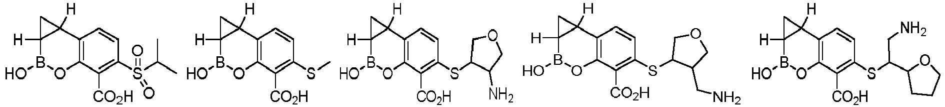

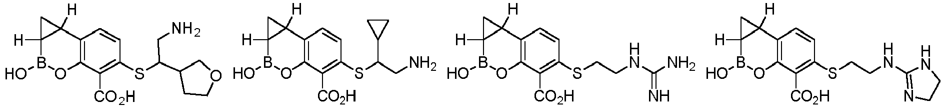

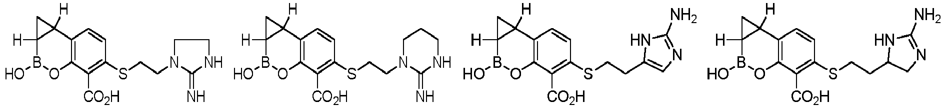

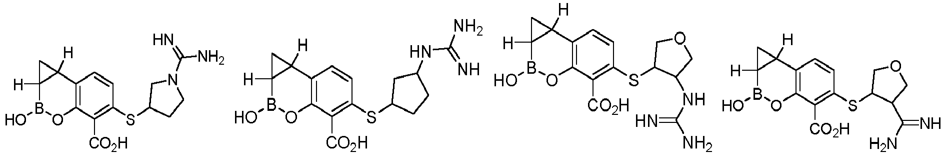

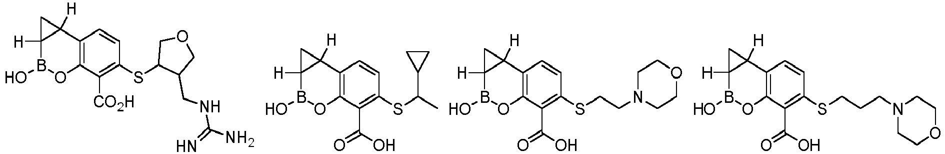

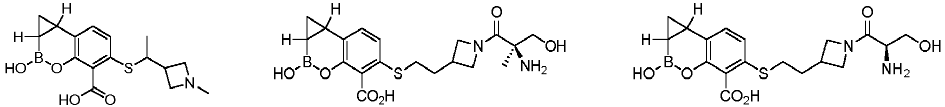

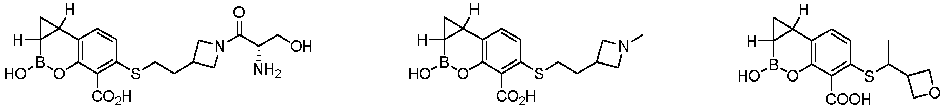

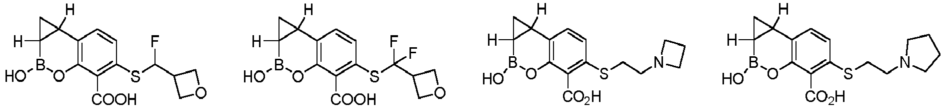

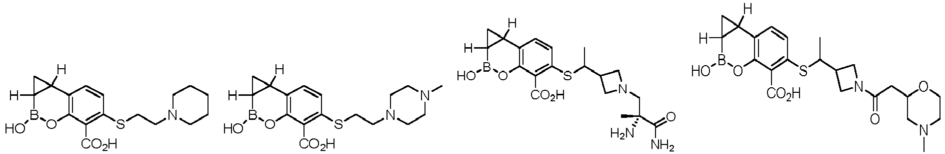

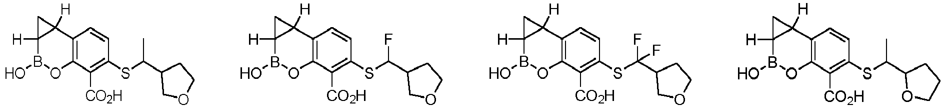

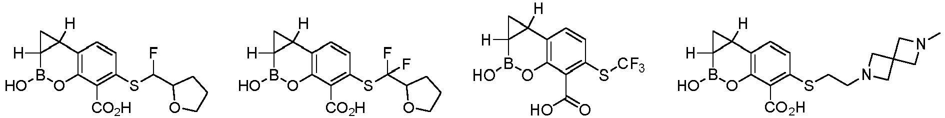

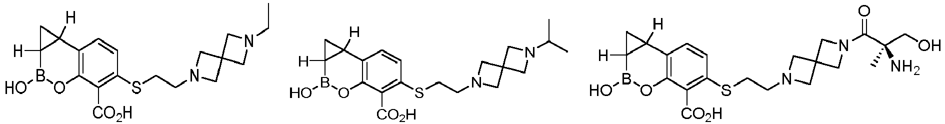

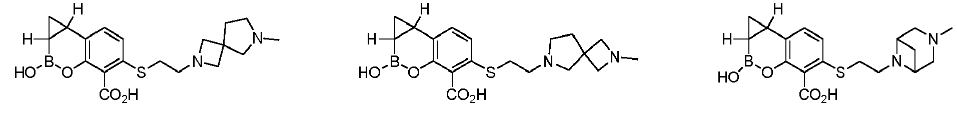

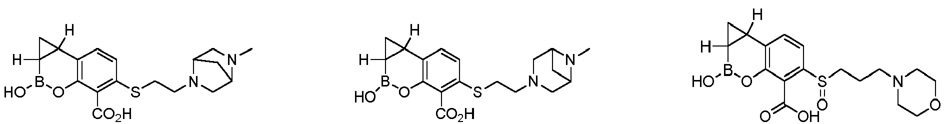

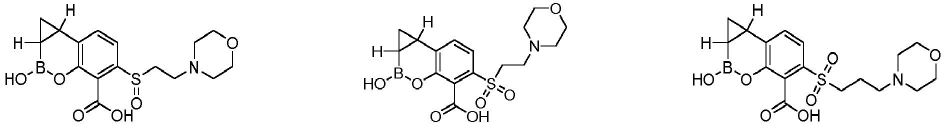

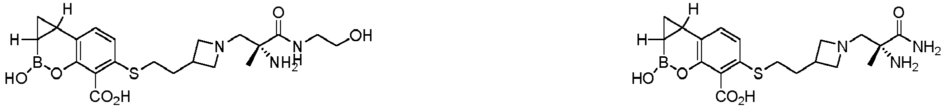

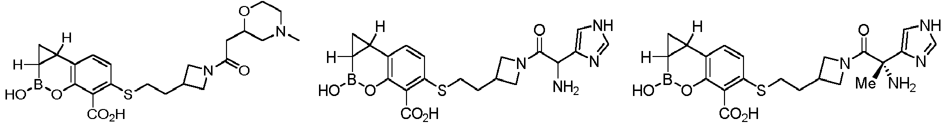

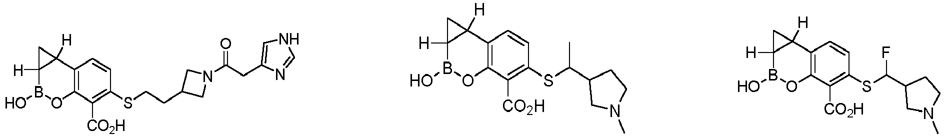

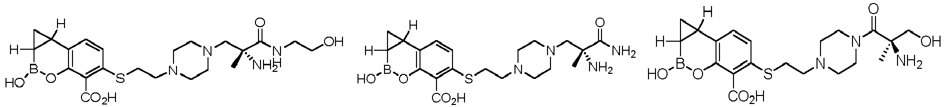

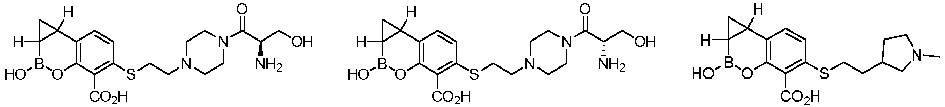

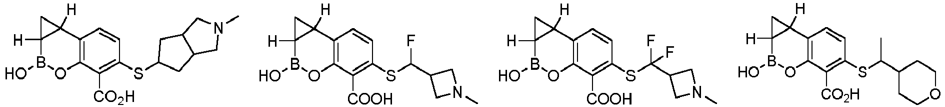

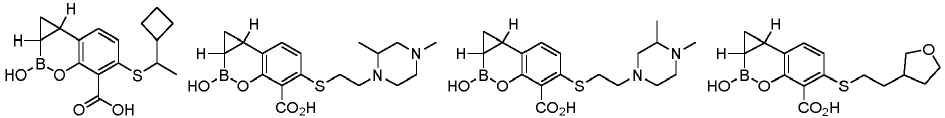

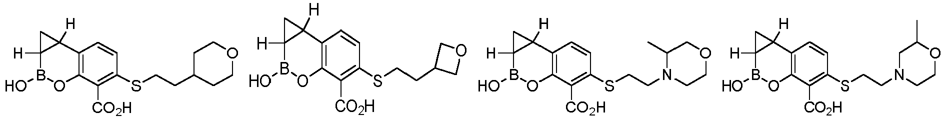

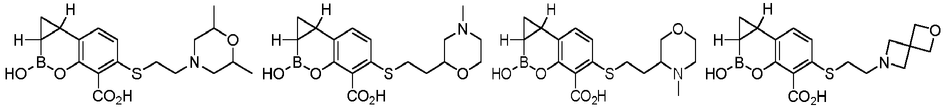

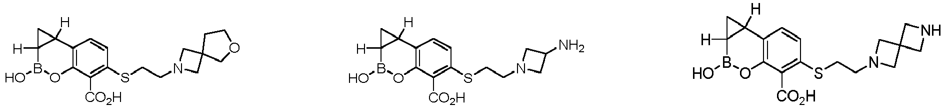

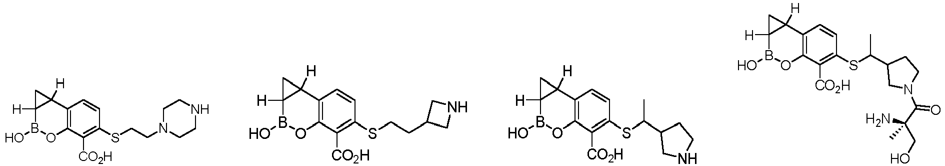

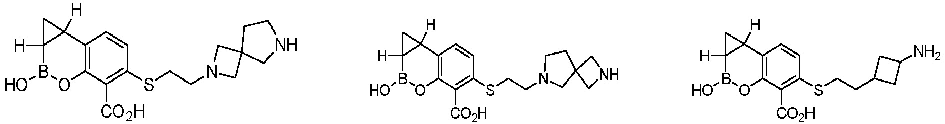

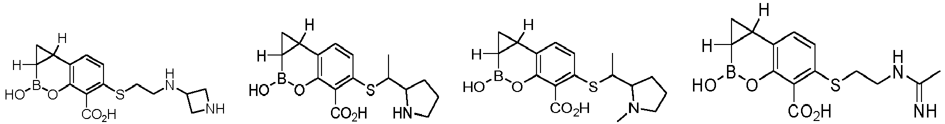

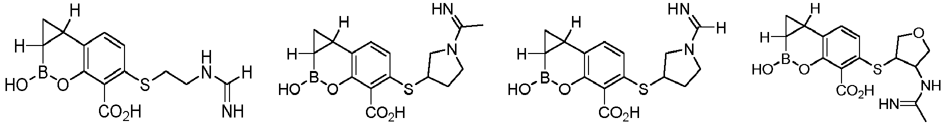

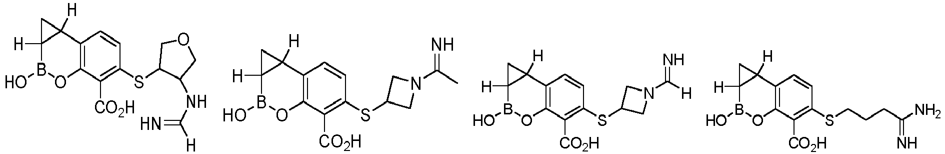

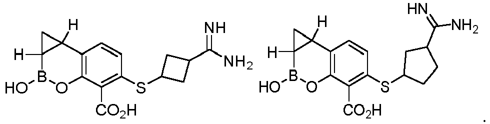

17. A compound of the following formula, an optical isomer thereof, and a pharmaceutically acceptable salt thereof, selected from:

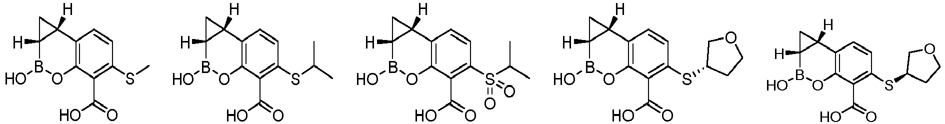

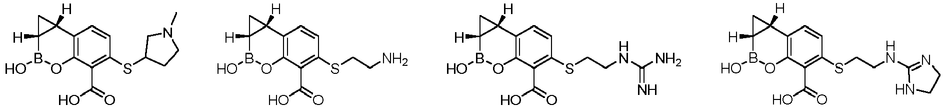

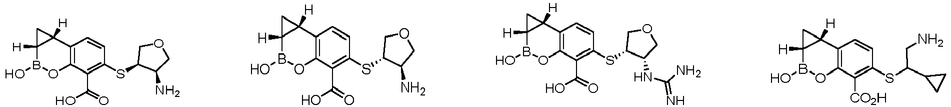

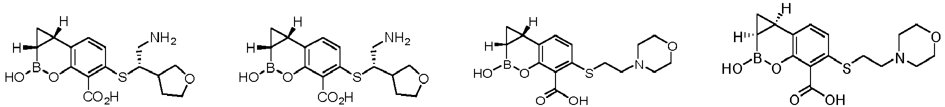

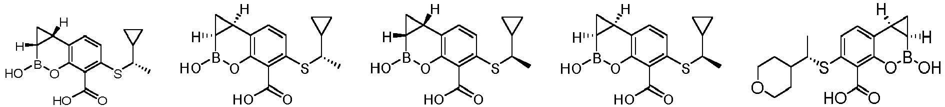

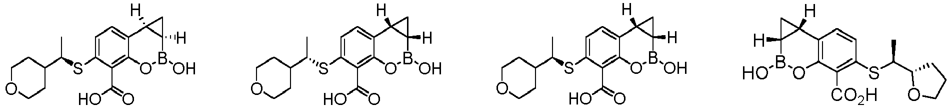

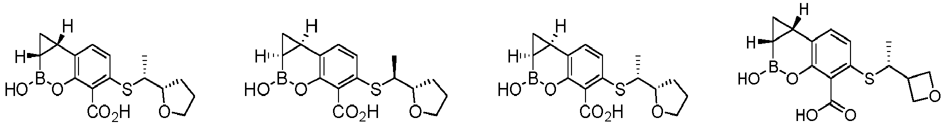

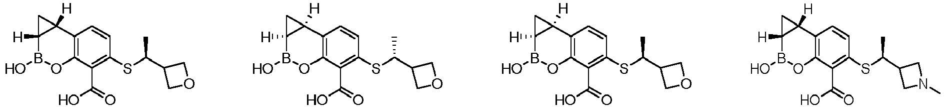

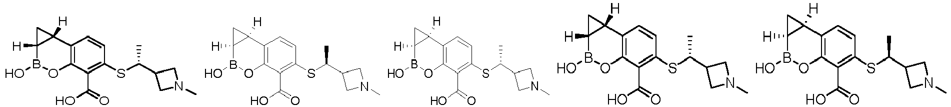

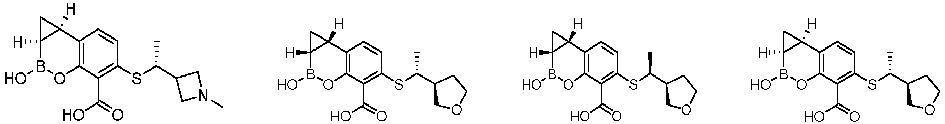

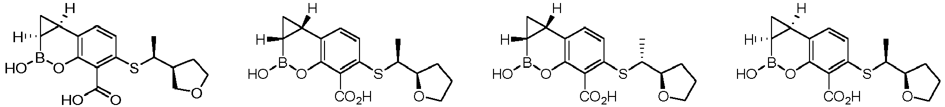

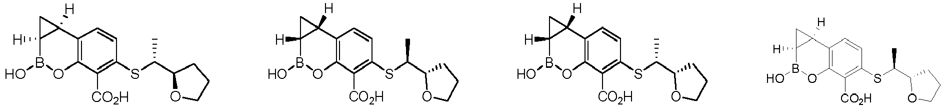

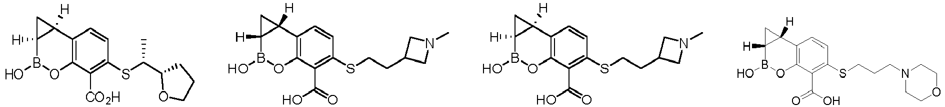

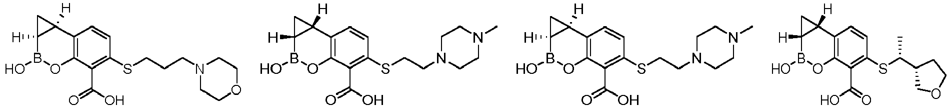

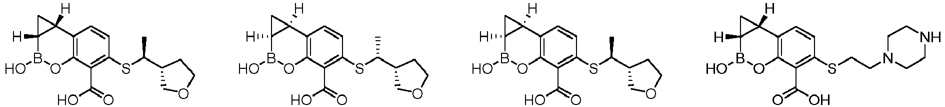

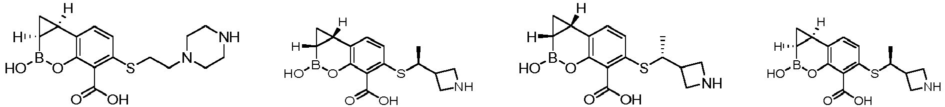

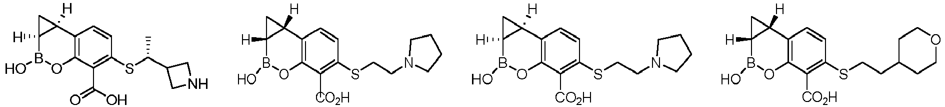

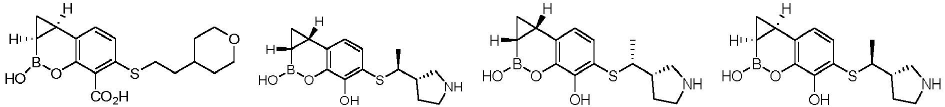

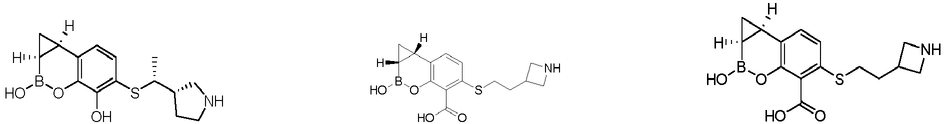

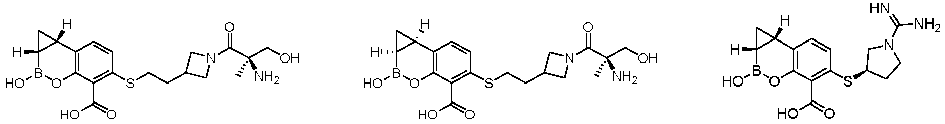

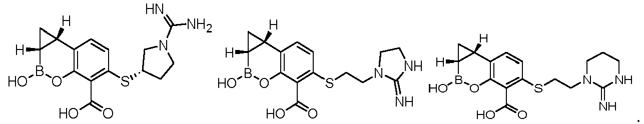

18. A use of the compound, the optical isomer thereof, and the pharmaceutically acceptable salt thereof according to any one of claims 1 to 17 in the manufacture of a medicament for the treatment of a bacterial infection disease.

19. The use according to claim 18, wherein the use further comprises a combination of the compound, the optical isomer thereof, and the pharmaceutically acceptable salt thereof according to any one of claims 1 to 17 with other β-lactam antibiotic.

20. The use according to claim 19, wherein the β-lactam antibiotic is selected from penicillin, cephalosporin, carbapenem, monocyclic β-lactam antibiotic, or a combination thereof.

21. The use according to claim 20, wherein the penicillin is selected from acid-resistant penicillin, enzyme-resistant penicillin, ampicillin, amoxicillin, pivampicillin, carbenicillin, sulbenicillin, ticarcillin, furbenicillin, azlocillin, and piperacillin;
optionally, the cephalosporin is selected from cefalexin, cefradine, cefazolin, cefuroxime, cefamandole, cefaclor, cefotaxime, ceftazidime, ceftriaxone, cefoperazone, ceftizoxime, cefepime, cefpirome, and ceftolozane;
optionally, the carbapenem is selected from imipenem, meropenem, panipenem, biapenem, ertapenem, and faropenem;
optionally, the monocyclic β-lactam is selected from aztreonam.
